(19) Europäisches Patentamt
European Patent Office
Office européen des brevets

(11) Veröffentlichungsnummer: **0 234 395**
**B1**

(12) **EUROPÄISCHE PATENTSCHRIFT**

(45) Veröffentlichungstag der Patentschrift: **06.02.91**

(51) Int. Cl.$^5$: **C 08 G 18/06,** C 09 D 5/44, C 09 D 175/04

(21) Anmeldenummer: **87101797.6**

(22) Anmeldetag: **10.02.87**

(60) **Teilanmeldung 90111597.2 eingereicht am 10/02/87.**

# (54) Aminourethane, Verfahren zu ihrer herstellung und ihre Verwendung.

(30) Priorität: **13.02.86 DE 3604434**
**19.07.86 DE 3624454**

(43) Veröffentlichungstag der Anmeldung:
**02.09.87 Patentblatt 87/36**

(45) Bekanntmachung des Hinweises auf die Patenterteilung:
**06.02.91 Patentblatt 91/06**

(84) Benannte Vertragsstaaten:
**AT BE CH DE ES FR GB GR IT LI LU NL SE**

(56) Entgegenhaltungen:
**EP-A-0 121 837**
**US-A-2 802 022**
**US-A-3 084 140**

**INT. POLYMER SCIENCE & TECHNOLOGY, Band 8, Nr. 5, 1981, Shawbury, Shrewsbury, GB; KAUCHUK I REZINA, Nr. 1, 1981, Seite 25; L. YA. RAPPOPORT et al.: "Polyurethane elastomers obtained without the use of diisocyanates"**

(73) Patentinhaber: **HOECHST AKTIENGESELLSCHAFT**
**Postfach 80 03 20**
**D-6230 Frankfurt am Main 80 (DE)**

(72) Erfinder: **Walz, Gerd, Dr.**
**Pfingstbornstrasse 99**
**D-6200 Wiesbaden (DE)**
Erfinder: **Hönel, Michael, Dr.**
**Wallstrasse 24**
**D-6500 Mainz (DE)**
Erfinder: **Brindöpke, Gerhard, Dr.**
**Loreleistrasse 18**
**D-6230 Frankfurt am Main 80 (DE)**
Erfinder: **Sprenger, Walter**
**Ringstrasse 8**
**D-6110 Dieburg (DE)**
Erfinder: **Finke, Manfred, Dr.**
**Behringstrasse 25**
**D-6233 Kelkheim (Taunus) (DE)**
Erfinder: **Lenz, Rüdiger, Dr.**
**Geisenheimer Strasse 88**
**D-6000 Frankfurt am Main (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 234 395 B1

**Beschreibung**

Die Erfindung bezieht sich auf härtbare, wasserdispergierbare Bindemittel auf Basis von Umsetzungsprodukten von modifizierten 2-Oxo-1,3-dioxolanen und primären Aminen, die mindestens eine primäre Aminogruppe und zusätzlich weitere basische Gruppierungen enthalten, und die insbesondere aus wäßriger Phase bei der kationischen Elektrotauchlackierung abgeschieden werden können.

In der DE—PS 22 52 536 werden selbstvernetzende Bindemittel für die kathodische Elektrotauchlackierung beschrieben, die aus einem Polyurethanharz, das durch Umsetzung von Epoxyd-Amin-Addukten, hergestellt aus einer epoxygruppenhaltigen organischen Verbindung mit einem sekundären Amin und einem teilweise blockierten Polyisocyanat, erhalten worden sind. Als Verkappungsmittel werden vorzugsweise primäre aliphatische Alkohole eingesetzt. Die Bindemittel müssen jedoch im allgemeinen bei relativ hohen Einbrenntemperaturen z.B. 180°C gehärtet werden.

Kathodische Elektrotauchlacke werden auch in den DE—Offenlegungsschriften 33 11 517 und 33 11 518 beschrieben, bei denen Polyamine mit mindestens zwei primären Aminogruppen mit Epoxydharzen zu Epoxid-Amin-Addukten umgesetzt und die erhaltenen Reaktionsprodukte mit Alkylencarbonat unter Bildung von β-Hydroxyurethanen zur Reaktion gebracht werden. Die erhaltenen β-Hydroxyurethane benötigen im allgemeinen zwar niedrigere Härtungstemperaturen als die vorstehend beschriebenen Bindemittel, jedoch ist die Einführung der primären Aminogruppen umständlich und benötigt zusätzliche Verfahrensschritte.

In der EP—Offenlegungsschrift 0 119 769 sind verschiedene Möglichkeiten angeführt, tertiäre Aminogruppen in das Grundgerüst eines Harzes einzuführen. Neben der Umsetzung von Epoxyverbindungen mit Aminkomponenten nach verschiedenen Verfahren werden auch solche von α,β-ungesättigte Carboxylgruppen enthaltenden Harzen oder Carboxylgruppen enthaltenden Harzen mit Aminokomponenten beschrieben. Die erhaltenen Verbindungen werden dann mit Alkylencarbonaten zu β-Hydroxyurethangruppen enthaltenden Bindemitteln umgesetzt.

Bei der Härtung werden unter Abspaltung von Diolen, die aber physiologisch nicht unbedenklich sind, Urethan- oder Harnstoffbindungen gebildet. Ein Nachteil der Umsetzung von Epoxydverbindungen mit Ketiminen ist die Einhaltung von wassersfreien Bedingungen bei der Reaktion, um eine vorzeitige Hydrolyse zu vermeiden.

Durch einen grundsätzlich anderen Weg werden die Verbindungen gemäß der vorliegenden Erfindung erhalten. Hierbei werden primäre aminogruppenhaltige und weitere basische Gruppen enthaltende Verbindungen mit Carbonaten umgesetzt, so daß stets basische Aminogruppen in den entstandenen Produkten enthalten sind. Die Härtung von Überzügen, welche die erfindungsgemäßen Verbindungen als Bindemittel enthalten, kann durch Bildung von Urethan- oder Harnstoffbindungen hervorgerufen werden. Die erfindungsgemäßen Verbindungen können durch Einbau von verkappten Isocyanatgruppen selbsthärtend sein oder können mit zugesetzten bekannten Härtern über die in den Bindemitteln vorhandenen funktionellen Gruppen gehärtet werden.

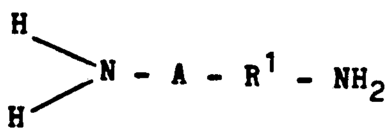

(I),

in der bedeuten

$R^1$ einen zweiwertigen Kohlenwasserstoffrest, vorzugsweise einen geradkettigen oder verzweigten Alkylenrest mit 2 bis 18 C-Atomen, und

A eine chemische Bindung oder $—(R^1—NH)_r—R^1—NH—$, worin r Null oder eine ganze Zahl von 1 bis 6 ist und $R^1$ die vorstehende Bedeutung hat,

Struktureinheiten, die sich ableiten von (β) oligomeren oder polymeren Verbindungen, die mindestens zwei endständige 2-Oxo-1,3-dioxolan-Gruppen enthalten, und Struktureinheiten, die sich ableiten von Polyaminen der allgemeinen Formel (I′)

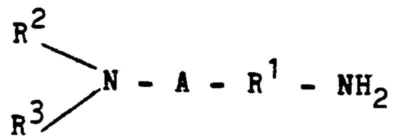

(I′),

in der bedeuten

$R^1$ und A gleiche Bedeutung wie in obiger Formel (I),

$R^2$ Wasserstoff und $R^3$ mindestens einer der Reste a) bis d)

a) $$-\overset{\overset{\displaystyle O}{\|}}{C}-O-CH_2-\overset{\overset{\displaystyle R^4}{|}}{C}H-CH_2-[-R-CH_2-\overset{\overset{\displaystyle R^4}{|}}{C}H-CH_2-O-]_m-B-(R^4)_n$$

in dem R gleich oder verschieden sein kann und für den Rest eines Diglycidylethers oder -esters, der

gegebenenfalls auch ($NR^2$)-Gruppen enthalten kann, wobei $R^2$ die obige Bedeutung hat, oder für einen zweiwertigen Kohlenwasserstoffrest mit 2 bis 18 C-Atomen steht, $R^4$ Hydroxyl oder den Rest

$$-O-\overset{O}{\overset{\|}{C}}-NH-PI-NH-\overset{O}{\overset{\|}{C}}-R^6$$

darstellt, in dem PI der Rest eines Polyisocyanats und $R^6$ der Rest eines aliphatischen, cycloaliphatischen oder alkylaromatischen einwertigen Alkohols, eines Aminoalkohols, eines

$$a)\quad -\overset{O}{\overset{\|}{C}}-O-CH_2-\overset{R^4}{\overset{|}{C}H}-CH_2-[-R-CH_2-\overset{R^4}{\overset{|}{C}H}-CH_2-O-]_m-B-(R^4)_n$$

in dem R gleich oder verschieden sein kann und für den Rest eines Diglycidylethers oder -esters, der gegebenenfalls auch ($NR^2$)-Gruppen enthalten kann, wobei $R^2$ die obige Bedeutung hat, oder für einen zweiwertigen Kohlenwasserstoffrest, vorzugsweise einen gegebenenfalls substituierten, verzweigten oder unverzweigten Alkylenrest mit 2 bis 18 C-Atomen, vorzugsweise 2 bis 10 C-Atomen, steht, $R^4$ Hydroxyl oder den Rest

$$-O-\overset{O}{\overset{\|}{C}}-NH-PI-NH-\overset{O}{\overset{\|}{C}}-R^6$$

darstellt, in dem PI der Rest eines Polyisocyanates und $R^6$ der Rest eines aliphatischen, cycloaliphatischen oder alkylaromatischen einwertigen Alkohols, eines Aminoalkohols, eines Ketoxims, einer CH— oder NH-aciden Verbindung ist, B den Rest eines Polyols bedeutet, m eine ganze Zahl von 1 bis 3, vorzugsweise 1, und n eine ganze Zahl von 1 bis 6, vorzugsweise 1 bis 3, ist, oder

$$b)\quad -\overset{O}{\overset{\|}{C}}-O-CH_2-\overset{R^4}{\overset{|}{C}H}-CH_2-[-R-CH_2-\overset{R^4}{\overset{|}{C}H}-CH_2-O-]_m-\overset{O}{\overset{\|}{C}}-\underset{\underset{R^7}{|}}{\overset{\overset{\overset{R^5}{|}}{(CH_2)_s}}{\overset{|}{C}}}-(CH_2)_s-R^4$$

in dem R, $R^4$ und m die schon genannte Bedeutung haben, $R^5$ Wasserstoff oder $R^4$ ist, $R^7$ einen Alkylrest mit 1 bis 8, vorzugsweise 1 bis 6 C-Atomen, insbesondere $CH_3$- bedeutet und s eine ganze Zahl von 1 bis 6, vorzugsweise 1 bis 2, darstellt, oder

$$c)\quad -\overset{O}{\overset{\|}{C}}-NH-PI-NH-\overset{O}{\overset{\|}{C}}-O-B-(-R^4)_n \qquad \text{oder}$$

d) die Gruppen $R^8$-CHOH—$CH_2$—O—CO— oder $R^8$—CHOH—$CH_2$—, in denen $R^8$ Wasserstoff, Alkyl mit 1 bis 18, vorzugsweise 1 bis 10 C-Atomen oder Reste von Glycidylestern oder -ethern, vorzugsweise Versaticsäureglycidylestern ist oder die Gruppe

$$PI^1-\underset{H}{\underset{|}{N}}-CO-$$

darstellt, in der $PI^1$ der Rest eines teilverkappten Polyisocyanats ist.

Weiterhin betrifft die Erfindung auch nicht selbstvernetzende Aminourethane gemäß Anspruch 2.

Die Menge an (α) und (γ) in den erfindungsgemäßen Aminourethanen beträgt im allgemeinen 35 bis 85 Mol-%, vorzugsweise 50 bis 60 Mol-%, und die von (β) 65 bis 15 Mol-%, vorzugsweise 40 bis 60 Mol-%.

Beispiele für Polyole des Restes B sind vorzugsweise Di- und Triole wie Ethylen- und Propylenglykol, Polycaprolactonpolyol, Trimethylolpropan, Glycerin, Neopentylglykol und Pentaerythrit.

Vorzugsweise besitzen die erfindungsgemäßen Aminourethane die allgemeine Formel (II)

$$R^3-\left[\overset{R^{2'}}{\overset{|}{N}}-A-R^1-\overset{H}{\overset{|}{N}}-\overset{O}{\overset{\|}{C}}-O-CH_2-\overset{R^4}{\overset{|}{C}H}-CH_2-R-CH_2-\overset{R^4}{\overset{|}{C}H}-CH_2-O-\overset{O}{\overset{\|}{C}}\right]_y-\overset{H}{\overset{|}{N}}-R^1-A-N\begin{matrix}R^2\\ R^3\end{matrix} \quad (II),$$

in der $R^1$ bis $R^4$ sowie A die obige Bedeutung haben,

$R^{2'}$ gleich $R^2$ ist, mit der Maßgabe, daß $R^{2'}$ nur Wasserstoff darstellt, falls das betreffende Stickstoffatome nicht am Kettenende steht,

y eine ganze Zahl von 1 bis 10, vorzugsweise 1 bis 5 bedeutet und

R gleich und verschieden sein kann und für den Rest eines Diglycidylethers oder -esters, der gegebenenfalls auch ($NR^2$)-Gruppen enthalten kann, wobei $R^2$ die obige Bedeutung hat, oder für einen zweiwertigen Kohlenwasserstoffrest, vorzugsweise einen gegebenenfalls substituierten, verzweigten oder unverzweigten Alkylenrest mit 2 bis 18 C-Atomen, vorzugsweise 2 bis 10 C-Atomen, steht.

Insbesondere ist y in der vorstehenden Formel (II) gleich 1, d.h. die besonders bevorzugten Aminourethane besitzen erfindungsgemäß die allgemeine Formel (IIa)

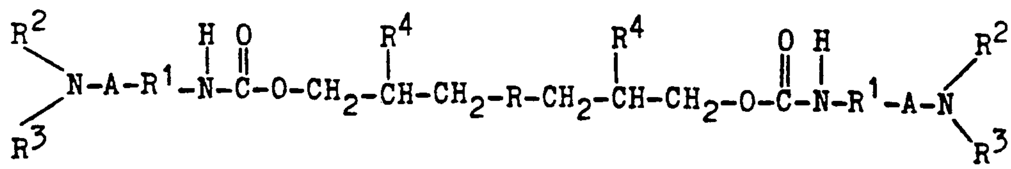
(IIa),

in der R, $R^1$ bis $R^4$ sowie A die obige Bedeutung haben.

Was den Rest R in diesen Formeln (II) und (IIa) anbelangt, so kann dieser nach einer Ausführungsform der Erfindung die nachstehende Formel aufweisen

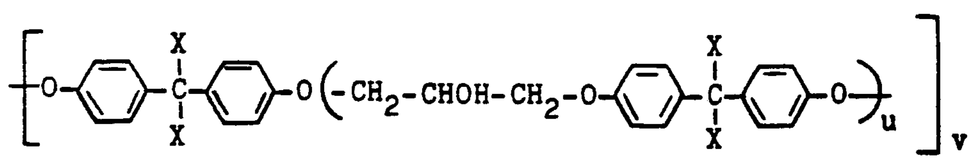
,

in der X Wasserstoff oder Methyl, u 0 bis 5 und v 1 bis 20, vorzugsweise 1 bis 6 ist. Die Werte für u und v sind als statistisches Mittel anzusehen, da durch die Molekulargewichtsverteilung der Glycidylether ein großer Bereich einbezogen werden kann.

Eine weitere Ausführungsform hat solche Aminourethane zum Inhalt, bei denen R in den Formeln (II) und (IIa) den Rest

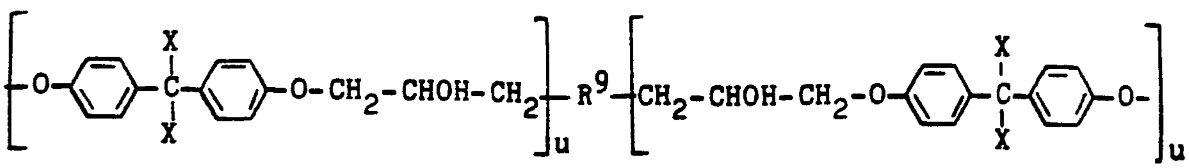

darstellt, worin X und u die genannte Bedeutung haben und $R^9$ O-Alkyl-O, N-Alkyl-N mit jeweils 2 bis 18 C-Atomen im Alkylrest sowie der Rest von Polyaminen, Polyolen, Polycaprolactonpolyolen, OH-gruppenhaltigen Polyestern, Polyethern, hydroxy-, carboxyl- und aminofunktionellen Polymerölen, Polycarbonsäuren, hydroxy- oder aminofunktionellen Polytetrahydrofuranen und Reaktionsprodukten von Polyaminen mit Glycidylestern von in α-Stellung verzweigten Carbonsäuren mit 8 bis 14 C-Atomen (sog. ®Versaticsäuren) ist. Vorzugsweise stellt $R^9$ ein Rest gemäß einer der folgenden Formeln dar:

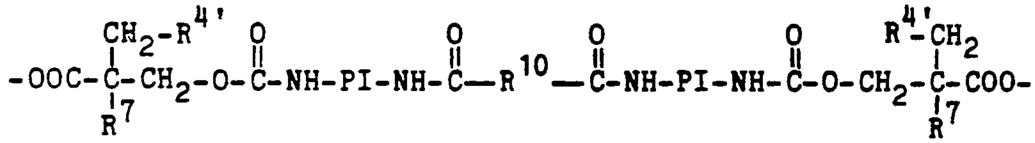

wobei die Reste $R^7$ und PI die obige Bedeutung haben, $R^{4'}$ neben $R^4$ auch zusätzlich Wasserstoff sein kann, $R^{10}$ für die unter $R^9$ angeführten Reste mit Ausnahme der Polycarbonsäuren und der carboxylfunktionellen Polymeröle steht oder den Rest

$$—OOC—R^{11}—CO—R^{10}—CO—R^{11}—COO—$$

bedeutet, in dem $R^{10}$ wie vorstehend definiert ist und $R^{11}$ den aliphatischen, cycloaliphatischen oder aromatischen Rest einer Polycarbonsäure darstellt. Beispiele hierfür sind o- und p-Phthalsäure, Tetrahydrophthalsäure, Bernsteinsäure, Trimellithsäure, wobei letztere vorzugsweise mit einem primären Diamin als Rest $R^{10}$ unter Imidbildung umgesetzt worden ist.

Nach einer anderen Ausführungsform besitzt R in Formel (II) die Struktur

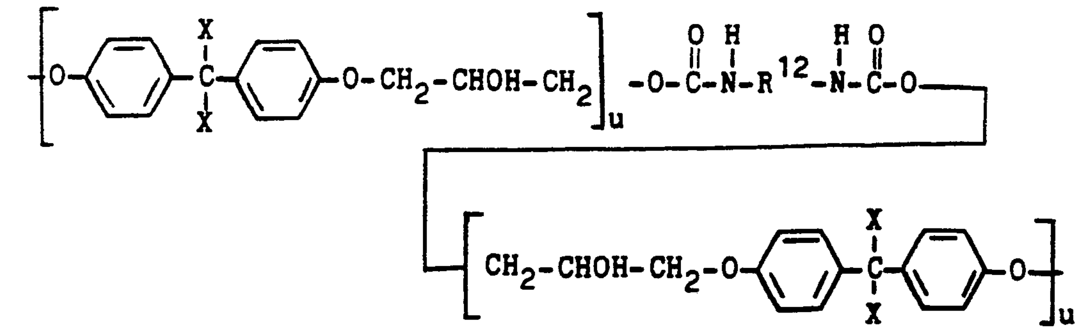

worin X und u die genannte Bedeutung haben und $R^{12}$ Alkylen mit 2 bis 18 C-Atomen, der Rest eines Poly(sek.)Amins oder aminofunktionelles Polytetrahydrofuran ist.

Nach einer weiteren Ausführungsform besitzt R in Formel (II) die Struktur

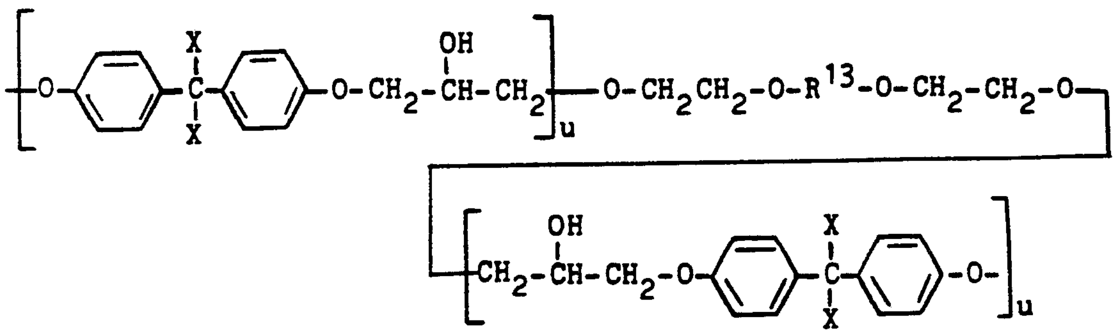

worin X und u die genannte Bedeutung haben, u vorzugsweise aber 1 ist, und $R^{13}$ den Rest

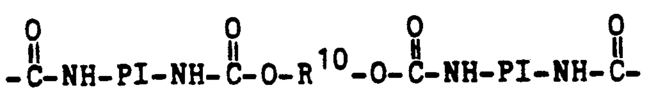

oder

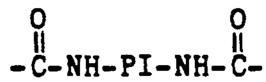

darstellt, wobei $R^{10}$ und PI die schon genannten Definitionen haben.

Weiterhin können ein Teil oder alle der vorhandenen Hydroxyl- oder Aminogruppen in den Verbindungen der Formeln (II) oder (IIa) mit teilverkappten Polyisocyanaten umgesetzt sein.

Von besonderem Interesse sind solche Produkte, bei denen als weitere Aminogruppe eine primäre oder sekundäre Aminogruppe vorhanden ist. Auf diese Weise kann der nach der Härtung verbleibende Anteil von basisch wirkenden Stickstoffgruppierungen im Molekül deutlich vermindert werden.

Gegenstand der Erfindung ist auch ein Verfahren zur Herstellung von selbsthärtenden Aminourethanen, das dadurch gekennzeichnet ist, daß man (α) Polyamine der allgemeinen Formel (I)

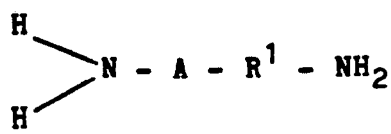

(I),

in der $R^1$ sowie A die obige Bedeutung besitzen, mit (β) oligomeren oder polymeren Verbindungen, die mindestens zwei endständige 2-Oxo-1,3-dioxolan-Gruppen und zumindest teilweise zusätzlich Urethan-Gruppen enthalten und mit Verbindungen (γ) (Kettenstopper) aus der Gruppe teilverkappte Polyisocyanate, Monoepoxide, Monocarbonate und Amine der allgemeinen Formel (I')

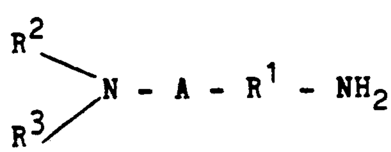

(I'),

in der $R^1$ bis $R^3$ und A die obige Bedeutung besitzen, umsetzt.

Weiterhin betrifft die Erfindung auch ein Verfahren zur Herstellung von nicht selbstverhärtenden Aminourethanen gemäß Anspruch 13.

Die Menge an Polyamin (α) und an Kettenstopper (γ) beträgt im allgemeinen 35 bis 85 Mol-%, vorzugsweise 50 bis 60 Mol-% und die von Komponente (β) 65 bis 15 Mol-%, vorzugsweise 40 bis 50 Mol-%.

Falls in der obigen Formel (I) der Komponente (α) das Symbol A für eine chemische Bindung steht, besitzen die erfindungsgemäß einsetzbaren Polyamine die Formel

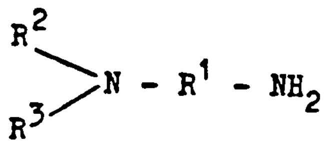 (Ia)

mit den für $R^1$ bis $R^3$ oben angegebenen Definitionen. Beispiele für derartige Polyamine sind: N-Methylethylendiamin, Hydroxyethylaminoethylamin, -propylamin, N,N'-dimethylethylendiamin, N,N'-Dimethylpropylendiamin, N,N'-Dihydroxyethylethylendiamin; Ethylendiamin, Propylendiamin, Hexamethylendiamin, Octamethylendiamin, Triacetondiamin, Dioxadecandiamin, Dioxadodecandiamin und höhere Homologe; cycloaliphatische Diamine wie 1,4-Cyclohexandiamin, 4,4'-Methylen-biscyclohexylamin und 4,4'-Iso-propylenbis-cyclohexylamin, Isophorondiamin und N-Aminoethylpiperazin. Selbstverständlich können auch Gemische dieser Polyamine untereinander, beispielsweise auch di-primäre mit mono-primären, wie auch mit denen gemäß der nachstehenden Formel (Ib) verwendet werden. Die dabei resultierenden Mischungen von Aminourethanen sind für die erfindungsgemäßen Zwecke gleichfalls gut geeignet.

In der obigen Formel (I) kann A auch für $-(R^1NH)_r-R^1-NH-$ stehen. Die Komponente (α) weist dann also die Formel

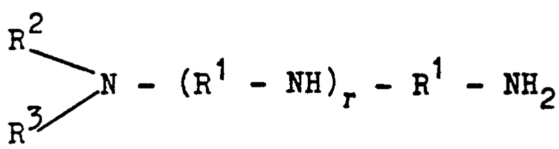 (Ib)

auf, in der $R^1$ bis $R^3$ die obige Bedeutung haben und r für 1 bis 6, vorzugsweise 1 bis 4 steht.

Beispiele hierfür sind: Diethylentriamin, Dipropylentriamin, Bishexamethylentriamin, Triethylentriamin, Tetraethylenpentamin, Pentaethylhexamin, Heptaethylenoctamin und ähnliche.

Die oligomere oder polymere Verbindung (β) besitzt vorzugsweise die allgemeine Formel (III)

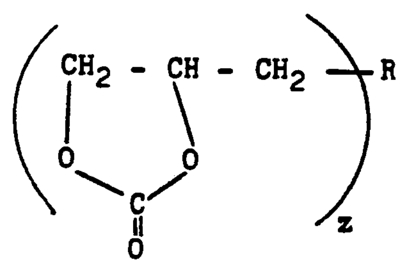 (III),

in der R die obige Bedeutung hat, jedoch in seiner Wertigkeit z entspricht, und z für ganze Zahlen von 1 bis 5, vorzugsweise 2 und 3 und insbesondere 2 steht.

In dieser Formel ist R bevorzugt der Rest eines Polyethers, Polyetherpolyols, Polyesters, Polyesterpolyols, ferner ein Alkylenrest, ein Poly(sek.)aminrest oder auch ein Umsetzungsprodukt einer Epoxy-Carbonat-Verbindung mit Polyaminen, aliphatischen oder aromatischen Polyolen, von Novolaken, Polycaprolactonpolyolen, OH-gruppenhaltigen Polyestern, Polyethern, Polyglykolen, hydroxy-, carboxyl- und aminofunktionellen Polymerölen, Polycarbonsäuren, hydroxy- oder aminofunktionellen Polytetrahydrofuran und Reaktionsprodukten von Polyaminen mit Glycidylestern von α-Alkylalkanmonocarbonsäuren und/oder α,α-Dialkylalkanmonocarbonsäuren der Summenformel $C_{12-14}H_{22-26}O_3$ (Versaticsäuren), einzeln oder im Gemisch. Die α-Alkylalkansäure- und α,α-Dialkylalkansäure-Gemische stellen Monocarbonsäuren dar, die eine $C_9$-, $C_{10}$- und $C_{11}$-Kette enthalten. Die Ester werden nachfolgend als Versaticsäureglycidylester bezeichnet. Hydroxyl-, carboxyl- und aminofunktionelle Polymeröle sind handelsüblich und stellen modifizierte Polybutadiene mit einem Molekulargewicht von 800 bis 10000 dar.

Das Molekulargewicht $M_w$ (Gewichtsmittel, bestimmt mit der Gelchromatographie, Polystyrolstandard) der Verbindungen gemäß Formel (III) liegt im allgemeinen zwischen 300 und 10000, vorzugsweise zwischen 800 und 4000.

Die Verbindungen gemäß Formel (III), wie auch die nachfolgend beschriebenen Verbindungen (IV) und (V), können durch Anlagerung von $CO_2$ an die entsprechenden epoxidgruppenhaltigen Verbindungen

hergestellt werden. Derartige Verfahren sind beispielsweise in der PCT(WO)-Patentanmeldung 84/03 701 sowie in den DE-Patentanmeldungen P 35 29 263.6 und P 36 00 602.5 beschrieben. Geeignete Ausgangspolyepoxide sind beispielsweise in Wagner/Sarx, "Lackkunstharze", Carl Hanser Verlag (1971), S. 174 ff sowie in der EP-Offenlegungsschrift 60 506 aufgeführt, auf die hier gleichfalls Bezug genommen werden.

Bevorzugte Ausgangsstoffe zur Herstellung der cyclischen Carbonate (III) und der gemischten Epoxyd-Carbonat-Verbindungen (IV) sind die Polyglycidylether von Polyphenolen z.B. Bisphenol A. Die Glycidyläther erhält man beispielsweise durch Umsetzung eines Polyphenols mit Epichlorhydrin. Polyphenole sind beispielsweise Bis-(4-hydroxyphenyl)-2,2-propan, Bis(4-hydroxyphenyl)-methan, 4,4'-Dihydroxybenzophenon, Bis(4-hydroxyphenyl)-1,1'-ether, Bis(4-hydroxyphenyl)-1,1'-isobutan, Bis(2-hydroxynaphthyl)-methan, 1,5-Dihydroxynaphthalin. Vorzugsweise sind zusätzlich freie Hydroxylgruppen zu den Epoxydgruppen im Polyglycidylether des Polyphenols enthalten.

Die Umsetzung der Komponenten (α) und (β) erfolgt im allgemeinen in den erforderlichen stöchiometrischen Verhältnissen bzw. Mengen nach üblichen Methoden bei erhöhten Temperaturen, gegebenenfalls unter Zusatz von Katalysatoren und gegebenenfalls unter Zusatz von inerten Lösungsmitteln. Die erfindungsgemäße Umsetzung in Gegenwart von gegenüber der Cyclocarbonatgruppe inerten Lösungsmitteln stellt dabei eine bevorzugte Verfahrensvariante dar. Bei der stöchiometrischen Bewertung der Ausgangsprodukte und auch der Umsetzungsprodukte bezüglich des Fortgangs der Umsetzung werden bei den Verbindungen der Komponente (α), die Aminzahl, deren Ermittlung in üblicher Weise durch Perchlorsäuretitration erfolgen kann, und bei den Verbindungen der Komponente (β) die Cyclocarbonat-Äquivalenzzahl, die in üblicher Weise durch Titration mit Kaliumhydroxidlösung bestimmt werden kann, zugrunde gelegt. Bei der erfindungsgemäßen Umsetzung der Komponenten (α) und (β) können die erfindungsgemäßen Polyaminoverbindungen einzeln oder als Gemische oder auch zeitlich nacheinander der Reaktion zugeführt werden, gegebenenfalls gelöst in inerten organischen Lösungsmitteln. In analoger Weise können auch einzelne oder verschiedene modifizierte cyclische Carbonate der Komponente (β) einzeln oder als Gemische oder auch zeitlich nacheinander, vorzugsweise in Gemisch mit gegenüber Cyclocarbonatgruppen inerten organischen Lösungsmitteln, der Reaktion zugeführt werden.

Es ist bei der erfindungsgemäßen Umsetzung zu beachten, daß solche Reaktions- und Verfahrensbedingungen eingehalten werden, unter denen die Cyclocarbonatgruppen der Komponente (β) nur mit den primären Aminogruppen der Komponente (α) reagieren können, was nach bekannten Methoden erreichbar ist, ohne daß auch entsprechende Reaktionen mit den vorhandenen sekundären Aminogruppen stattfinden, die reaktionsträger sind.

Gemäß einer Ausführung der Erfindung ist es möglich, das erfindungsgemäße Umsetzungsprodukt als selbsthärtende Verbindung herzustellen. Dabei werden an die gegebenenfalls im cyclischen Carbonat vorhandenen Hydroxyl- bzw. sekundären Aminogruppen teilverkappte Polyisocyanate angelagert. Die Hydroxylgruppen können auch durch Anlagerung von Formaldehyd an einem aromatischen Kern der phenolischen Komponente gebildet werden. Diese Reaktion wird unter solchen Bedingungen durchgeführt, daß das cyclische Carbonat nicht angegriffen wird.

Polyisocyanate, die für diese Verbindungen mit dem Rest PI eingesetzt werden, können beliebige Polyisocyanate sein, beispielsweise ein aliphatisches, cycloaliphatisches oder aromatisches Polyisocyanat. Ein Teil der Isocyanatgruppen kann in bekannter Weise mit üblichen Verkappungsmitteln umgesetzt worden sein. Typische Beispiele für die verwendeten Polyisocyanate sind Xylylendiisocyanat, Diphenylmethan-4,4'-diisocyanat, Triphenylmethyl-4,4'-triisocyanat, Triphenylmethantriisocyanat, Polyphenyl-polymethyl-isocyanat, 2,2,4(2,4,4)-Methylcyclohexyldiisocyanat, Dicyclohexylmethyldiisocyanat, Diethylfumarhexylisocyanat, Bis-(3-Methyl-4-isocyanatocyclo-hexyl)-methan, 2,2-Bis-(4-isocyanatocyclohexyl-)propan, den Methylester, des Lysindiisocyanat, das Biuret des Hexamethylendiisocyanats, Diisocyanate dimerer Säuren, 1-Methyl-benzol-2,4,5-triisocyanat, Biphenyl-2,4,4'-triisocyanat, das Triisocyanat aus 3 Mol Hexamethylendiisocyanat und 1 Mol Wasser mit 16% NCO-Gehalt und weitere, wenigstens zwei NCO-Gruppen pro Molekül enthaltende Verbindungen, vorzugsweise Isophorondiisocyanat, Hexamethylendiisocyanat sowie Tri- und Tetramethylhexamethylendiisocyanat, insbesondere aber 2,4- oder 2,6-Toluylendiisocyanat oder Gemische dieser Verbindungen. Die in den Verbindungen dem Rest PI zugrundeliegenden Polyisocyanate können gleich oder verschieden sein.

Neben diesen einfachen Polyisocyanaten sind auch solche geeignet, die Heteroatome in dem die Isocyanatgruppen verknüpfenden Rest enthalten. Beispiele hierfür sind Polyisocyanate, die Carbodiimidgruppen, Allophonatgruppen, Isocyanuratgruppen, Urethangruppen, acylierte Harnstoffgruppen und Biuretgruppen aufweisen.

Geeignete Polyisocyanate sind schließlich auch die bekannten, endständigen Isocyanatgruppen aufweisenden Präpolymere, wie sie insbesondere durch Umsetzung der obengenannten einfachen Polyisocyanate, vor allem Diisocyanate mit unterschüssigen Mengen an organischen Verbindungen mit mindestens zwei gegenüber Isocyanatgruppen reaktionsfähigen Gruppen zugänglich sind. Bevorzugt werden diese Präpolymeren allerdings als externe Härterkomponente bei den fremdvernetzenden Systemen eingesetzt.

Als Verkappungsmittel eignen sich aliphatische, cycloaliphatische oder alkylaromatische (einwertige) Alkohole z.B. niedere aliphatische Alkohole wie Methyl-, Äthyl-, die verschiedenen Propyl-, Butyl- und

Hexylalkohole, Heptyl-, Octyl-, Nonyl-, Decylalkohol und ähnliche, ferner ungesättigte Alkohole wie Allylalkohole, cycloaliphatische Alkohole wie Cyclopentanol, Cyclohexanol, alkylaromatische Alkohole wie Benzylalkohol, Methyl sowie p-Methoxy- und p-Nitrobenzylalkohol und Monoäther von Glykolen wie Ethylen-glykolmonoethyläther, -monobutyläther und ähnliche. Weitere Verkappungsmittel sind Ketoxime, zweckmäßigerweise mit 3 bis 20 C-Atomen, vorzugsweise 3 bis 10 C-Atomen, wie Acetonoxim, Methylethylketonoxim (=Butanonoxim), Hexanonoxim (wie Methyl-Butylketonoxim), Heptanonoxim (wie Methyl-n-Amylketonoxim), Octanonoxim und Cyclohexanonoxim, CH-acide Verbindungen wie Malonsäurealkylester, Acetessigster sowie Cyanessigester mit jeweils 1 bis 4 C-Atomen in der Estergruppe, NH-acide Verbindungen wie Caprolactam, Aminoalkohole wie Diäthyläthanolamin. Das als Blockierungsmittel bekannte Phenol kann in solchen Fällen eingesetzt werden, bei denen das Umsetzungsprodukt zur Herstellung von lösungsmittelhaltigen Lacken verwendet wird.

Die erfindungsgemäßen Umsetzungsprodukte können nach verschiedenen Verfahren hergestellt werden. Beispielsweise kann eine Verbindung der Formel (III), in der R z.B. der Rest eines Polyglycidyläthers vom Bisphenol A sein kann, bei dem die Epoxydgruppen zu Carbanatgruppen umgewandelt worden sind, mit einer Verbindung der Formel (Ia), in der $R^2$ und $R^3$ die angegebene Bedeutung haben, umgesetzt werden. Will man ein selbsthärtendes Produkt erhalten, wird die Verbindung (III) zuerst über vorhandene OH-Gruppen mit einem teilverkappten Polyisocyanat unter Urethanbildung umgesetzt, worauf die Reaktion mit der Verbindung (Ia) erfolgt. Ferner kann man teilverkappte Polyisocyanate zuerst mit einem Aminoalkylierungsprodukt, das durchschnittlich mindestens eine NH-Gruppe pro Molekül aufweist und das aus Phenol und/oder einem substituierten Phenol, vorzugsweise Monoalkyl- oder Monoaryl- oder Monoaralkylphenol mit einer oder gegebenenfalls zwei phenolischen Hydroxylgruppen, einem primären Alkylamin und/oder primären Alkanolamin und/oder primär-tertiären Alkyldiamin und Formaldehyd oder einer formaldehydabspaltenden Verbindung erhalten worden ist, in einer weiteren Reaktionsstufe mit einem gemischten Epoxid-Carbonat (s. nachfolgend Formel IV) d.h. einer Verbindung, in der neben cyclischen Carbonatgruppen noch Ausgangs-Epoxyidgruppen vorhanden sind, umsetzen. So erhält man auch wieder Verbindungen mit zwei Carbonatgruppen der Formel (III).

Andererseits ist es aber auch möglich, den erfindungsgemäßen Aminourethanen beispielsweise einen üblichen Härter zuzusetzen, wie sie für fremdvernetzende 2-Komponentenlacke verwendet werden. Hierfür kommen beispielsweise in Frage: verkappte Polyisocyanate, etwa wie sie oben für die selbsthärtenden Aminourethane beschrieben sind, weiter β-Hydroxyester von mindestens bifunktionellen Polycarbonsäuren, Umsetzungsprodukte von Malonsäuredialkylestern mit Aldehyden und Ketonen, die unter Wasserabspaltung zu ungesättigten Dicarbonsäureestern reagieren (Knoevenagel'sche Synthese), Umesterungshärter sowie Michael-Additionsprodukte, beispielsweise wie sie in den DE—A—33 15 469 und 34 17 441 sowie in der DE—A—36 02 981.5 beschrieben sind.

Als Härterkomponente für die erfindungsgemäßen Aminourethane, vorzugsweise in nicht-wäßrigen Lacken, eignen sich auch epoxigruppenhaltige Verbindungen, wie z.B. niedermolekulare Polyepoxide, epoxidgruppenhaltige Copolymerisate sowie Di- oder Polyglycidylether von aliphatischen oder aromatischen Alkoholen. Weiterhin sind hier als Härterkomponenten auch oligomere oder polymere Verbindung zu nennen, welche mindestens zwei 1,3-Dioxolan-2-on-Gruppen oder mindestens eine 1,3-Dioxolan-2-on-Gruppe und eine Epoxidgruppe pro Molekül enthalten; hierzu gehören beispielsweise die Verbindungen (III) und (IV).

Um der unter Verwendung dieser Umsetzungsprodukte erhaltenen Lackschicht die nötige Flexibilisierung zu geben, können die Verbindungen (I) und (III) bereits die nötigen Voraussetzungen mitbringen. Andererseits ist es auch möglich, entweder über den eingebauten Härter in Form von teilverkappten Polyisocyanaten oder über den zugemischten Härter die benötigte Flexibilisierung in das System einzubringen.

Diese Flexibilisierung kann aber auch, wie vorstehend bereits erwähnt, über Verbindungen gemäß der Formel (III) erfolgen. Hierbei geht man beispielsweise von gemischten Epoxyd-Carbonaten aus, die solche der allgemeinen Formel (IV) darstellen

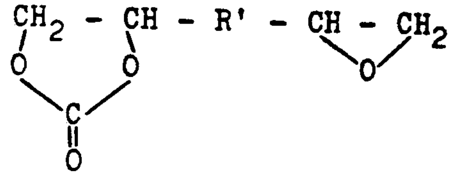

(IV)

wobei R' die Bedeutung von R in Formel (III) hat. Diese gemischten Epoxid-Carbonate werden mit Verbindungen umgesetzt, die einen flexibilisierenden Effekt auf das Molekül ausüben, beispielsweise die bereits früher genannten Polyamine, aliphatische oder aromatische Polyole, wie Diole, Triole oder Tetraole, beispielsweise Ethylen- oder Propylenglykol, Polyalkylenglykole, Neopentylglykol, Glycerin, Trimethylolpropan, Pentaerythrit sowie Polycaprolactonpolyole, weiter OH-gruppenhaltige Polyester, Polyether, Polyglykole, hydroxy-, carboxyl- und aminofunktionelle Polymeröle, Polycarbonsäuren, hydroxy- und aminofunktionelle Polytetrahydrofurane und Umsetzungsprodukte von Polyaminen mit Versaticsäureglycidylestern. Diese Umsetzungen werden unter Bedingungen vorgenommen, bei denen

nur die verbliebenen Epoxydgruppen reagieren und die Carbonatgruppen nicht angegriffen werden. Auf diese Weise erhält man auch wieder Verbindungen der Formel (III), die endständige cyclische Carbonatgruppen tragen, die mit den Aminoverbindungen (I) umgesetzt werden können.

Die endständigen Aminogruppen können mit sogenannten Kettenstoppern, das sind z.B. Monocarbonate im Falle von selbst vernetzenden Amino urethanen, oder Monoepoxydverbindungen und teilverkappte Polyisocyanate umgesetzt werden, wobei die Umsetzung gleichzeitig oder in mehreren getrennten Stufen durchgeführt werden können. Auf diese Weise entstehen die Struktureinheiten a) bis d) an den endständigen Aminogruppen. Hierbie ist es auch möglich, zunächst die Polyamine (I), in denen zumindestens einer der beiden Reste $R^2$ oder $R^3$ Wasserstoff bedeutet, in entsprechender Weise mit diesen Kettenstoppern (Blockierungsmitteln) umzusetzen und diese so partiell blockierten, nur noch mono-primären Polyamine, gegebenenfalls zusammen mit di-primären Polyaminen, mit den Verbindungen (β) zur Reaktion bringen. Dieser Weg bietet sich vor allem bei den Blockierungsmitteln an, die zu den Struktureinheiten c) (← teilweise blockiertes Polyisocyanat) oder d) (← Monocarbonat, Monoepoxid) führen.

Als Monocarbonatverbindungen eignen sich hierfür solche der Formel (V)

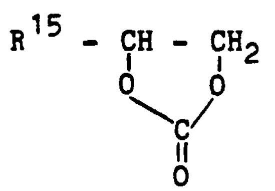

(V)

in der $R^{15}$ Wasserstoff, Alkyl mit 1 bis 18, vorzugsweise 1 bis 10 C-Atomen, oder Reste des Versaticsäureglycidylesters, Glycidylester oder Glycidylether darstellt, bei denen die Epoxydgruppe in der weiter oben angegebenen Weise zu cyclischen Carbonaten umgesetzt worden ist.

Neben den Monocarbonaten und Monoepoxyden können auch teilverkappte Polyisocyanate eingesetzt werden, da diese Verbindungen zuerst mit einer freien $NH_2$-Gruppe reagieren. Prinzipiell kann jede Aminreaktion herangezogen werden, die bevorzugt an der primären Aminogruppe ansetzt, bevor die im Molekül enthaltenen sekundären Aminogruppen reagieren. Andererseits kann die zum Kettenabbruch eingesetzte Verbindung auch zur Flexibilisierung des resultierenden Lackes dienen, indem entsprechende langkettige Verbindungen, die in der Praxis bekannt sind, eingebaut werden.

Um zu den Struktureinheiten a) oder b) zu gelangen, können Monocarbonate entsprechend der vorstehend Formel (IV) mit Polyolen, die den Rest B enthalten, oder mit Monocarbonsäuren der Formel

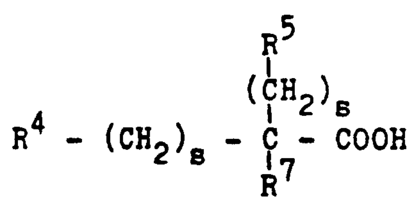

selektiv an der Epoxidgruppe umgesetzt werden. Anschließend können vorhandene OH-Gruppen ganz oder teilweise mit teilverkappten Polyisocyanaten zur Reaktion gebracht werden. Die so erhaltene, modifizierte Monocarbonatverbindung kann danach analog den unter Formel (V) angeführten Monocarbonaten eingesetzt werden, d.h. sie können im Gemisch mit den Cyclocarbonatverbindungen gemäß Formel (III) mit den Polyaminen gemäß Formel (I) zu den erfindungsgemäßen Aminourethanen umgesetzt werden. Alternativ kann das di-primäre Polyamin (I) zunächst selektiv an einer primären Aminogruppe mit dieser modifizierten Monocarbonatverbindung zur Reaktion gebracht und danach gegebenenfalls im Gemisch mit weiterem di-primären Amin mit der Cyclocarbonatverbindung (III) umgesetzt werden.

Zu Struktureinheiten c) gelangt man beispielsweise, indem man ein di-primäres Polyamin der Formel (I) selektiv an einer primären Aminogruppe mit einem teilverkappten Polyisocyanat zu einem Harnstoffderivat umsetzt. Das so hergestellte mono-primäre Polyamin wird dann, ggf. in Abmischung mit weiterem di-primären Polyamin, mit Cyclocarbonaten gemäß Formel (III) zu den erfindungsgemäßen Aminourethan umgesetzt.

Um zu den Struktureinheiten d) zu gelangen, werden beispielsweise Monocarbonate der Formel (V) oder entsprechenden Monoepoxide analog der vorstehend beschriebenen Weise verwendet.

Die Temperatur bei der Umsetzung der Komponenten (α) und (β) beträgt im allgemeinen 20 bis 150, vorzugsweise 50 bis 100°C. Die Umsetzung wird im allgemeinen so lange vorgenommen bis z.B. eine konstante Aminzahl der erfindungsgemäßen Verbindungen erreicht ist.

Die Bedingungen bei der Herstellung der Ausgangskomponenten (siehe auch Abschnitt I bis VII der Beispiele) sowie bei der Herstellung der erfindungsgemäßen Aminourethane (→ Abschnitt VIII der Beispiele), können in weiten Grenzen schwanken. Die hierzu in den Beispielen gemachten Angaben sollen

daher nur richtungsweisend sein. Der Einsatz von geringen Mengen eines geeigneten Katalysators, wie basische Verbindungen, ist bei den Reaktionen auch möglich.

Die erfindungsgemäßen Umsetzungsprodukte werden beispielsweise neben Lackzubereitungen nach herkömmlicher Zusammensetzung auch zur Herstellung von wäßrigen Systemen verwendet, die Überzüge mit sehr guten Eigenschaften ergeben.

Insbesondere sind sie geeignet für die elektrische Abscheidung, obwohl sie auch für andere Überzugsverfahren, beispielsweise für lösungsmittelhaltige Systeme verwendet werden können. Um wäßrige Zubereitungen zu erhalten, ist es vorteilhaft, die basischen Aminogruppen teilweise oder vollständig zu neutralisieren, um damit Überzugsmassen zu erhalten, die aus wäßriger Lösung bei einem Bad-pH zwischen etwa 3 und 9 elektrisch abgeschieden werden können.

Die Neutralisation der basischen Gruppen wird im allgemeinen mit wasserlöslichen Säuren, beispielsweise Ameisensäure, Essigsäure, Milchsäure oder Phosphorsäure vorgenommen. Die Menge der Zugabe der Säure hängt im Einzelfall von den Eigenschaften des verwendeten Harzes ab und wird im allgemeinen nur so weit durchgeführt, daß das Harz solubilisiert oder dispergiert wird.

Wäßrige Zubereitungen, die einen besonders niedrigen Gehalt an flüchtigen organischen Lösungsmittel besitzen, erhält man beispielsweise durch Abdestillation der in den Bindemitteln von der Herstellung oder Lösung enthaltenen Lösungsmittel. Bevorzugt wird dieser Verfahrensschritt unter vermindertem Druck durchgeführt.

Den Lackzubereitungen mit den erfindungsgemäßen Aminourethanen können gegebenenfalls auch verschiedene Zusatzstoffe wie Pigmente, Pigmentpasten, Antioxidantien, oberflächenaktive Mittel, Lösungsmittel, Verlauf- und Verdickungsmittel, Reaktivverdünner, Katalysatoren und dergleichen zugesetzt werden. Diese Zusatzstoffe sind bekannt und werden üblicherweise in der Lackindustrie eingesetzt. Die elektrische Ablagerung der Lackteilchen erfolgt nach bekannten Verfahren, worauf hier Bezug genommen wird. Die Abscheidung kann auf allen elektrisch leitenden Substraten erfolgen, z.B. Metall wie Stahl, Kupfer, Aluminium und dergleichen.

Nach der Abscheidung wird der Überzug bei erhöhten Temperaturen, die im allgemeinen von der Beschaffenheit der Härterkomponente abhängig sind, durch übliche Verfahren gehärtet, wobei Temperaturen von 100 bis 220°, vorzugsweise 130 bis 180° verwendet werden.

In den nachfolgenden Beispielen bedeutet T Gewichtsteile und % Gewichtsprozent. Die Aminzahlen beziehen sich stets auf Festharz.

## Beispiele

I. Teilverkappte Polyisocyanate

*Härtende Verbindungen*

1.) Zu 174 T Toluylendiisocyanat (2 Äquiv. NCO; 80% 2,4-20% 2,6-Isomeres) wurden bei 60—70°C in Gegenwart von 0,3% Benzyltrimethylammoniumhydroxid® (Triton B) als Katalysator 137 T 2-Äthylhexanol (1,05 Äquiv. OH) zugegeben und bis zu einem NCO-Wert von ca. 12,8% umgesetzt.

2.) Beispiel I.1) wurde wiederholt mit dem Unterschied, daß anstelle des 2-Äthylhexanol 94,5 T Äthylenglykolmonoäthyläther (1,05 Äquiv. OH) eingesetzt wurden. Die Umsetzung erfolgte bis zu einem NCO-Wert von 14.8%.

*Nichthärtende Verbindungen*

3.) Zu 348 T Toluylendiisocyanat (4 Äquiv.) in 385 T Diglykoldimethyläther wurden in Gegenwart von 0,3% Triton B als Katalysator 550 T® Capa 200 (2 Äquiv.; Handelsname eines Polycaprolactondiols mit einer mittleren Molmasse von 550) bei 50—70°C langsam zugegeben. Die Reaktionsmischung wurde bei dieser Temperatur bis zu einem NCO-Wert von 9,4% geführt und hatte einen Feststoffanteil von 70%.

4.) Beispiel I.3.) wurde wiederholt mit dem Unterschied, daß anstelle von Capa 200 830 T Capa 205 (2 Äquiv. OH; Handelsname eines Polycaprolactondiols mit einer mittleren Molmasse von 830) eingesetzt wurden. Die Reaktion wurde bis zu einem NCO-Wert von 7,1% geführt.

5.) Beispiel I.3.) wurde wiederholt mit dem Unterschied, daß anstelle von Toluylendiisocyanat 444 T Isophorondiisocyanat (4 Äquiv.) eingesetzt wurden. NCO-Wert = 6,6%.

II. Umsetzungen mit teilverkappten Polyisocyanaten

1.) Zu 540 T Capa 305 (3 Äquivalente OH; Handelsname eines Polycaprolactontriols mit einer mittleren Molmasse von 540) in 628 T Diglykoldimethyläther wurden bei 40—60°C in Gegenwart von 0,3% Triäthylamin als basischem Katalysator 915 T der Verbindung I.1.) (3 Äquiv. NCO) zugegeben und die Reaktion fortgeführt, bis der NCO-Wert auf 0% gesunken war. Das Produkt liegt als 70 %ige Lösung vor.

2.) Zu 134 T Dimethylolpropionsäure (2 Äquiv. OH) wurden bei 30—60°C in Gegenwart von 0,3% Zinkacetylacetonat als Katalysator, 615 T der Verbindung I.1) (2 Äquiv. NCO) langsam zugefügt bis der NCO-Wert auf ca. 0% gesunken war (Säurezahl 75 mg KOH/g. Es ist auch möglich, als Lösungsvermittler etwa 500 T 2-Butanon zuzusetzen, das nach der Umsetzung unter vermindertem Druck wieder abdestilliert werden kann.

3.) Zu 268 T Dimethylolpropionsäure wurden wie unter II.2 beschrieben 615 T der Verbindung I.1) (2 Äquiv. NCO) und 1638 T der Verbindung I.3) (2 Äquiv. NCO, 70 %ig) zugegeben, gegebenenfalls nach Zugabe von 2000 T 2-Butanon als Lösungsvermittler SZ = 54,5.

4.) Beispiel II.3) wurde wiederholt mit 536 T Dimethylolpropionsäure (8 Äquiv. OH), 1842 T der

Verbindung I.1) (6 Äquiv. NCO) und anschließender Zugabe von 1683 T der Verbindung I.3) (2 Äquiv. NCO, 70 %ig) SZ = 63,1.

5.) 236 T Dimethylolpropionsäure (2 Äquiv. OH) wurden wie in Beispiel II.2 mit 1820 T der Verbindung I.5) (2 Äquiv. NCO, 70 %ig) zur Reaktion gebracht. NCO-Wert ca. 0%. Gegebenenfalls Lösungsvermittler: 1000 T 2-Butanon. SZ = 74,3.

In den Beispielen II.2)—II.5) kann anstelle von 2-Butanon auch Diglykoldimethylether als Lösungsvermittler verwendet werden, wobei das Lösemittel nach erfolgter Umsetzung im Reaktionsgut verbleibt. Die Wahl des Lösemittels richtet sich nach den unter IV verwendeten Bedingungen.

6.) Zu 1195 T der Verbindung VI.1) (siehe dort) (2 Äquiv. Carbonat, 2 Äquiv. prim. OH, 80 %ig) wurden bei 40—80°C in Gegenwart von 0,3% Zinkacetylacetonat als Katalysator 1820 T der Verbindung I.5) (2 Äquiv. NCO, 70 %ig) zugefügt und bis zu einem NCO-Wert von ca. 0% umgesetzt. Anschließend wurde mit Diglykoldimethylether auf einen Festkörpergehalt von 70% eingestellt.

Carbonatäquivalentgewicht ca. 1115.

7.) Beispiel II.6) wurde wiederholt mit dem Unterschied, daß 683 T der Verbindung VI.1) (1 Äquiv. Carbonat, 1 Äquiv. prim. OH, 80 %ig) mit 307 T der Verbindung I.1) (1 Äquiv. NCO) zur Reaktion gebracht wurden, bis der NCO-Wert ca. 0% betrug. Carbonatäquivalentgewicht ca. 785. Anschließend wurde mit Diglykoldimethylether auf einen Festkörpergehalt von 70% eingestellt.

8.) Beispiel II.7) wurde wiederholt mit dem Unterschied, daß anstelle der Verbindung VI.1) 1333 T der Verbindung VI.2) (1 Äquiv. Carbonat, 1 Äquiv. prim. OH, 80 %ig) und 921 T der Verbindung I.1) (3 Äquiv. NCO) verwendet wurden. Carbonatäquivalentgewicht ca. 1987.

9.) Beispiel II.7) wurde wiederholt mit dem Unterschied, daß anstelle der Verbindung VI.1) 1195 T der Verbindung VI.3) (1 Äquiv. Carbonat, 80 %ig, 2 Äquiv. prim. OH) und 615 T der Verbindung I.1) (2 Äquiv. NCO) verwendet wurden.

Carbonatäquivalentgewicht ca. 1571.

10.) Zu 540 T Capa 305 (3 Äquiv. OH) in 666 T Diglykoldimethylether wurden bei 40—80°C in Gegenwart von 0,3% Zinkacetylacetonat 615 T der Verbindung I.1) zugegeben und bis zu einem NCO-Wert von ca. 0% umgesetzt. Die noch vorhandenen OH-Gruppen des Reaktionsgemisches wurden anschließend bei 40—80°C mit 222 T Isophorondiisocyanat (2 Äquiv. NCO) bis zu einem NCO-Wert von ca. 3,0% zur Reaktion gebracht. Die erhaltene Lösung wurde bei 10—30°C, gegebenenfalls unter Kühlung zu 176 T 4,7-Dioxadecan-1,10-diamin gegeben und bis zu einer Aminzahl von ca. 36 mg KOH/g (berechnet auf Festharz) umgesetzt. Das erhaltene Produkt hatte einen Feststoffanteil von 70% in Diglykoldimethylether.

11.) Zu 103 T Diethylentriamin (2 Äquiv. prim. Amin) wurden 307 T der Verbindung I.1) (1 Äquiv. NCO) zugegeben und bei 10—30°C gehalten, bis der NCO-Wert auf ca. 0% gesunken war. Aminzahl 275 mg KOH/g (bezogen auf Festharz).

12.) Beispiel II.6) wurde wiederholt, mit dem Unterschied, daß anstelle der Verbindung VI.1) 1165 T der Verbindung VI.4) (2 Äquiv. prim. OH, 2 Äquiv. Carbonat, 80 %ig) verwendet wurde. Carbonatäquivalentgewicht ca. 1103.

Vollverkappte Isocyanate

13.) Zu 186,5 g (0,84 Mol) Isophorondiisocyanat in 149 g Diglykoldimethylether wurden bei 50°C in Gegenwart von 0,35 g Zinkacetylacetonat portionsweise 35,6 g (0,27 Mol) Trimethylolpropan gegeben. Anschließend wurde 3 Std. auf 60°C erwärmt (NCO: 9,6%; Theorie: 9,96%). Zu diesem Reaktionsgemisch wurde bei 60°C 126,1 g (0,88 Mol) 2-Octanonoxim getropft und 2 Std. bei 60°C nachgerührt (NCO:<0,1%).

14.) Zu 130,5 g (0,75 Mol) Toluylendiisocyanat (80% 2,4- und 20% 2,6-Isomeres) in 118 g Diglykoldimethylether wurden bei 50°C in Gegenwart von 0,28 g Zinkacetylacetonat portionsweise 31,8 g (0,24 Mol) Trimethylolpropan gegeben und bis zu einem NCO-Wert von 11,73% (Theorie: 11,79%) umgesetzt. Anschließend wurden zu diesem Reaktionsgemisch bei 60°C 112,6 g (0,79 Mol) 2-Octanonoxim zugetropft und 2 h bei 60°C nachgerührt (NCO:<0,1%).

III. Umsetzungen von Biscarbonaten und teilverkappten Polyisocyanaten

1.) Zu 1759 T eines Biscarbonats auf Basis von ® Epikote 1001 (2 Äquiv. Carbonat; 60 %ig in Diglykoldimethyläther) wurden bei 60—80°C 618 T der Verbindung I.1.) (2 Äquiv. NCO) langsam zugegeben und in Gegenwart von 0,3% triton B bis zu einem NCO-Wert von ca. 0% umgesetzt. Das Produkt ist 70 %ig und hat ein Carbonatäquivalentgewicht von 835.

2.) Beispiel III.1.) wurde wiederholt mit dem Unterschied, daß 1507 T des Bicarbonats auf Basis von Epicote 1001 und 268,5 T der Verbindung I.2.) zugegeben worden. Nach Zusatz von 113 T Diglykoldimethyläther wird eine 70 %ige Lösung des Produktes erhalten. Carbonatäquivalentgewicht: 658.

3.) Zu 5182 T Verbindung VI.7.)(s.d.) (2 Äquiv. Carbonat; 70 %ig Diglykoldimethyläther) wurden bei 60—80°C 1236 T des teilverkappten Toluylendiisocyanats I.1.) (4 Äquiv. NCO) langsam zugegeben und in Gegenwart von 0,3 Titon B bis zu einem NCO-Wert von ca. 0% umgesetzt. Das erhaltene Produkt wurde mit 530 T Diglykoldimethyläther auf 70% Festkörpergehalt eingestellt. Carbonatäquivalentgewicht ca. 2432.

4.) bis 6.) Beispiel III.3.) wurde wiederholt mit dem Unterschied, daß die Verbindung VI.7.) durch die in der nachstehenden Tabelle 1 angeführten Produkte ersetzt wurde.

TABELLE 1

| | Ersatz von VI.7 durch VI.12 | VI.13 | VI.14 | Carbonataquivalentgew. |
|---|---|---|---|---|
| Beisp. 4 | 5582 | | | 2572 |
| Beisp. 5 | | 3654 | | 1897 |
| Beisp. 6 | | | 4054 | 2037 |

7.) zu 2374 T der Verbindung VI.5 (siehe dort) (2 Äquiv. Carbonat, 2 Äquiv. sek. OH, 70 %ig) wurden bei 40—80°C in Gegenwart von 0,3 % Zinkacetylacetonat als Katalysator 307 T der Verbindung I.1) (1 Äquiv. NCO) gegeben und bis zu einem NCO-Wert von ca. 0% umgesetzt. Mit Diglykoldimethylether wurde auf einen Festkörpergehalt von 70% eingestellt. Carbonatäquivalentgewicht 985.

IV. Formaldehyd-Phenoladdukte

1.) 220 T p-Nonylphenol und 129 T 2-Äthylhexylamin wurden unter Kühlung in 182 T Toluol eingebracht. Nach Abklingen der leichten Exotherme wurden 33 T Paraformaldehyd (91%ig) zugegeben und das Reaktionsgut langsam auf 80—100°C erhitzt. Diese Temperatur wurde solange gehalten, bis ca. 20 T Wasser ausgekreist worden sind. Das erhaltene Produkt hatte eine Aminzahl von 155 mg KOH/g. Die Lösung wurde unter vermindertem Druck bei 80—100°C von Toluol befreit und mit Diglykoldimethyläther bis zu einem Feststoffgehalt von 70 % versetzt.

V. Bisphenole

1.) 758 T der Verbindung IV.1.) (2 Äquiv. sek. Amin, 70 %ig) und 1283 T der Verbindung I.3) (2 Äquiv. NCO, 70 %ig) wurden bei 40—60°C gemischt und solange bei dieser Temperatur behandelt, bis die Aminzahl und der NCO-Wert auf Null gesunken sind.

2.) Beispiel V.1.) wurde wiederholt mit dem Unterschied, daß anstelle der Verbindung I.3.) 1683 T der Verbindung I.4.) eingesetzt wurden.

VI. Umsetzungsprodukte Carbonat/Epoxyverbindung und Polyol

1.) 416 T des Monocarbonats von ®Epikote 828 (1 Äquiv. Epoxid) wurden in 310 T Ethylenglykol (5 mol) gelöst und bei 100—150°C unter Verwendung von 0,2% Kaliumjodid bis zu einer Epoxidzahl von ca. 0 umgesetzt. Anschließend wurde überschüssiges Ethylenglykol unter vermindertem Druck abgezogen und der Ansatz mit Diglykoldimethylether auf 80% Festkörpergehalt eingestellt. Carbonatäquivalentgewicht ca. 478.

2.) Beispiel VI.1) wurde wiederholt, wobei als Epoxidkomponente 1210 T des Monocarbonats von ®Epikote 1001 80%ig in Diglykoldimethylether, 1 Äquiv. Epoxid) eingesetzt wurde. Carbonatäquivalentgewicht ca. 1066.

3.) 416 T des Monocarbonats von ®Epikote 828 (1 Äquiv. Epoxid), 540 T Capa 305 und 240 T Diglykoldimethylether wurden auf ca. 60°C erwärmt und portionsweise mit bis zu 0,3% Bortrifluoridetherat versetzt. Anschließend wurde bei 80—150°C bis zu einer Epoxidzahl von ca. 0 umgesetzt. Das erhaltene Produkt ist ca. 80 %ig, Carbonatäquivalentgewicht ca. 956.

4.) Beispiel VI.1) wurde wiederholt, wobei als Epoxidkomponente 404 T eines Monocarbonats auf Basis von ®Denacol EX 920 Handelsname für ein Polyoxypropylenglylkoldiglycidylether der Fa. Nagase, Japan) — 1 Äquiv. Epoxid) eingesetzt wurde. Carbonatäquivalentgewicht ca. 466.

5.) 832 T des Monocarbonats von ®Epikote 828 (2 Äquiv. Epoxid), 830 T Capa 205 und 712 T Diglykoldimethylether wurden gemischt und bei 70—140°C in Gegenwart von ca. 0,3% Bortrifluoridetherat zur Reaktion gebracht, bis eine Epoxidzahl von ca. 0 erreicht wurde. Das 70 %ige Produkt (Diglykoldimethylether) besitzt ein Carbonatäquivalentgewicht von ca. 831.

6.) 832 T des Monocarbonats des Bisphenol-A-Diglycidyläthers (Epoxydzahl 3,9), 2300 T der Verbindung V.1.) (2 Äquiv. Phenol.OH, 70 %ig) und 357 T Diglykoldimethyläther wurden gemischt und bei 70—100°C in Gegenwart von 1% Triäthylamin, bezogen auf Festharz, bis zu einer Epoxydzahl von ca. Null umgesetzt. Das Produkt ist 70 %ig in Diglykoldimethyläther und besitzt ein Carbonatäquivalentgewicht von ca. 1226.

7.) 2510 T des Monocarbonats von Epikote 1001 (Epoxydzahl ca. 1,5, 80 %ig), 2314 T der Verbindung V.1.) (2 Äquiv. Phenol.OH, 70 %ig in Diglykoldimethyläther), und 358T Diglykoldimethyläther wurden gemischt und analog VI.1.) bis zu einer Epoxydzahl von ca. Null umgesetzt. Das 70%ige Produkt in Diglykoldimethyläther besitzt ein Carbonatäquivalentgewicht von ca. 1814.

8.) bis 11.) Beispiel VI.1.) wurde wiederholt mit dem Unterschied, daß die Verbindung V.1.) durch die in der Tabelle 2 angeführten Hydroxylverbindungen unter Verwendung von 0,3% Bortrifluorid-Diethyletherat ersetzt werde. Die dabei erhaltenen Verbindungen besaßen die in der Tabelle 2 wiedergegebenen Carbonatäquivalentgewichte. Die Bezeichnung PG 600 steht für Polyäthylenglykol mit einer mittleren Molmasse von 600.

TABELLE 2

| | Ersatz v. V.1) durch T (2 equiv. OH, 70%ig) | | | | Carbonatäquivalent-gewicht |
|---|---|---|---|---|---|
| | V.2.) | Capa 200 | Capa 205 | PG 600 | |
| Vi. Beisp. 8 | 2714 | | | | 1366 |
| Beisp. 9 | | 786 | | | 691 |
| Beisp. 10 | | | 1186 | | 831 |
| Beisp. 11 | | | | 857 | 716 |

12.) bis 14.) Beispiel VI.2.) wurde wiederholt mit dem Unterschied, daß auch hier die Verbindung V.1.) durch die in Tabelle 3 angeführten Hydroxylverbindungen ersetzt würde.

TABELLE 3

| | Ersatz v. V.1) durch T (2 equiv. OH, 70%ig) | | | Carbonatäquivalent-gewicht |
|---|---|---|---|---|
| | V.2.) | Capa 200 | Capa 205 | |
| VI. Beisp. 12 | 2714 | | | 1954 |
| Beisp. 13 | | 786 | | 1279 |
| Beisp. 14 | | | 1186 | 1419 |

VII. Umsetzungsprodukte Carbonat/Epoxidverbindung und Carbonsäure

1.) 1510 T Der Verbindung II.5) (2 COOH-Äquiv.) und 832 T eines Monocarbonats auf Basis von ®Epikote —28 (2 Aquiv. Epoxid) wurden in Gegenwart von 0,2—0,4% Cordova Accelerator AMC-2 als Katalysator bei 50—100°C bis zu einer Epoxidzahl von ca. 0 und einer SZ < 5 mg KOH/g FH umgesetzt. Gegebenenfalls können ca. 1000 T 2-Butanon als Lösungsvermittler zugesetzt werden, die anschließend unter vermindertem Druck wieder abgezogen werden. Das Reaktionsprodukt wurde mit ca. 1000 T Diglykoldimethylether auf einen Festkörpergehalt von 70% eingestellt. Die Reaktion kann auch in Gegenwart von Diglykoldimethylether mit 70% Festkörperanteil unter Einsatz von 0,5—1% Triethlamin bei 70—140°C bis zum Erreichen der oben angeführten Kennzahlen durchgeführt werden. Carbonatäquivalentgewicht ca. 1171.

2.) 3652 T der Verbindung II.3) (4 Äquiv. COOH) und 1664 T eines Monocarbonats auf Basis von ® Epikote 828 (4 Äquiv. Epoxid) wurden entsprechend Beispiel VII.1) umgesetzt. Epoxidzahl ca. 0, SZ< 5 mg KOH/g.

3.) Bei einer Wiederholung des Beispiels VII.2) wurde die Verbindung II.3) durch ein Gemisch aus 2157 T der Verbindung II.2) (2 Äquiv. COOH) und 1498 T der Verbindung VII.1) (2 Äquiv. COOH) ersetzt. Carbonatäquivalentgewicht ca. 1329.

VIII. Herstellung der Bindemittel

1.1.) Keine Beispiele der Erfindung zu 1497 T eines Biscarbonats auf Basis von Epikote 1001 (2 Äquiv. Carbonat, 70 %ig in Diglykoldimethyläther) wurden 204 T N,N-Dimethylaminopropylamin bei 60—80° zugegeben und bis zum Erreichen einer Aminzahl von 89,5 mg KOH/g zur Reaktion gebracht. Das erhaltene Produkt wurde mit Methoxypropanol auf einen Festkörpergehalt von ca. 70% eingestellt.

1.2.) keine Beispiele der Erfindung 63 T der Verbindung VIII.1.1.) wurden mit 37 T der Verbindung II.1.) vermischt. Nach Zugabe von 5 T Dibutylzinndilaurat und 366 T deionisiertem Wasser wurde eine 15 %ige Klarlacklösung erhalten bei einem MEQ-Wert von 70 (mmol Ameisensäure/100 g Festharz), die einen pH-Wert von 6 besaß. Die Klarlacklösung ergab bis zu 250 Volt einen abscheidbaren Film, der bei 160°C für 20 Minuten ausgehärtet wurde und eine Schichtdicke von 20 µm bei einer Abscheidespannung von 200 Volt aufwies.

2.1) keine Beispiele der Erfindung Zu x T Diamin (2 Äquivalent primär. Amin) wurden 305 T des teilverkappten Polyisocyanats I.1.) (1 Äquiv. NCO) zugegeben und bei 20—40°C gehalten, bis der NCO-Wert auf 0% gesunken war.

TABELLE 4

| Amin | x(T) | Aminzahl (mg KOH/g Festharz) |
|---|---|---|
| 2.1.1. Diethylentriamin | 103 | 275 |
| 2.1.2 Triethylentetramin | 146 | 373 |

2.2.) keine Beispiele der Erfindung zu 4737 T der Verbindung III.1.) (4 Äquiv. Carbonat) und 1974 T der Verbindung VI.9.) (2 Äquiv. Carbonat) wurden bei 60—80°C 352 T 4,7-Dioxadecan-1,10-diamin (4 Äquiv. prim. Amin), 408 T der obigen Verbindung 2.1.1) (1 Äquiv. prim. Amin) und 451 T des obigen Produkts 2.1.2) (1 Äquiv. prim. Amin) zugegeben und bis zu einer Aminzahl von 28,5 mg KOH/g Festharz zur Reaktion gebracht. Anschließend wurde mit Methoxypropanol auf einen Festkörpergehalt von 70% eingestellt. Durch Zugabe von deionisiertem Wasser ließ sich daraus eine 15 %ige Klarlacklösung bei einem MEQ-Wert von etwa 20 mit einem pH-Wert von 6,6 herstellen. Die Aufbruchsspannung war kleiner als 450 Volt, bei 300 Volt Abscheidespannung wurde eine Schichtdicke von 20 µm erreicht, die während 20 Minuten bei 160°C zu einer harten Lackschicht eingebrannt wurde.

3.) Zu 4123 T der Verbindung III.2.) (4,4 Äquiv. Carbonat) und 304 T eines Carbonats auf Basis Versaticsäureglycidylester (1 Äquiv. Carbonat) wurden bei 60—80°C 206 T Diäthylentriamin zugegeben, bis die Aminzahl 33 mg KOH/g Festharz betrug. In diese Lösung wurde eine Mischung, bestehend aus 102 T N,N-Dimethylaminopropylamin und 262 T Methoxypropanol, unter Einhaltung der oben angeführten Temperatur einlaufen gelassen. Die Aminzahl betrug 48,1 mg KOH/g Festharz; Festkörpergehalt 70%. MEQ-Wert 50 (15 %ige Klarlacklösung) pH-Wert 6,1.

4.) Zu 4123 T der Verbindung III.2.) (4,4 Äquiv. Carbonat) und 608 T eines Carbonats auf Basis von Versaticsäureglycidylester (2 Äquiv. Carbonat) wurden bei 60—80°C 438 T Triäthylentetramin gegeben und die Reaktion bis zu einer Aminzahl von 86 mg KOH/g Festharz geführt. Das Reaktionsgut wurde mit Methoxypropanol auf einen Fest körpergehalt von 60% eingestellt. MEQ-Wert 50 (15 %ige Klarlacklösung), pH 6. Die Aufbruchsspannung betrug 400 Volt und bei einer Abscheidungsspannung von 200 Volt wurde eine Trockenfilmschichtstärke von 12 bis 14 µm erhalten (bei 160°C für 20 Minuten eingebrannt).

5.) kein Beispiel der Erfindung zu 2370 T der Verbindung III.1.) (2 Äquiv. Carbonat) wurden 204 T N,N-Dimethylaminopropylamin bei 60—80°C gegeben und die Reaktion bis zu einer Aminzahl von ungefähr 60 mg KOH/g Festharz fortgeführt. Das Reaktionsgut wurde mit Methoxypropanol auf einen Festkörpergehalt von 60% eingestellt. MEQ-Wert 50 (15 %ige Klarlacklösung) pH-Wert von 7,3, Aufbruchsspannung ungefähr 250 Volt.

6.) Zu 4740 T der Verbindung III.1.) (4 Äquiv. Carbonat) wurden 204 T N,N-Dimethylaminopropylamin und 103 T Diäthylentriamin bei 60—80°C zugegeben. Das erhaltene Festharz hatte eine Aminzahl von 46 mg KOH/g Festharz und wurde mit Methoxypropanol auf einen Festkörpergehalt von 60% eingestellt. MEQ-Wert 65 (15 %ige Klarlacklösung) pH-Wert 6,8 Aufbruchspannung 300 Volt.

7.) Zu 2620 T (80% Feststoff) eines Biscarbonats auf Basis von Epikote 1001 (ca. 4 Äquiv. Carbonat) wurden 204 T N,N-Dimethylaminopropylamin und 103 T Diäthylentriamin bei 60—80°C gegeben und umgesetzt. Aminzahl ca. 70 mg KOH/g Festharz. Der Ansatz wurde mit Methoxypropanol auf einen Festkörpergehalt von 60% eingestellt. MEQ-Wert 75 (15 %ige Klarlacklösung) pH-Wert 7, Aufbruchspannung etwa 300 Volt.

8.) 6948 T der Verbindung III.1.) (2 Äquiv. Carbonat) und 608 T des Carbonats auf Basis des Versaticsäureglycidylesters (2 Äquiv. Carbonat) in 453 T Methoxypropanol wurden vorgelegt und auf ca. 80°C erhitzt. Zu dieser Lösung wurde eine Mischung aus 272 T Pentaäthylenhexamin (2 Äquiv. prim. Amin, 85 %ig entsprechend Aminzahl) und 176 T 4,7-Dioxadekan-1,10-diamin (2 Äquiv. prim. Amin) langsam zugegeben und während 4 bis 8 Stunden bis zu einer Aminzahl von 38 m KOH/g umgesetzt. Das Produkt hat einen Festkörpergehalt von 70% in einer 9:2 Mischung aus Diglykol-dimethyläther/Methoxypropanol. Der MEQ-Wert betrug 35 und der pH-WErt 6,2.

9.) bis 11.) Beispiel 8.) wurde wiederholt mit den in Tabelle 5 angegebenen Mengen der Verbindungen III.4.) bis III.6.).

TABELLE 5

| | III.4 | III.5 | III.6 | Aminz. | MEQ | pH | LM-Verhältnis |
|---|---|---|---|---|---|---|---|
| Beisp. 9 | 7348 | | | 36.2 | 30 | 6,4 | 5:1 |
| Beisp. 10 | | 5420 | | 46.3 | 35 | 6,1 | 7:2 |
| Beisp. 11 | | | 5820 | 44 | 30 | 6,5 | 4:1 |

12.) 3503 T der Verbindung VI.6) (3 Äquiv. Carbonat), 4754 T der Verbindung III.1.) (4 Äquiv. Carbonat), 608 T des Carbonats des Versaticsäureglycidylesters (2 Äquiv. Carbonat) und 600 T Methoxypropanol wurden vorgelegt und auf ca. 80°C erhitzt. Zu dieser Lösung wurde eine Mischung aus 440 T Tetraäthylenpentamin (TEPA) (4 Äquiv. prim. Amin), 85 %ig entsprechend Aminzahl) und 352 T 4,7-Dioxadecan-1,10-diamin (4 Äquiv. prim. Amin) langsam zugegeben und während 8 Stunden bis zu einer Aminzahl von 51 mg KOH/g umgesetzt. Das produkt besaß einen Festkörpergehalt von 70% in Diglykoldimethyläther/Methoxypropanol (4:1). MEQ-Wert 25, pH-Wert 6.

13.) bis 15.) Beispiel 12 wurde wiederholt mit dem Unterschied, daß anstelle der Verbindung VI.6.) und des Tetraäthylenpentamins die in der Tabelle 6) angeführten Verbindungen eingesetzt wurden.

TABELLE 6

| | VI.8 | VI.9 | VI.10 | PEHA | TEPA | TETA | Aminz. | MEQ | pH |
|---|---|---|---|---|---|---|---|---|---|
| Beisp. 13 | 3905 | | | | 440 | | 45 | 30 | 6.0 |
| Beisp. 14 | | 1975 | | 272 | | 146 | 55.3 | 25 | 6.2 |
| Beisp. 15 | | | 2375 | 272 | | 146 | 52.8 | 25 | 6.1 |

PEHA = Pentaethylenhexamin TETA = Triethylentetramin

16.) Zu 4757 T der Verbindung III.1.) (4 Äquiv. Carbonat) und 608 T des Carbonats des Versaticsäureglycidylesters (2 Äquiv. Carbonat) wurde eine Mischung von 220 T TEPA (2 Äquiv. prim. Amin, 85 %ig entsprechend Aminzahl) und 352 T 4,7-Dioxadecan-1,10-diamin (4 Äquiv.prim. Amin) bei 80°C zugesetzt. Die Umsetzung wurde bis zu einer Aminzahl von 37,4 mg KOH/g geführt und der Ansatz mit 506 T Methoxypropanol auf einen Festkörpergehalt von 70% eingestellt. MEQ-Wert 25—30, pH 6,4.

17.) Zu 2377 T der Verbindung III.1.) (2 Äquiv. Carbonat) 460 T des Biscarbonats des Bisphenol A-Diglycidyläthers (2 Äquiv. Carbonat) und 608 T des Carbonats des Versaticsäureglycidylesters wurde eine Mischung von 220 T TEPA (2 Äquiv. prim. Amin) und 352 T 4,7-Dioxadecan-1,10-diamin (4 Äquiv. prim. Amin) bei 80°C zugesetzt. Die Umsetzung wurde bis zu einer Aminzahl von 51 mg KOH/g geführt und der Ansatz mit 703 T Methoxypropanol auf einen Festkörpergehalt von 70% eingestellt. MEQ 30—35, pH 6,3.

18.) Zu 818 T der Verbindung VI.11.) (0,8 Äquiv. Carbonat) 6504 T der Verbindung III.5.) (1,2 Äquiv. Carbonat) und 608 T des Carbonats des Versaticsäureglycidylesters (2 Äquiv. Carbonat) wurden bei 80°C 292 T TEPA (4 Äquiv. prim. Amin) zugegeben. Die Umsetzung wurde bis zu einer Aminzahl von 37 mg KOH/g geführt und der Ansatz mit 386 T Methoxypropanol auf einen Festkörpergehalt von 70% eingestellt. MEQ-Wert 30, pH 6,5.

19.) Zu 4489 T der Verbindung II.9) (2 Äquiv. Carbonat, 70 %ig), 2374 T der Verbindung VI.5 (2 Äquiv. Carbonat, 70 %ig) und 2376 T der Verbindung III.1) (2 Äquiv. Carbonat, 70 %ig) wurde eine Lösung von 352 T 4,7-Dioxadecan-1,10-diamin (4 Äquiv. prim. Amin), 210 T Tetraethylpentamin (2 Äquiv. prim. Amin) und 272 T Pentaethylhexamin (2 Äquiv. prim. Amin) in 2700 T Methoxypropanol langsam einlaufen gelassen und bei 60—90°C bis zu einer Aminzahl von ca. 44 mg KOH/g umgesetzt. Das erhaltene Bindemittel besitzt einen Feststoffanteil von 70%.

20.) 1664 T eines Monocarbonats auf Basis von ®Epicote 828 (4 Äquiv. Epoxid), 1080 T Capa 305 (6 Äquiv. prim. OH), 830 T Capa 205 (2 Äquiv. prim. OH) und 1507 T Diglykoldimethylether wurden gemischt und bei 70—140°C in Gegenwart von ca. 0,3% Bortrifluoridetherat zur Reaktion gebracht, bis eine Epoxidzahl von ca. 0 erreicht wurde. Zu dieser Lösung wurde unter 40—80°C unter Verwendung von 0,3% Zinkacetylacetonat 2456 T I.1) (8 Äquiv. NCO) bis zu einem NCO-Wert von ca. 0% einreagiert. In diese Mischung wurden 2620 T eines Biscarbonats auf Basis von ®Epicote 1001 (4 Äquiv. Carbonat, 80 %ig, in Diglykoldimethylether) eingebracht und bei 60—90°C 210 T Tetraethylenpentamin (2 Äquiv. prim. Amin), 272 T Pentaethylenhexamin (2 Äquiv. prim. Amin) und 352 T 4,7-Dioxadecan-1,10-diamin (4 Äquiv. prim. Amin) in 1809 T Methoxypropanol einlaufen gelassen. Die Reaktion wurde bis zu einer Aminzahl von ca. 44 mg KOH/g geführt, Feststoffanteil 70%.

21.) Zu einer Mischung bestehend aus 4779 T der Verbindung II.6) (3 Äquiv. Carbonat, 70%ig), 1576 T der Verbindung II.12) (1 Äquiv. Carbonat, 70 %ig) und 2665 T der Verbindung VI.2) (2 Äquiv. Carbonat, 80 %ig) wurden bei 40—80°C in Gegenwart von 0,3% Zinkacetylacetonat 2440 T der Verbindung I.1) (8 Äquiv. NCO) einlaufen gelassen und bis zu einem NCO-Wert von ca. 0% umgesetzt. Zu dieser Lösung wurde eine Mischung von 210 T Tetraethylenpentamin (2 Äquiv. prim. Amin), 174 T N,N'-Diaminopropylethylendiamin (2 Äquiv. prim. Amin), 131 T Dipropylentriamin (2 Äquiv. prim. Amin) und 1646 T Methoxypropanol bei 60—90°C einlaufen gelassen und die Umsetzung bis zu einer Aminzahl von 35,3 mg KOH/g geführt. Feststoffanteil 70%.

22.) Zu einer Mischung aus 3346 T der Verbindung VII.1) (2 Äquiv. Carbonat, 70%ig), 4737 T der Verbindung III.1) (4 Äquiv. Carbonat, 70 %ig) und 608 T eines Carbonats auf Basis von Versaticsäureglycidylester (2 Äquiv. Carbonat) wurde bei 60—90°C eine Mischung von 420 T Tetraethylenpentamin (4 Äquiv. prim. Amin), 352 T 4,7-Dioxadecan-1,10-diamin (4 Äquiv. prim. Amin) und

591 T Methoxypropanol einlaufen gelassen und bis zu einer Aminzahl von ca. 48,5 mg KOH/g zur Reaktion gebracht, Feststoffanteil 70%.

23.) Zu einer Mischung aus 2839 T der Verbindung II.8) (1 Äquiv. Carbonat, 70%ig), 1121 T der Verbindung II.7) (1 Äquiv. Carbonat, 70%ig), 594 T der Verbindung III.1) (0,5 Äquiv. Carbonat, 70%ig) und 2815 T der Verbindung III.7) (2 Äquiv. Carbonat, 70%ig) wurde bei 60—90%C eine Mischung von 210 T Tetraethylenpentamin (2 Äquiv. prim. Amin), 104 T Diethylentriamin (2 Äquiv. prim. Amin), 250 T Bis(3-aminopropyl-)polytetrahydrofuran 1100 (0,5 Äquiv. prim. Amin) und 242 T Methoxypropanol einlaufen gelassen und die Reaktion bis zu einer Aminzahl von ca. 30 mg KOH/g geführt, Feststoffanteil 70%.

24.) Zu einer Mischung aus 2377 T der Verbindung III.1) (2 Äquiv. Carbonat, 70%ig), und 2374 T der Verbindung VI.5) (2 Äquiv. Carbonat, 70 %ig) wurde bei 60—90°C eine Mischung, bestehend aus 210 T Tetraethylpentamin (2 Äquiv. prim. Amin), 410 T II.10) (1 Äquiv. prim. Amin), 2218 T II.9) (1 Äquiv. prim. Amin, 70%ig) und 266 T Methoxypropanol einlaufen gelassen und der Ansatz bis zu einer Aminzahl von ca. 40,8 mg KOH/g umgesetzt, Feststoffgehalt 70%.

25.) kein Beispiel der Erfindung. Zu einer Mischung aus 7594 T VII.2) (4 Äquiv. Carbonat, 70%ig), 1310 T eines Biscarbonats auf Basis von ®Epikote 1001 (2 Äquiv. Carbonat, 80 %ig in Diglykoldimethylether) wurde bei 60—90°C eine Mischung, bestehend aus 210 T Tetraethylenpentamin (2 Äquiv. prim. Amin), 176 T 4,7-Dioxadecan-1,10-diamin (2 Äquiv. prim. Amin), 131 T Dipropylentriamin (2 Äquiv. prim. Amin) und 14 T Methoxypropanol einlaufen gelassen und der Ansatz bis zu einer Aminzahl von ca. 33 mg KOH/g umgesetzt, Feststoffanteil 70%.

26.) kein Beispiel der Erfindung. 860 T Bishexamethylentriamin (8 Äquiv. $NH_2$) wurden in 2315 T Methoxypropanol vorgelegt. Zu dieser Lösung wurden bei 20—40°C 622 T der Verbindung I.1) (2 Äquiv. NCO) einlaufen gelassen und bis zu einem NCO-Wert von ca. 0% umgesetzt. Dann wurden 4737 T der Verbindung III.1) (4 Äquiv. Carbonat, 70%ig Diglykoldimethylether), 3246 T der Verbindung III.7) (2 Äquiv. Carbonat, 70 %ig Diglykoldimethylether) zugegeben und bei 60—90°C bis zu einer Aminzahl von ca. 32 mg KOH/g zur Reaktion gebracht, Feststoffanteil 60%.

27.) Herstellung eines nicht selbsthärtenden Bindemittels A) 830 T Capa 205 (2 Äquiv. OH), 3,4 T Triethylamin, 486 T Diglykoldimethylether und 304 T (2 Mol) Tetrahydrophthalsäureanhydrid wurden gemischt und langsam auf 80°C bis 120°C erhitzt. Die Reaktion wurde bis zu einer Säurezahl von 100 bis 105 geführt. In dieser Lösung wurden 832 T (2 Äquiv. Epoxid) eines Monocarbonats auf basis ®Epikote 828 zugefügt und in Gegenwart von AMC-2 auf 100—128°C erwärmt, bis die EP-Zahl ~ 0 und die SZ<1 waren.

B) 2.452 T des Produkts A (80% in Diglykoldimethylether), 608 T (2 Äquiv. Carbonat) eines Monocarbonats auf Basis von Versaticsäureglycidylether, 4.491 T (6 Äquiv. Carbonat) eines Biscarbonats auf Basis von ®Epikote 1001, 1.075 T Bishexamethylentriamin (10 Äquiv. primär. Amin) wurden mit 1.482 T 1-Methoxypropanol-2 versetzt und so lange bei 50—90°C erwärmt, bis eine Aminahl von 41—42 erreicht war.

IX. Prüfung der erfindungsgemäß hergestellten Bindemittel

1.) Aus den Bindemitteln gemäß der Beispiele VIII. 1, 2, 5, 6 und 8—26 wurden Klarlacke gemäß folgenden Ansatz hergestellt:

Ansatz A: 286 T der entsprechenden Harzlösung X g 10 %ige wäßrige Ameisensäure entsprechend Tabelle 7, berechnet auf Festharz, 10 T Dibutylzinndilaurat Einbrennbedingungen: 20 Minuten bei 160°C

Die Ansätze wurden jeweils mit deionisiertem Wasser auf einen Festkörpergehalt von ca. 15% eingestellt. Die Abscheidedauer betrug jeweils 2 Minuten, die dafür benötigten Spannungen, die erzielte Filmdicke und die erzielten Eigenschaften sind in den Tabellen 7 und 7a angeführt.

Pigmentzusammensetzung

2.) Aus den Bindemitteln entsprechend den Beispielen VIII.12.), 14.), 15.), 17.), 19.), 20.) und 26.) wurden pigmentierte Lacke gemäß folgenden Ansätzen hergestellt:

Ansatz B: 84 T Titandioxyd (®Kronos RN59 der Fa. Cronus-Titangesellschaft, Leverkusen)
1 T Ruß (Raven 1170 der Firma Columbian Carbor., Hamburg)
10 T Aluminiumsilica (®Lanco ASP 200 der Firma Langer, Ritterhude)
5 T Bleisilikat (EP 202 der Firma Chemag Frankfurt/Main)

100 T

Die angeführten Harze wurden mit Methoxypropyl auf einen Festkörpergehalt von 60% eingestellt und mit der angeführten Pigmentmischung gemäß Schema B auf einem Dreiwalzenstuhl bis zu einer Korngröße von < 5 µm angerieben. Von den Beispielen 12.) bis 17.) wurden zwei Bindemittelmischungen mit unterschiedlicher Pigmentierungshöhe (d.h. Verhältnis Festharz:Pigment in Gewichtsteilen) gemäß den Zusammensetzungen C und D hergestellt, die jeweils mit deionisiertem Wasser auf einen Festkörpergehalt von ca. 18% verdünnt wurden. Die Beispiele 19.), 20.) und 26.) wurden nur zu Bindemittelmischungen gemäß Zusammensetzung D eingestellt.

C) 750 T Bindemittel gemäß Tabelle 8
90 T Pigmentmischung gemäß Schema B
22,5 T Dibutylzinndilaurat

X T 10 %ige Ameisensäure gemäß Tabelle 8
D) 750 T Bindemittel gemäß Tabelle 8
180 T Pigmentmischung gemäß Schema B
22,5 T Dibutylzinndilaurat
X T 10 %ige Ameisensäure gemäß Tabelle 8
9 T eines Antikrater-Additivs (®Additol VXW 4922/280 der Firma Hoechst AG)
9 T Verlaufmittel (Additol VXL 1359 der Fa. Hoechst AG)
18 T 2,2,4-Trimethylpentandiol-1,3-monoisobutyrat

3.) Das Bindemittel gemäß Beispiel VIII. 27.) wurde mit den vollverkappten Isocyanaten II.13) bzw. II.14) im Verhältnis 70:30 gemischt und in Gegenwart von 1% Blei als Bleioctoat oder 1% Zinn als Dibutylzinndilaurat auf Bonder-Blechen 132 aufgezogen. Die Lackfilme wurden bei 130, 140 und 150°C eingebrannt und auf ihre Beständigkeit gegen Aceton geprüft. Ein Lackfilm kann als vollständig gehärtet angesehen werden, wenn der Film mindestens 1 Min. gegenüber Aceton beständig ist.

Aceton-Test
Einbrenntemperatur

| Bindemittel/Härter | 130°C | | 140°C | | 150°C | |
|---|---|---|---|---|---|---|
| 70:30 | Blei | Zinn | Blei | Zinn | Blei | Zinn |
| 27/1 | 15s | <15s | >1min | 15s | >1min | >1min |
| 27/2 | >1min | >1min | >1min | >1min | >1min | >1min |

Tabelle 7

| eingesetztes Harz VIII. | 1 | 2 | 5 | 6 | 8 | 9 | 10 | 11 | 12 | 13 | 14 | 15 | 16 | 17 | 18 |
|---|---|---|---|---|---|---|---|---|---|---|---|---|---|---|---|
| Ameisensäure, 10%ig (T) | 46 | 46 | 46 | 60 | 32,2 | 27,6 | 32,2 | 27,6 | 27,6 | 23, | 27,6 | 27,6 | 23, | 27,6 | 27,6 |
| Bad-pH | 6,0 | 6,0 | 7,3 | 6,8 | 6,2 | 6,4 | 6,1 | 6,5 | 5,0 | 5,8 | 6,1 | 6,3 | 6,6 | 6,4 | 6,6 |
| max.Aufbruchspannung (V) | 300 | 400 | 250 | 300 | 400 | 450 | 400 | 450 | 500 | 500 | 500 | 500 | 500 | 350 | 300 |
| Abscheidespannung (V) 1) | 200 | 300 | 150-200 | 200 | 300 | 300 | 300 | 300 | 400 | 400 | 400 | 400 | 400 | 250 | 200 |
| Filmdicke (µm) | 10-15 | 12-14 | 10-15 | 10-12 | 15 | 12 | 15 | 14 | 15 | 12 | 16 | 21 | 10 | 12 | 20 |
| Verlauf 2) | 4-5 | 3-4 | 4-5 | 3-4 | 1-2 | 1 | 2 | 2 | 1 | 1-2 | 0-1 | 0-1 | 2 | 1-2 | 2 |
| Haftung 2) | 4-5 | 2-3 | 4-5 | 2-3 | 0 | 0 | 0 | 0 | 0 | 0 | 0 | 0 | 0 | 0 | 0 |
| Vernetzung 3) | 40-80 | 80-100 | 20-60 | 80-100 | 80-10 | 80-100 | 100 | 100 | 100 | 100 | 100 | 100 | 60-80 | 60-80 | 80 |
| Schlagtiefung 4) | 60 | 60 | 20 | 60 | 140 | 160 | 160 | 160 | 160 | 140 | 160 | 140 | 140 | 120 | 120 |

1) bis 28-30°C

2) 0 bester Wert
5 schlechtester Wert

3) MEK-Doppelhülle,
1 kg Auflage

4) inch-pound

Tabelle 7a

| eingesetztes Harz | VIII. 19 | 20 | 21 | 22 | 23 | 24 | 25 | 26 |
|---|---|---|---|---|---|---|---|---|
| Ameisensäure, 10%ig (T) | 27,6 | 19 | 23 | 19 | 23 | 19 | 32,2 | 46 |
| Bad-pH | 6,0 | 6,5 | 6,7 | 6,8 | 5,5 | 6,7 | 4,5 | 5,5 |
| max. Aufbruchspannung (V) | 400 | 350-400 | 200-250 | 200-250 | 300 | 300-350 | 200-250 | 400 |
| Abscheidespannung (V) [1] | 300 | 300 | 200 | 200 | 250 | 250 | 200 | 200 |
| Filmdicke (µm) | 30 | 25 | 17 | 18 | 20 | 20 | 25 | 20 |
| Verlauf [2] | 0 | 0 | 2 | 2 | 0 | 0 | 3 | 0 |
| Haftung [2] | 0 | 0 | 2 | 3 | 0 | 0 | 2 | 0 |
| Vernetzung [3] | 100 | 100 | 100 | 100 | 100 | 100 | 100 | 100 |
| Schlagtiefung [4] | 160 | 160 | 140 | 100 | 140 | 160 | 140 | 160 |

1) - 4) s. Tabelle 7

Tabelle 8

| eingesetztes Harz, VII | Pigmentierungshöhe 1 : 0,2 | | | | | 1 : 0,4 | |
|---|---|---|---|---|---|---|---|
| Beispiel | 12 | 14 | 15 | 17 | 19 | 20 | 26 |
| Ameisensäure, 10%ig (T) | 7,04 | 6,56 | 5,20 | 5,20 | 27,6 | 27,6 | 46 |
| Bad-pH | 5,2 | 6,3 | 5,6 | 5,8 | 6,0 | 6,0 | 5,7 |
| max. Aufbruchspannung (V) | >500 | 400 | >500 | >500 | 350-400 | 400 | 400 |
| Abscheidespannung (V) 1) | 400 | 350 | 400 | 400 | 300 | 300 | 300 |
| Filmdicke (µm) | 15 | 25 | 23 | 22 | 0 | 25 | 22 |
| Verlauf 2) | 0-1 | 1-2 | 0 | 0 | 0 | 0 | 0 |
| Haftung 2) | 0-1 | 0-1 | 0 | 0 | 0 | 0 | 0 |
| Vernetzung 3) | >100 | >100 | >100 | >100 | >100 | >60* | >100 |
| Schlagtiefung 4) | 4 | 10 | 40 | 40 | 80-120 | 10 | 80-120 |
| Erichsen-Tiefung (mm) | 8,0 | 7,0 | 10 | 9,3 | 8-9 | 7,5-8,5 | 8-9 |

1)-4) s. Tabelle 7

* Wert: >100 bei 180°C, 20 min

**Patentansprüche für die Vertragsstaaten: BE CH DE FR GB GR IT LI LU NL SE**

1. Selbstvernetzende Aminourethane bestehend im wesentlichen aus Struktureinheiten, die sich ableiten von (α) Polyaminen der algemeinen Formel(I)

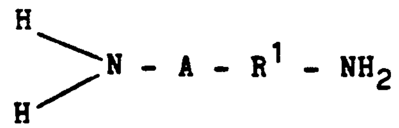

(I),

in der bedeuten

$R^1$ einen zweiwertigen Kohlenwasserstoffrest, vorzugsweise einen geradkettigen oder verzweigten Alkylenrest mit 2 bis 18 C-Atomen, und

A eine chemische Bindung oder $—(R^1—NH)_r—R^1—NH—$, worin r Null oder eine ganze Zahl von 1 bis 6 ist und $R^1$ die vorstehende Bedeutung hat,

Struktureinheiten, die sich ableiten von (β) oligomeren ode polymeren Verbindungen, die mindestens zwei endständige 2-Oxo-1,3-dioxolan-Gruppen und zumindest teilweise zusatzlich Urethan-Grüppen enthalten, und Struktureinheiten, die sich ableiten von Polyaminen (8) der allgemeinen Formel (I')

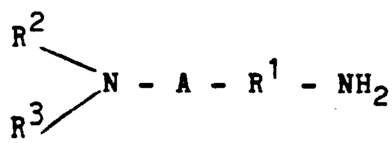

(I'),

in der bedeuten

$R^1$ und A gleiche Bedeutung wie in obiger Formel (I),

$R^2$ und $R^3$ Alkyl mit 1 bis 8 C-Atomen oder Hydroxyalkyl mit 1 bis 8 C-Atomen im Alkylrest oder

$R^2$ und $R^3$ zusammen eine cyclische Ringverbindung oder

$R^2$ Wasserstoff und $R^3$ mindestens einer der Reste a) bis d)

a) 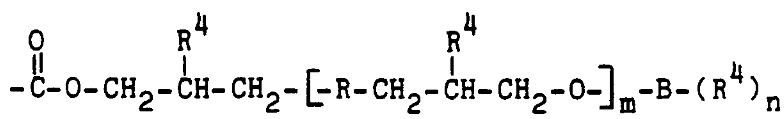

in dem R gleich oder verschieden sein kann und für den Rest eines Diglycidylethers oder -esters, der gegebenenfalls auch $(NR^2)$-Gruppen enthalten kann, wobei $R^2$ die obige Bedeutung hat, oder für einen zweiwertiger Kohlenwasserstoffrest mit 2 bis 18 C-Atomen steht, $R^4$ Hydroxyl oder den Rest

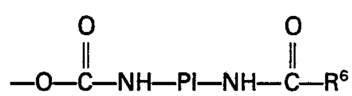

darstellt, in dem PI der Rest eines Polyisocyanats und $R^6$ der Rest eines aliphatischen, cycloaliphatischen oder alkylaromatischen einwertigen Alkohols, eiens Aminoalkohols, eines Ketoxims, einer CH— oder NH-aciden Verbindung ist, B den Rest eines Polyols bedeutet, m eine ganze Zahl von 1 bis 3 und n eine ganze Zahl von 1 bis 6 ist, oder

b) 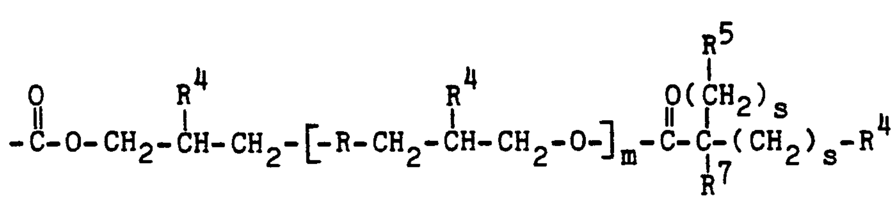

in dem R, $R^4$ und m die schon genannte Bedeutung haben, $R^5$ Wasserstoff oder $R^4$ ist, $R^7$ einen Alkylrest mit 1 bis 8, C-Atomen bedeutet und s eine ganze Zahl von 1 bis 6 darstellt, oder

c) 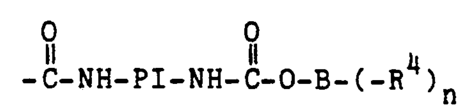 oder

d) die Gruppen $R^8—CHOH—CH_2—O—CO—$ oder $R^8—CHOH—CH_2$, in denen $R^8$ Wasserstoff, Alkyl mit 1

bis 18 C-Atomen oder Reste von Glydicylestern oder -ethern, vorzugsweise Versaticsäureglycidylestern ist oder die Gruppe

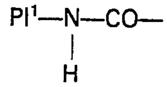

darstellt, in der $Pl^1$ der Rest eines teilverkappten Polyisocyanats ist.

2. Aminourethane bestehend im wesentlichen aus Struktureinheiten, die sich ableiten von (α) Polyaminen der allgemeinen Formel (I) Gegenstand der Erfindung sind selbstvernetzende Aminourethane bestehend im wesentlichen aus Struktureinheiten, die sich ableiten von (α) Polyaminen der allgemeinen Formel (I)

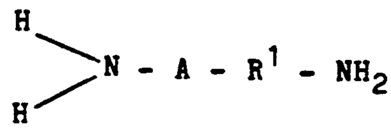
(I),

in der bedeuten

$R^1$ einen zweiwertigen Kohlenwasserstoffrest, vorzugsweise einen geradkettigen oder verzweigten Alkylenrest mit 2 bis 18, vorzugsweise 2 bis 4 C-Atomen, und

A eine chemische Bindung oder $-(R^1-NH)_r-R^1-NH-$, worin r Null oder eine ganze Zahl von 1 bis 6, vorzugsweise 1 bis 3, ist und $R^1$ die vorstehende Bedeutung hat,

Struktureinheiten, die sich ableiten von (β) oligomeren oder polymeren Verbindungen, die mindestens zwei, vorzugsweise zwei bis fünf endständige 2-Oxo-1,3-dioxolan-Gruppen (=cyclische Carbonatgruppen) und zumindest teilweise zusatzlich Urethangruppen enthalten, und Struktureinheiten, die sich ableiten von Polyaminen (γ) der allgemeinen Formel (I')

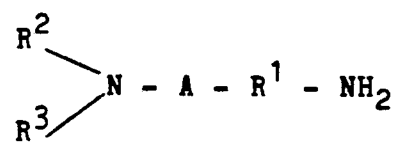
(I'),

in der bedeuten

$R^1$ und A gleiche Bedeutung wie in obiger Formel (I),

$R^2$ und $R^3$ Alkyl mit 1 bis 8, vorzugsweise 1 bis 4 C-Atomen oder Hydroxyalkyl mit 1 bis 8, vorzugsweise 1 bis 2 C-Atomen im Alkylrest oder

$R^2$ und $R^3$ zusammen eine cyclische Ringverbindung, vorzugsweise einen 5-, 6- oder 7-gliedrigen aliphatischen Ring oder

$R^2$ Wasserstoff und $R^3$ mindestens einer der Reste a) bis d) Ketoxims, einer CH— oder NH-aciden Verbindung ist, B den Rest eines Polyols bedeutet, m eine ganze Zahl von 1 bis 3 und n eine ganze Zahl von 1 bis 6 ist, oder

b) 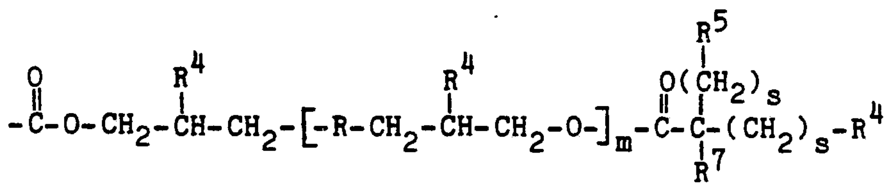

in dem R, $R^4$ und m die schon genannte Bedeutung haben, $R^5$ Wasserstoff oder $R^4$ ist, $R^7$ einen Alkylrest mit 1 bis 8 C-Atomen bedeutet und s eine ganze Zahl von 1 bis 6 darstellt, oder

c) 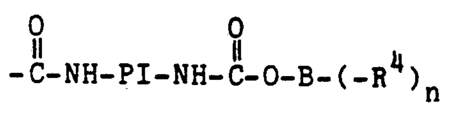 oder

d) die Gruppen $R^8-CHOH-CH_2-O-CO-$ oder $R^8-CHOH-CH_2-$, in denen $R^8$ Wasserstoff, Alkyl mit 1 bis 18 C-Atomen oder Reste von Glycidylestern oder -ethern, vorzugsweise Versaticsäureglycidylestern ist oder die Gruppe

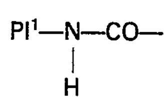

darstellt, in der $Pl^1$ der Rest eines teilverkappten Polyisocyanats ist.

3. Aminourethane nach Anspruch 1 oder 2, dadurch gekennzeichnet, daß die Menge an (a) und (α) zusammen 35 bis 85 Mol-% und die von (β) 65 bis 15 Mol-% beträgt.

4. Aminourethane nach mindestens einem der Ansprüche 1 bis 3, dadurch gekennzeichnet, daß sie die allgemeine Formel (II) besitzen

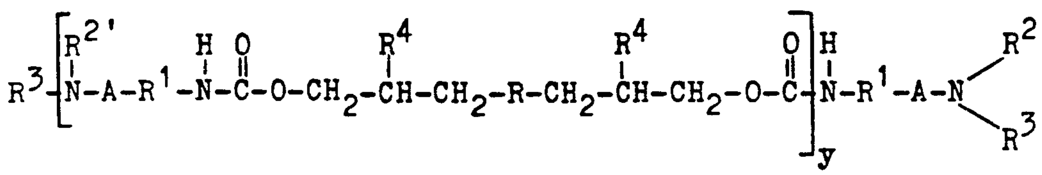

(II),

in der R und $R^1$ bis $R^4$ sowie A die obige Bedeutung haben, $R^{2'}$ gleich $R^2$ ist, mit der Maßgabe, daß $R^{2'}$ nur Wasserstoff darstellt, falls das betreffende Stickstoffatom nicht am Kettenende steht, und

y eine ganze Zahl von 1 bis 10, vorzugsweise 1 bis 5 bedeutet.

5. Aminourethane nach einem oder mehreren der Ansprüche 1 bis 4, dadurch gekennzeichnet, daß sie die allgemeine Formel (IIa)

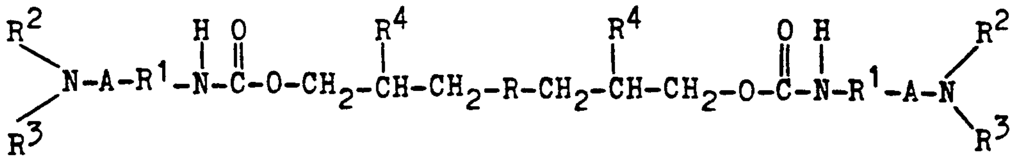

(IIa),

besitzen, n der R und $R^1$ bis $R^4$ sowie A die obige Bedeutung haben.

6. Aminourethane nach Anspruch 4 und/oder 5, dadurch gekennzeichnet, daß R in den Formeln (II) bzw. (IIa) der Rest

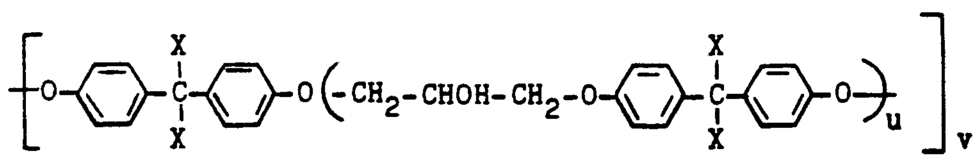

,

ist, in dem X Wasserstoff oder Methyl, u Null bis 5 und v 1 bis 20 ist.

7. Aminourethane nach Anspruch 4 und/oder 5, dadurch gekennzeichnet, daß R in den Formeln (II) bzw. (IIa) den Rest

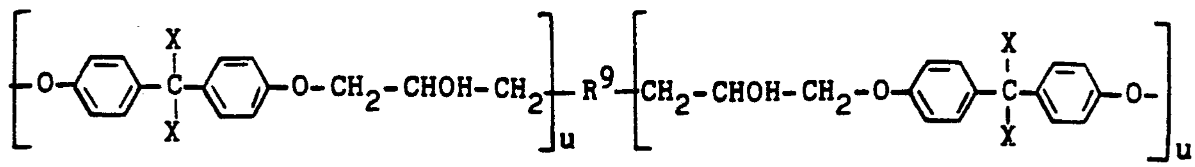

darstellt, worin X und u die genannte Bedeutung haben und $R^9$ 0-Alkyl-O,

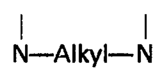

mit jeweils 2 bis 18 C-Atomen im Alkylrest sowie der Rest von Polyaminen, Polyolen, Polycaprolactonpolyolen, OH-gruppenhaltigen Polyestern, Polyethern, hydroxy-, carboxyl- und aminofunktionellen Polymerölen, Polycarbonsäure, hydroxy- oder aminofunktionellen Polytetrahydrofuranen und Reaktionsprodukten von Polyaminen mit Versaticsäureglycidylestern ist.

8. Aminourethane nach Anspruch 7, dadurch gekennzeichnet, daß $R^9$ den Rest

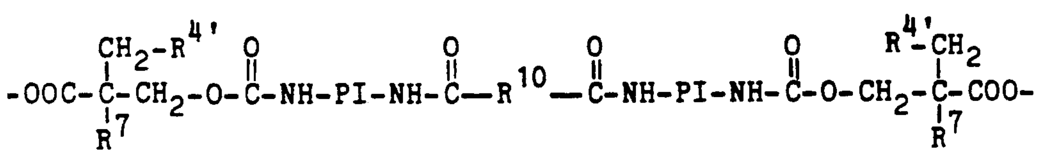

darstellt, wobei die Reste $R^7$ und PI die obige Bedeutung haben, $R^{4'}$ gleich $R^4$ ist und zusätzlich auch Wasserstoff sein kann, $R^{10}$ für die unter $R^9$ angeführten Reste mit Ausnahme der Polycarbonsäuren und der carboxylfunktionellen Polymeröle steht oder den Rest

$$—OOC—R^{11}—CO—R^{10}—CO—R^{11}—COO—$$

darstellt, in der $R^{10}$ wie vorstehend definiert ist und $R^{11}$ den aliphatischen, cycloaliphatischen oder aromatischen Rest einer Polycarbonsäure bedeutet.

9. Aminourethane nach Anspruch 4 und/oder 5, dadurch gekennzeichnet, daß R in den Formeln (II) bzw. (IIa) der Rest

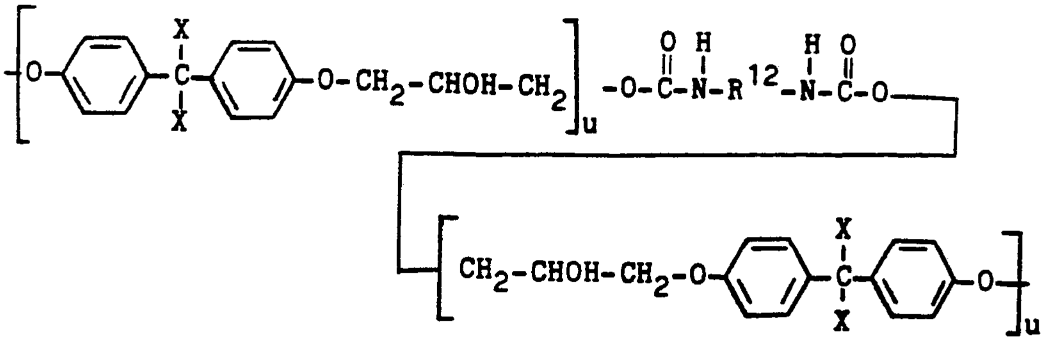

ist, worin X und u die genannte Bedeutung haben und $R^{12}$ Alkylen mit 2 bis 18 C-Atomen, der Rest eines Poly(sek.)Amins oder aminofunktionellen Polytetrahydrofurans ist.

10. Aminourethane nach Anspruch 4 und/oder 5, dadurch gekennzeichnet, daß R in Formel (II) bzw. (IIa) der Rest

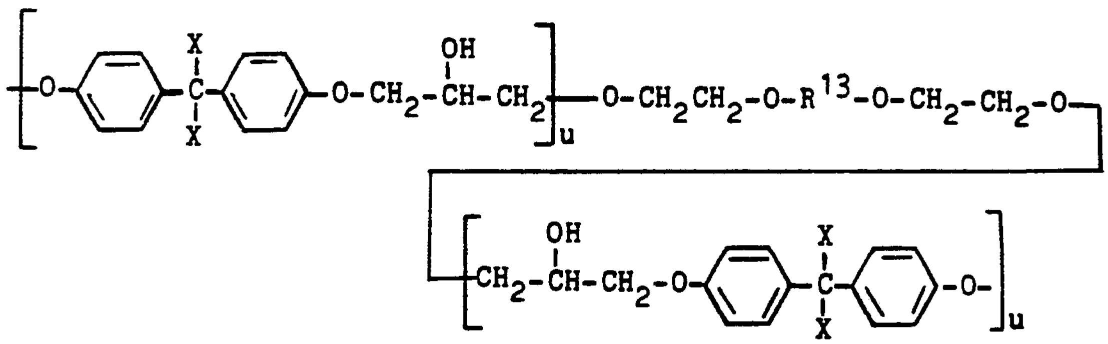

ist, worin X und u die genannte Bedeutung haben, u vorzugsweise aber 1 ist, und $R^{13}$ der Rest

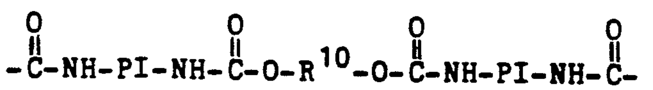 oder 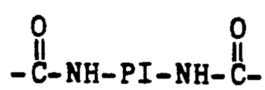

darstellt, wobei $R^{10}$ und PI die schon genannten Definitionen haben.

11. Aminourethane nach einem oder mehreren der Ansprüche 1 bis 10, dadurch gekennzeichnet, daß ein Teil oder alle der vorhandenen Hydroxyl- oder Aminogruppen mit teilverkappten Polyisocyanaten umgesetzt ist.

12. Verfahren zur Herstellung der selbsthärtenden Aminourethane nach einem oder mehreren der Ansprüche 1 sowie 3 bis 11, dadurch gekennzeichnet, daß man (α) Polyamine der allgemeinen Formel (I)

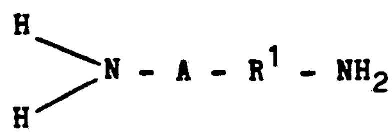 (I),

in der $R^1$ sowie A die Bedeutung gemäß Anspruch 1 besitzen, mit (β) oligomeren oder polymeren Verbindungen, die mindestens zwei endständige 2-Oxo-1,3-dioxolan-Gruppen und zumindest teilweise zusatzlich Urethan-grüppen enthalten und mit Verbindungen (γ) (Kettenstopper) aus der Gruppe teilverkappte Polyisocyanate, Monoepoxide, Monocarbonate und Amine der allgemeinen Formel (I')

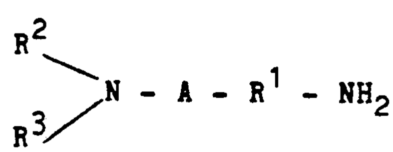 (I'),

in der $R^1$ bis $R^3$ und A die Bedeutung gemäß Anspruch 1 besitzen, umsetzt.

13. Verfahren zur Herstellung der Aminourethane nach einem oder mehreren der Ansprüche 2 bis 11, dadurch gekennzeichnet, daß man (α) Polyamine der allgemeinen Formel (I)

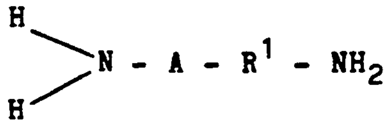
(I),

in der $R^1$ sowie A die Bedeutung gemäß Anspruch 1 besitzen, mit (β) oligomeren oder polymeren Verbindungen, die mindestens zwei endständige 2-Oxo-1,3-dioxolan-Gruppen enthalten und mit Verbindungen (γ) (Kettenstopper) aus der Gruppe teilverkappte Polyisocyanate, Monoepoxide und Amine der allgemeinen Formel (I')

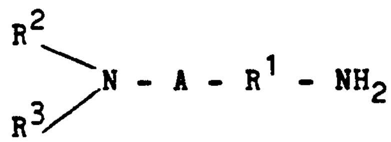
(I'),

in der $R^1$ bis $R^3$ und A die Bedeutung gemäß Anspruch 2 besitzen, umsetzt.

14. Verfahren nach Anspruch 12 oder 13, dadurch gekennzeichnet, daß die Menge an (α) und (γ) zusammen 35 bis 85 Mol-% und die von (β) 65 bis 15 Mol-% beträgt.

15. Verfahen nach mindestens einem der Ansprüche 12 bis 14, dadurch gekennzeichnet, daß die oligomere oder polymere Verbindung (β) mindestens zwei 2-Oxo-1,3-dioxolan-Gruppen enthält und die allgemeine Formel (III)

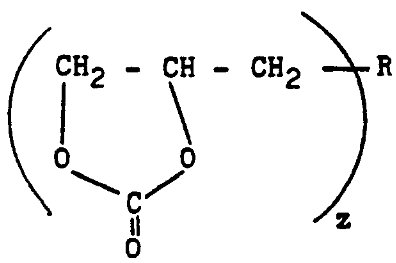
(III),

aufweist, in der R die Bedeutung gemäß Anspruch 1 besitzt, jedoch in seiner Wertigkeit z entspricht, und z für 2 bis 5 steht.

16. Verfahren nach Anspruch 15, dadurch gekennzeichnet, daß z für 2 steht.

17. Verfahren nach Anspruch 15 und/oder 16, dadurch gekennzeichnet, daß R ein Alkylenrest oder der Rest eines Polyethers, Polyetherpolyols, Polyesters, Polyesterpolyols, Poly(sek.)aminrestes, eines Umsetzungsproduktes einer Epoxy-Carbonat-Verbindung mit Polyaminen, Polyolen, Polycaprolactonpolyolen, OH-Gruppen enthaltenden Polyestern, Polyethern, Polyglykolen, hydroxy-, carboxyl- und aminofunktionellen Polymerölen, Polycarbonsäure, hydroxyl- oder aminofunktionellen Polytetrahydrofuranen oder eines Reaktionsproduktes von Polyaminen mit Versaticsäurepolyglycidylestern ist.

18. Verfahren nach einem oder mehreren der Ansprüche 12 bis 17, dadurch gekennzeichnet, daß die Verbindung (β) aus Epoxid-Carbonaten der allgemeinen Formel (IV)

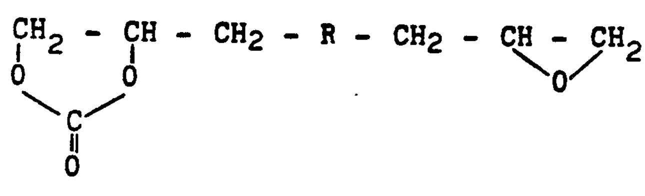
(IV)

durch Umsatz von an die Epoxidgruppe addierbaren, multifunktionellen Verbindungen hergestellt ist, wobei R die oben angegebene Bedeutung hat und die Umsetzungen unter Bedingungen vorgenomen werden, bei denen nur die Epoxydgruppen reagieren und die Carbonatgruppen nicht angegriffen werden.

19. Verfahren nach Anspruch 12 oder 13, dadurch gekennzeichnet, daß als Kettenstopper (γ) Monocarbonatverbindungen der Formel

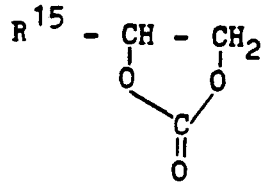
(V)

verwendet werden, in der $R^{15}$ Wasserstoff, Alkyl mit 1 bis 18, vorzugsweise 1 bis 10 C-Atomen, oder Reste des Versaticsäureglycidylesters, Glycidylester- oder Glycidyläther darstellt.

20. Verfahren nach einem oder mehreren der Ansprüche 12 bis 19, dadurch gekennzeichnet, daß die Umsetzung bei 20 bis 150°C erfolgt.

21. Verfahren nach einem oder mehreren der Ansprüche 12 bis 20, dadurch gekennzeichnet, daß man die basischen Aminogruppen der erhaltenen Aminourethane, vorzugsweise gelöst in einem inerten, wassermischbaren organischen Lösungsmittel, teilweise oder vollständig mit wasserlöslichen Säuren, gegebenenfalls unter Zusatz von Wasser, neutralisiert.

22. Elektrotauchlacke auf Wasserbasis, enthaltend die Aminourethane gemaß einem oder mehreren der Ansprüche 1 sowie 3 bis 11, wobei zumindest ein Teil der basischen Amincgruppen dieser Aminourethane neutralisiert ist.

23. Elektrotauchlacke auf Wasserbasis, enthaltend die Aminourethane gemaß einem oder mehreren der Ansprüche 2 bis 11, wobei zumindest ein Tel der basischen Aminogruppen dieser Aminourethane neutralisiert ist.

24. Verwendung der Aminourethane nach einem oder mehreren der Ansprüche 1 bis 11 oder gemäß Anspruch 22 oder 23 als Bindemittel in Lackzubereitungen.

25. Verwendung nach Anspruch 23, dadurch gekennzeichnet, daß es sich um wäßrige Systeme handelt.

**Patentansprüche für die Vertragsstaaten: AT ES**

1. Verfahren zur Herstellung von selbsthärtenden Aminourethanen, dadurch gekennzeichnet, daß man (α) Polyamine der algemeinen Formel(I)

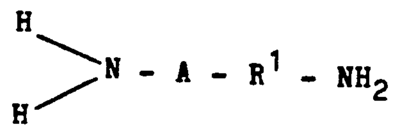

(I),

in der bedeuten

$R^1$ einen zweiwertigen Kohlenwasserstoffrest, vorzugsweise einen geradkettigen oder verzweigten Alkylenrest mit 2 bis 18 C-Atomen, und

A eine chemische Bindung oder $—(R^1—NH)_r—R^1—NH—$, worin r Null oder eine ganze Zahl von 1 bis 6 ist und $R^1$ die vorstehende Bedeutung hat, mit

(β) oligomeren oder polymeren Verbindungen, die mindestens zwei endständige 2-Oxo-1,3-dioxolan-Gruppen und zumindest teilweise zusätzlich Urethan-Grüppen enthalten, und mit

(γ) Verbindungen aus der Gruppe teilverkappte Polyisocyanate, Monoepoxide, Monocarbonate und Amine der allgemeinen Formel (I')

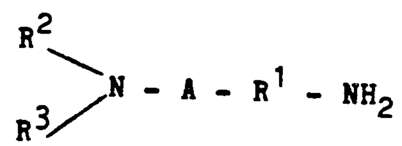

(I'),

umsetzt, in der bedeuten

$R^1$ und A gleiche Bedeutung wie in obiger Formel (I),

$R^2$ und $R^3$ Alkyl mit 1 bis 8 C-Atomen oder Hydroxyalkyl mit 1 bis 8 C-Atomen im Alkylrest oder

$R^2$ und $R^3$ zusammen eine cyclische Ringverbindung oder

$R^2$ Wasserstoff und $R^3$ mindestens einer der Reste a) bis d)

a) 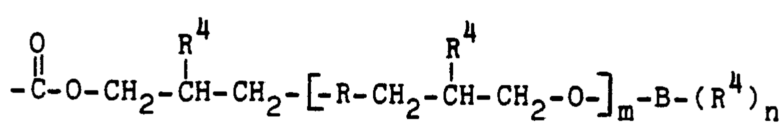

in dem R gleich oder verschieden sein kann und für den Rest eines Diglycidylethers oder -esters, der gegebenenfalls auch $(NR^2)$-Gruppen enthalten kann, wobei $R^2$ die obige Bedeutung hat, oder für einen zweiwertigen Kohlenwasserstoffrest mit 2 bis 18 C-Atomen steht, $R^4$ Hydroxyl oder den Rest

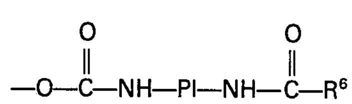

darstellt, in dem PI der Rest eines Polyisocyanats und $R^6$ der Rest eines aliphatischen, cycloaliphatischen oder alkylaromatischen einwertigen Alkohols, eines Aminoalkohols, eines Ketoxims, einer CH— oder NH-aciden Verbindung ist, B den Rest eines Polyols bedeutet, m eine ganze Zahl von 1 bis 3 und n eine ganze Zahl von 1 bis 6 ist, oder

b) 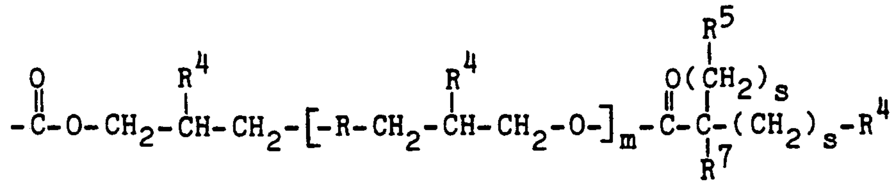

in dem R, $R^4$ und m die schon genannte Bedeutung haben, $R^5$ Wasserstoff oder $R^4$ ist, $R^7$ einen Alkylrest mit 1 bis 8, C-Atomen bedeutet und s eine ganze Zahl von 1 bis 6, darstellt, oder

c) 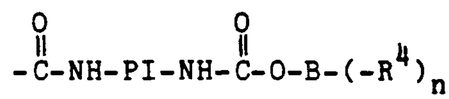 oder

d) die Gruppen $R^8$—CHOH—$CH_2$—O—CO— oder $R^8$—CHOH—$CH_2$—, in denen $R^8$ Wasserstoff, Alkyl mit 1 bis 18 C-Atomen oder Reste von Glycidylestern oder -ethern, vorzugsweise Versaticsäureglycidylestern ist oder die Gruppe

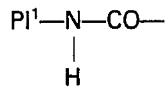

darstellt, in der $PI^1$ der Rest eines teilverkappten Polyisocyanats ist.

2. Verfahren zur Herstellung von Aminourethanen, dadurch gekennzeichnet, daß man (α) Polyamine der allgemeinen Formel (I)

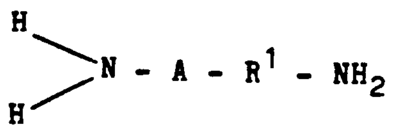 (I),

in der bedeuten

$R^1$ ein zweiwertigen Kohlenwasserstoffrest, vorzugsweise einen geradkettigen oder verzweigter Alkylenrest mit 2 bis 18 C-Atomen, und

A eine chemische Bindung oder —$(R^1—NH)_r$—$R^1$—NH—, worin r Null oder eine ganze Zahl von 1 bis 6 ist und $R^1$ die vorstehende Bedeutung hat, mit

(β) oligomeren oder polymeren Verbindungen, die mindestens zwei endständige 2-Oxo-1,3-dioxolan-Gruppen enthalten, und mit

(γ) Verbindungen aus der Gruppe teilverkappte Polyisocyanate, Monoepoxide und Amine der allgemeinen Formel (I')

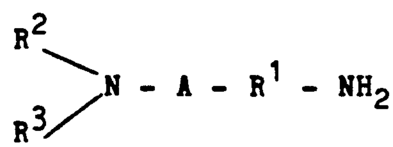 (I'),

umsetzt, in der bedeuten

$R^1$ und A gleiche Bedeutung wie in obiger Formel (I),

$R^2$ Wasserstoff und $R^3$ mindestens einer der Reste a) bis (d)

a) 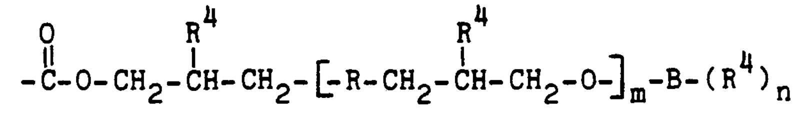

in dem R gleich oder verschieden sein kann und für den Rest eines Diglycidylethers oder -esters, der gegebenenfalls auch ($NR^2$)-Gruppen enthalten kann, wobei $R^2$ die obige Bedeutung hat, oder für einen zweiwertigen Kohlenwasserstoffrest mit 2 bis 18 C-Atomen steht, $R^4$ Hydroxyl oder den Rest

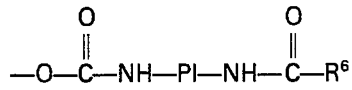

darstellt, in dem PI der Rest eines Polyisocyanats und $R^6$ der Rest eines aliphatischen, cycloaliphatischen

oder alkylaromatischen einwertigen Alkohols, eines Aminoalkohols, eines Ketoxims, einer CH— oder NH-aciden Verbindung ist, B den Rest eines Polyols bedeutet, m eine ganze Zahl von 1 bis 3 und n eine ganze Zahl von 1 bis 6 ist, oder

b) 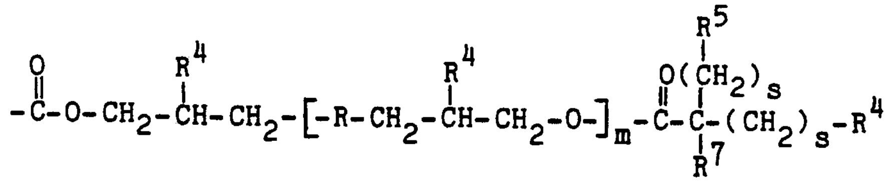

in dem R, $R^4$ und m die schon genannte Bedeutung haben, $R^5$ Wasserstoff oder $R^4$ ist, $R^7$ einen Alkylrest mit 1 bis 8 C-Atomen bedeutet und s eine ganze Zahl von 1 bis 6 darstellt, oder

c) 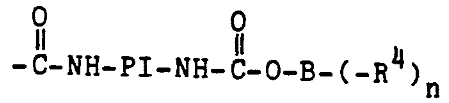 oder

d) die Gruppen $R^8—CHOH—CH_2—O—CO—$ oder $R^8—CHOH—CH_2—$, in denen $R^8$ Wasserstoff, Alkyl mit 1 bis 18 C-Atomen oder Reste von Glycidylestern oder -ethern, vorzugsweise Versaticsäureglycidylestern ist oder die Gruppe

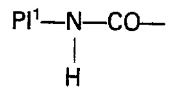

darstellt, in der $Pl^1$ der Rest eines teilverkappten Polyisocyanats ist.

3. Verfahren nach Anspruch 1 oder 2, dadurch gekennzeichnet, daß die Menge an (α) und (γ) zusammen 35 bis 85 Mol% und die von (β) 65 bis 15 Mol-% beträgt.

4. Verfahren nach mindestens einem der Ansprüche 1 bis 3, dadurch gekennzeichnet, daß es sich bei der Komponente (a) um N-Methylethylendiamin, N,N-Dimethylaminopropylamin, Diethylentriamin, Tetraethylenpentamin, Pentaethylenhexamin oder 4,7-Dioxadecan-1,10-diamin handelt.

5. Verfahren nach einem oder mehreren der Ansprüche 1 bis 4, dadurch gekennzeichnet, daß die oligomere oder polymere Verbindung (β) mindestens zwei 2-Oxo-1,3-dioxolan-Gruppen enthält und die allgemeine Formel (III)

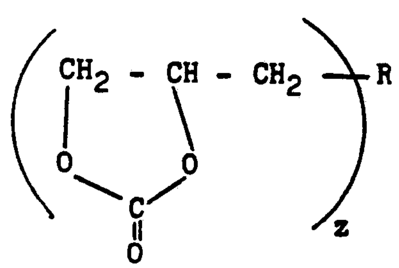 (III),

aufweist, in der R gleich oder verschieden sein kann und für den Rest eines Diglycidylethers oder -esters, der gegebenenfalls auch ($NR^2$)-Gruppen enthalten kann, wobei $R^2$ die obige Bedeutung hat, oder für einen zweiwertigen Kohlenwasserstoffrest mit 2 bis 18 C-Atomen steht wobei die Wertigkeit von R dem Zahlenwert von z entspricht und z ganze Zahlen von 2 bis 5 bedeutet.

6. Verfahren nach Anspruch 5, dadurch gekennzeichnet, daß z für 2 steht.

7. Verfahren nach Anspruch 5 und/oder 16, dadurch gekennzeichnet, daß R ein Alkylenrest oder der Rest eines Polyethers, Polyetherpolyols, Polyesters, Polyesterpolyols, Poly(sek.)aminrestes, eines Umsetzungsproduktes einer Epoxy-Carbonat-Verbindung mit Polyaminen, Polyolen, Polycaprolactonpolyolen, OH-Gruppen enthaltenden Polyestern, Polyethern, Polyglykolen, hydroxy-, carboxyl- und aminofunktionellen Polymerölen, Polycarbonsäure, hydroxyl- oder aminofunktionellen Polytetrahydrofuranen oder eines Reaktionsproduktes von Polyaminen mit Versaticsäurepolyglycidylestern ist.

8. Verfahren nach Anspruch 5 und/oder 6, dadurch gekennzeichnet, daß R in Formel (III) der Rest

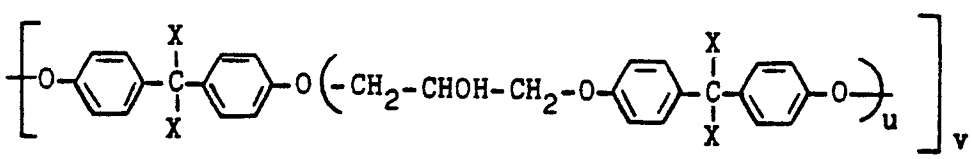,

ist, n dem X Wasserstoff oder Methyl, u Null bis 5 und v 1 bis 20 ist.

9. Verfahren nach einem oder mehreren der Ansprüche 1 bis 8, dadurch gekennzeichnet, daß die Verbindung (β) aus Epoxid-Carbonaten der allgemeinen Formel

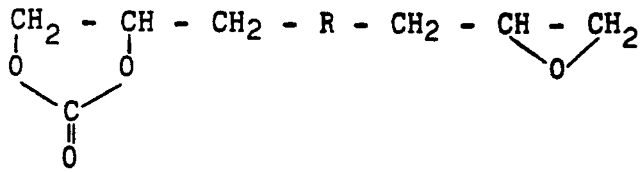

(IV)

durch Umsatz von an die Epoxidgruppe addierbaren, multifunktionellen Verbindungen hergestellt ist, wobei R die oben angegebene Bedeutung hat und die Umsetzungen unter Bedingungen vorgenomen werden, bei denen nur die Epoxydgruppen reagieren und die Carbonatgruppen nicht angegriffen werden.

10. Verfahren nach Anspruch 1, dadurch gekennzeichnet, daß als Verbindungen (γ) Monocarbonatverbindungen der Formel

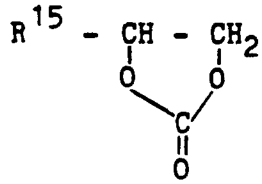

(V)

verwendet werden, in der $R^{15}$ Wasserstoff, Alkyl mit 1 bis 18, vorzugsweise 1 bis 10 C-Atomen, oder Reste des Versaticsäureglycidylesters, Glycidylester- oder Glycidyläther darstellt.

11. Verfahren nach einem oder mehreren der Ansprüche 1 bis 10, dadurch gekennzeichnet, daß die Umsetzung bei 20 bis 150°C erfolgt.

12. Verfahren nach einem oder mehreren der Ansprüche 1 bis 11, dadurch gekennzeichnet, daß man die basischen Aminogruppen der erhaltenen Aminourethanen, vorzugsweise gelöst in einem inerten, wassermischbaren organischen Lösungsmittel, teilweise oder vollständig mit wasserlöslichen Säuren, gegebenenfalls unter Zusatz von Wasser, neutralisiert.

13. Elektrotauchlacke auf Wasserbasis, enthaltend Aminourethane erhalten nach einem oder mehreren der Ansprüche 1 sowie 3 bis 12, wobei zumindest ein Teil der basischen Aminogruppen dieser Aminourethane neutralisiert ist.

14. Elektrotauchlacke auf Wasserbasis, enthaltend die Aminourthane erhalten nach einem oder mehreren der Ansprüche 2 bis 12, wobei zumindest ein Tel der basischen Aminogruppen dieser Aminourethane neutralisiert ist.

15. Verwendung der Aminourethane, erhalten nach einem oder mehreren der Ansprüche 1 bis 12 oder gemäß Anspruch 13 oder 14 als Bindemittel in Lackzubereitungen.

16. Verwendung nach Anspruch 15, dadurch gekennzeichnet, daß es sich um wäßrige Systeme handelt.

**Revendications pour les Etats contractants: BE CH DE FR GB GR IT LI LU NL SE**

1. Aminouréthannes autoréticulants, consistant essentiellement en des motifs structurels qui dérivent:

(α) de polyamines de formule générale (I)

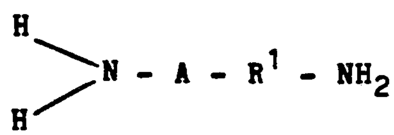

(I),

dans laquelle

$R^1$ représente un reste d'hydrocarbures divalents, avantageusement un reste alkylène linéaire ou ramifié ayant 2 à 18 atomes de carbone, et

A représente une liaison chimique ou $-(R^1-NH)_r-R^1-NH$, formule dans laquelle r est nul ou représente un nombre entier valant 1 à 6, et $R^1$ a le sens précité,

(β) Des composés oligomères ou polymères contenant au moins deux groupes oxo-2 dioxolannes-1,3 terminaux et en outre, au moins en partie, des groupes uréthannes, et

(γ) des polyamines de formule générale (I')

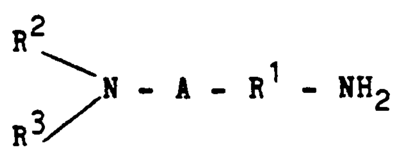

(I'),

dans laquelle

$R^1$ et A ont le même sens que dans la formule (I) ci-dessus,

$R^2$ et $R^3$ représentent chacun un groupe alkyle ayant 1 à 8 atomes de carbone ou un groupe hydroxyalkyle ayant 1 à 8 atomes de carbone et dans le reste alkyle, ou bien

$R^2$ et $R^3$ forment ensemble un composé cyclique, ou bien $R^2$ représente un atome d'hydrogène et $R^3$ représente au moins l'un des restes a) à d):

a) 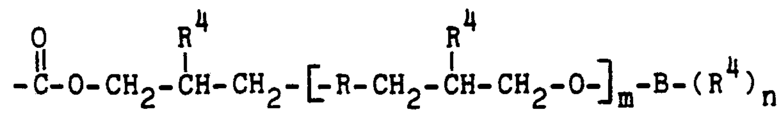

dans laquelle les symboles R peuvent être identiques ou différents, ils représentent le reste d'un oxyde diglycidylique ou ester diglycidylique pouvant éventuellement contenir aussi des groupes ($NR^2$), $R^2$ ayant le sens ci-dessus, ou bien $R^3$ représente un reste d'hydrocarbures divalents comportant 2 à 18 atomes de carbone; $R^4$ représente un groupe hydroxyle ou le reste

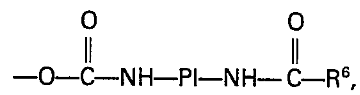

dans lequel PI représente le reste d'un polyisocyanate et $R^6$ le reste d'un alcool monovalent aliphatique, cycloaliphatique ou arylaromatique, d'un aminoalcool, d'une cétoxime, d'un composé comportant un groupe CH ou NH acide, B représente le reste d'un polyol, m est un nombre entier valant 1 à 3 et n est un nombre entier valant 1 à 6, ou

b) 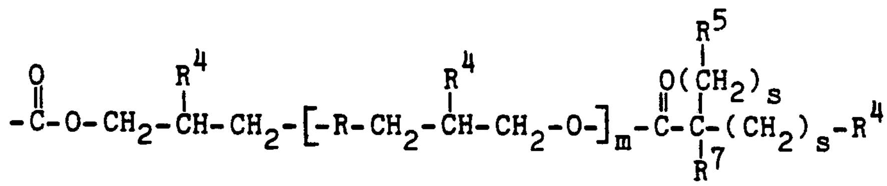

dans laquelle R, $R^4$ et m ont le sens déjà indiqué; $R^5$ représente un atome d'hydrogène ou le reste $R^4$; $R^7$ représente un reste alkyle ayant 1 à 8 atomes de carbone, et s est un nombre entier valant 1 à 6, ou bien

c) 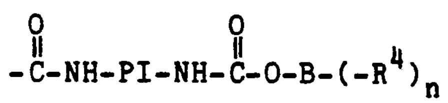 ou

d) les groupes $R^8—CHOH—CH_2—O—CO—$ ou $R^8—CHOH—CH_2—$,

dans lesquels $R^8$ représente un atome d'hydrogène, un groupe alkyle ayant 1 à 18 atomes de carbone ou des restes d'esters ou d'oxydes glycidyliques, avantageusement des esters glycidyliques d'acide versatique, ou le groupe

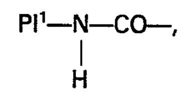

dans lequel $PI^1$ représente le reste d'un polyisocyanate partiellement coiffé.

2. Aminouréthannes consistant essentiellement en des motifs structurels qui dérivent (α) de polyamines de formule générale (I)

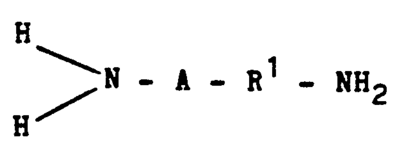 (I),

dans laquelle

$R^1$ représente un reste d'hydrocarbures divalents, avantageusement un reste alkylène linéaire ou ramifié ayant 2 à 18 atomes de carbone, et

A représente une liaison chimique ou $—(R^1—NH)_r—R^1—NH$, formule dans laquelle r est nul ou représente un nombre entier valant 1 à 6, et $R^1$ a le sens précité,

(β) Des composés oligomères ou polymères contenant au moins deux groupes oxo-2 dioxolannes-1,3 terminaux et

(γ) des polyamines de formule générale (I')

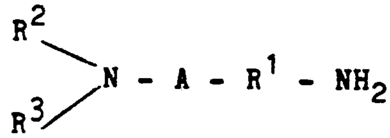 (I'),

dans laquelle

$R^1$ et A ont le même sens que dans la formule (I) ci-dessus,

$R^2$ représente un atome d'hydrogène et $R^3$ représente au moins l'un des restes a) à d)

a) 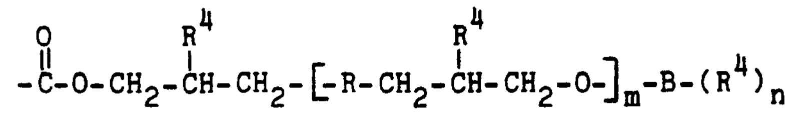

dans lequel les R peuvent être identiques ou différents et représentent chacun le reste d'un oxyde ou ester diglycidylique, qui peut éventuellement contenir aussi des groupes ($NR^2$), $R^2$ ayant le sens ci-dessus, ou bien $R^3$ représente un reste d'hydrocarbures divalents comportant 2 à 18 atomes de carbone, $R^4$ représente un groupe hydroxyle ou le reste

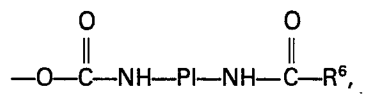

dans lequel PI représente le reste d'un isocyanate et $R^6$ le reste d'un alcool monovalent aliphatique, cycloaliphatique ou alkylaromatique, d'un aminoalcool, d'une cétoxime, d'un composé comportant un groupe CH ou NH acide, B représente le reste d'un polyol, m est un nombre entier valant 1 à 3 et n est un nombre entier valant 1 à 6, ou

b) 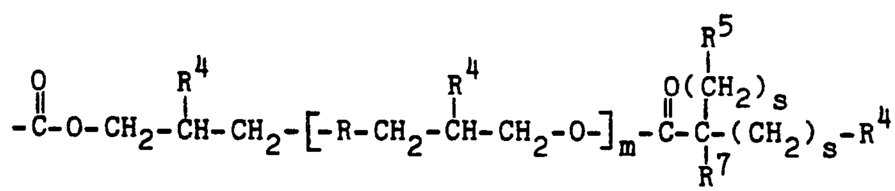

dans laquelle R, $R^4$ et m ont le sens déjà indiqué; $R^5$ représente un atome d'hydrogène ou le reste $R^4$; $R^7$ représente un reste alkyle ayant 1 à 8 atomes de carbone, et s est un nombre entier valant 1 à 6, ou bien

c) 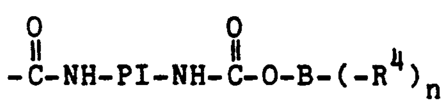 ou

d) les groupes $R^8—CHOH—CH_2—O—CO—$ ou $R^8—CHOH—CH_2—$,

dans lesquels $R^8$ représente un atome d'hydrogène, un groupe alkyle ayant 1 à 18 atomes de carbone ou des restes d'esters ou d'oxydes glycidyliques, avantageusement des esters glycidyliques d'acide versatique, ou le groupe

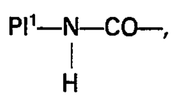

dans lequel $PI^1$ représente le reste d'un polyisocyanate partiellement coiffé.

3. Aminouréthannes selon la revendication 1 ou 2, caractérisés en ce que la quantité de (α) et de (γ) représente ensemble 35 à 85 moles% et la quantité de (β) représente 65 à 15 moles%.

4. Aminouréthannes selon l'une au moins des revendications 1 à 3, caractérisés en ce qu'ils possèdent la formule générale (II)

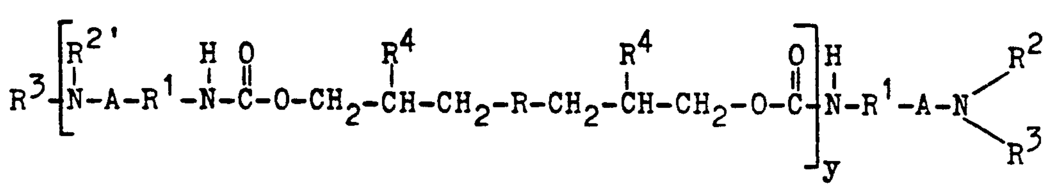 (II),

dans laquelle les symboles R et $R^1$ à $R^4$, ainsi que A, one le sens ci-dessus; $R^{2'}$ est identique à $R^2$, à la condition que $R^{2'}$ ne représente de l'hydrogène que si l'atome d'azote en cause ne se trouve pas en extrémité de chaîne, et

y est un nombre entier valant 1 à 10, avantageusement 1 à 5.

5. Aminouréthannes selon une ou plusieurs des revendications 1 à 4, caractérisés en ce qu'ils possèdent la formule générale (IIa)

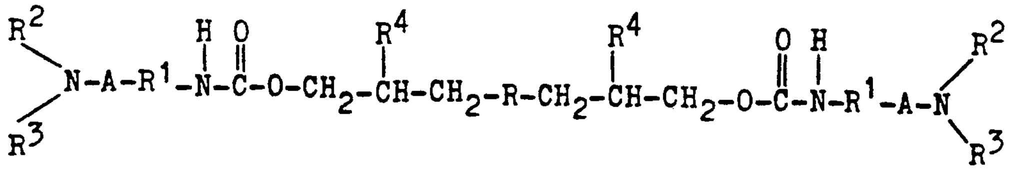
(IIa),

dans laquelle les symboles R et $R^1$ à $R^4$ ainsi que A ont le sens précité.

6. Aminouréthannes selon la revendication 4 et/ou 5, caractérisés en ce que, dans les formules (II) ou (IIa), le symbole R représente le reste

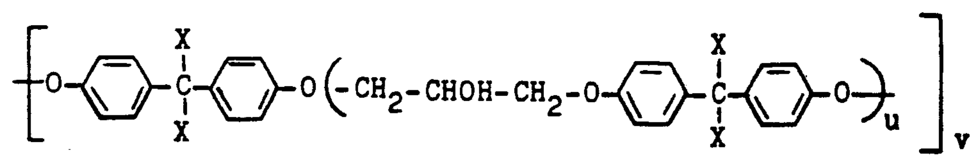
,

dans lequel X représente un atome d'hydrogène ou un groupe méthyle; u est un nombre nul ou valant jusqu'à 5 et v vaut 1 à 20.

7. Aminouréthannes selon la revendication 4 et/ou 5, caractérisés en ce que, dans les formules II) ou IIa), le symbole R représente la formule

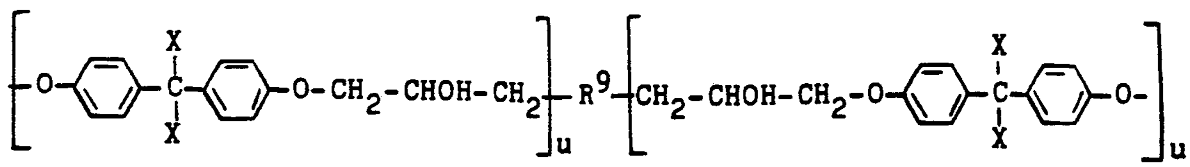

dans laquelle X et u ont le sens précité, et $R^9$ représente un groupe O-alkyl-O, —N-alkyl-N, ayant chacun 2 à 18 atomes de carbone dans le reste alkyle, ainsi que le reste de polyamines, de polyols de polycaprolactone polyols, de polyesters contenant des groupes OH, de polyéthers, d'huile polymère contenant des groupes fonctionnels hydroxy, carboxyles et amino, d'acides polycarboxyliques, de polytétrahydrofuranne contenant des groupes fonctionnels hydroxyles et amino, et des produits de réaction de polyamines avec des esters glycidyliques de l'acide versatique.

8. Aminouréthannes selon la revendication 7, caractérisés en ce que $R^9$ représente le reste

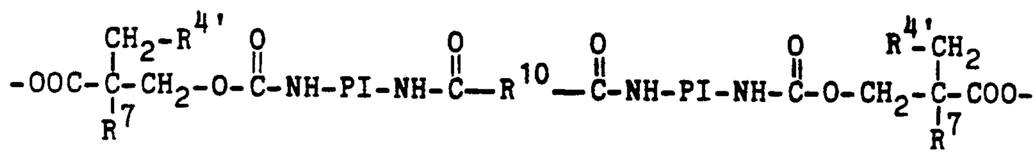

dans lequel les restes $R^7$ et PI ont le sens précité; $R^{4'}$ est identique à $R^4$ et peut représenter en outre aussi un atome d'hydrogène; $R^{10}$ représente les restes indiqués pour $R^9$, à l'exclusion des acides polycarboxyliques et des huiles polymères contenant des groupes fonctionnels carboxyles, ou bien $R^{10}$ représente le reste

$$—OOC—R^{11}—CO—R^{10}—CO—R^{11}—COO—$$

dans lequel $R^{10}$ a le sens défini ci-dessus et $R^{11}$ représente le reste aliphatique, cycloaliphatique ou aromatique d'un acide polycarboxylique.

9. Aminouréthannes selon la revendication 4 et/ou 5, caractérisés en ce que, dans les formules (II) ou (IIa), R représente le reste

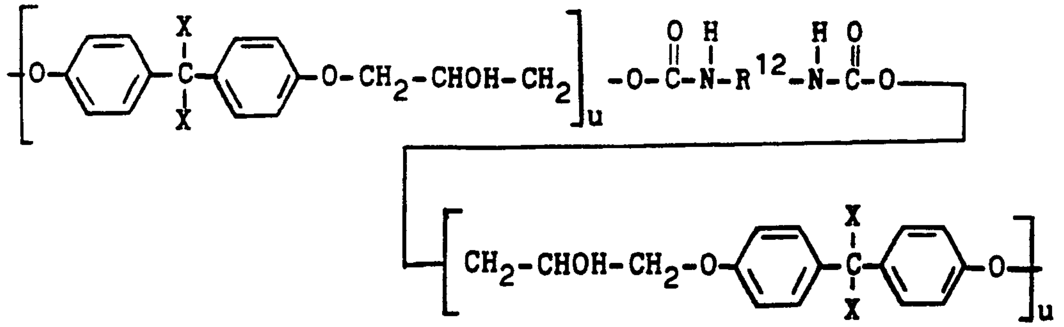

dans lequel X et u ont le sens précité, et $R^{12}$ représente un groupe alkylène ayant 2 à 18 atomes de carbone, le reste d'une polyamine secondaire ou d'un polytétrahydrofuranne comportant des groupes amino fonctionnels.

10. Aminouréthannes selon la revendication 4 et/ou 5, caractérisés en ce que, dans les formules (II) ou (IIa), R représente le reste

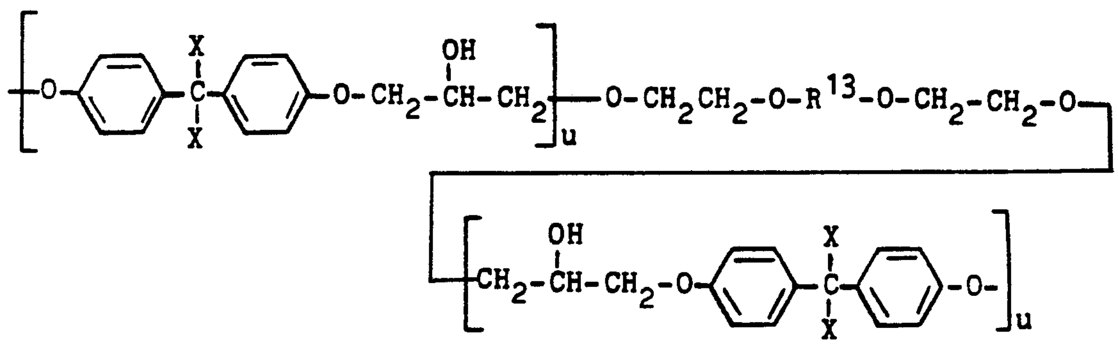

dans lequel X et u ont le sens précité, mais u vaut de préférence 1, et $R^{13}$ représente le reste

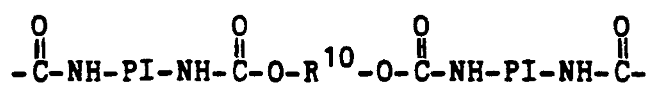 ou 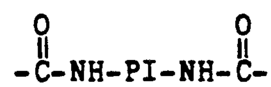

où $R^{10}$ et PI ont les définitions déjà indiquées.

11. Aminouréthannes selon une ou plusieurs des revendications 1 à 10, caractérisés en ce qu'une partie ou la totalité des groupes hydroxyles ou amino présents ont été mis en réaction avec des polyisocyanates partiellement coiffés.

12. Procédé pour préparer les aminouréthannes autodurcissables selon une ou plusieurs des revendications 1 ainsi que 3 à 11, procédé caractérisé en ce qu'on fait réagir:

(α) des polyamines de formule générale (I)

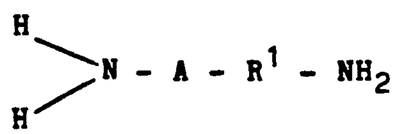 (I),

dans laquelle $R^1$ ainsi que A ont le sens indiqué à la revendication 1, avec (β) des composés oligomères ou polymères, qui contiennent au moins deux groupes oxo-2 dioxolannes-1,3 terminaux et au moins partiellement des groupes uréthannes supplémentaires, et avec

(γ) des composés (agents d'arrêt ou de terminaison des chaînes) choisis dans l'ensemble constitué par des polyisocyanates partiellement coiffés, des monoépoxydes, des monocarbonates et des amines de formule générale (I')

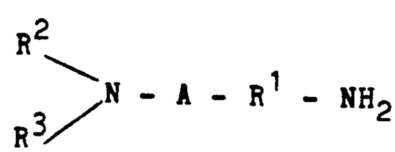 (I'),

dans laquelle $R^1$ à $R^3$ et A ont le sens indiqué à la revendication 1.

13. Procédé pour préparer les aminouréthannes selon une ou plusieurs des revendications 2 à 11, caractérisé en ce qu'on fait réagir

(α) des polyamines de formule générale (I)

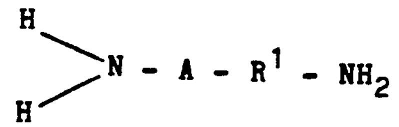

(I),

dans laquelle $R^1$ ainsi que A ont le sens indiqué à la revendication 1,

avec (β) des composés oligomères ou polymères, qui contiennent au moins deux groupes oxo-2 dioxolannes-1,3 terminaux, et avec

(γ) des composés (agents d'arrêt de chaîne) choisis dans l'ensemble constitué par des polyisocyanates partiellement coiffés, des monoépoxydes et des amines de formule générale (I')

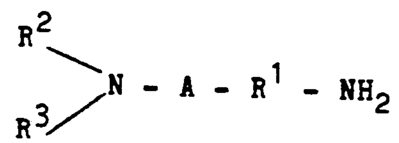

(I'),

dans laquelle $R^1$ à $R^3$ et A ont le sens indiqué à la revendication 2.

14. Procédé selon la revendication 12 ou 13, caractérisé en ce que la quantité de (α) et de ( ) représente ensemble 35 à 85 moles% et la quantité de (β) représente 65 à 15 moles%.

15. Procédé selon l'une au moins des revendications 12 à 14, caractérisé en ce que le composé (β) oligomère ou polymère contient au moins deux groupes oxo-2 dioxolannes-1,3 et présente la formule générale (III)

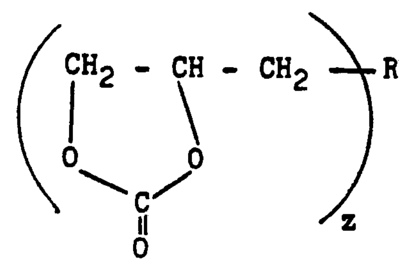

(III),

dans laquelle R a le sens selon la revendication 1, mais présente une valence correspondant à z, et z vaut 2 à 5.

16. Procédé selon la revendication 15, caractérisé en ce que z vaut 2.

17. Procédé selon la revendication 15 et/ou 16, caractérisé en ce que R représente un reste alkylène ou le reste d'un polyéther, d'un polyéther polyol, d'un polyester, d'un polyester polyol, d'une polyamine secondaire, d'un produit de réaction d'un époxy-carbonate avec des polyamines, des polyols, des polycaprolactone polyols, des polyesters contenant des groupes OH, des polyéthers, des polyglycols, des huiles polymères comportant des groupes fonctionnels hydroxyles, carboxyles et amino, des acides polycarboxyliques, des polytétrahydrofurannes contenant des groupes fonctionnels hydroxyles ou amino, ou un produit de réaction de polyamines avec des esters polyglycidyliques de l'acide versatique.

18. Procédé selon une ou plusieurs des revendications 12 à 17, caractérisé en ce que le composé (β) est préparé à partir d'époxy-carbonate de formule générale (IV)

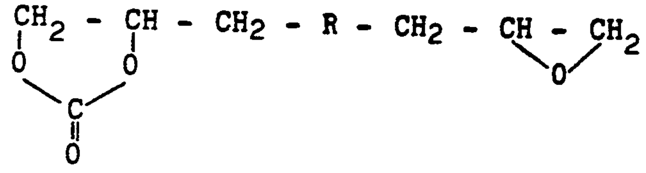

(IV)

par réaction de composés comportant plusieurs groupes fonctionnels et pouvant être fixés par addition sur le groupe époxyde, le symbole R ayant le sens indiqué ci-dessus, et les réactions étant effectuées dans des conditions dans lesquelles seuls les groupes époxydes réagissent et les groupes carbonates ne sont pas attaqués.

19. Procédé selon la revendication 12 ou 13, caractérisé en ce qu'on utilise comme agent (γ) d'arrêt des chaînes, des monocarbonates de formule

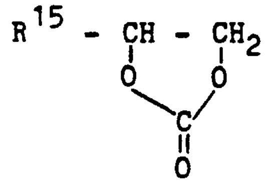 (V)

dans laquelle $R^{15}$ représente un atome d'hydrogène, un groupe alkyle ayant 1 à 18, avantageusement 1 à 10 atomes de carbone, ou des restes de l'ester glycidylique de l'acide versatique, des esters glycidyliques ou des oxydes de glycidyle.

20. Procédé selon une ou plusieurs des revendications 12 à 19, caractérisé en ce que la réaction a lieu entre 20 et 150°C.

21. Procédé selon une ou plusieurs des revendications 12 à 20, caractérisé en ce qu'on soumet les groupes amino basiques des aminouréthannes obtenus, qui sont avantageusement dissous dans un solvant organique inerte miscible à l'eau, partiellement ou totalement à une neutralisation à l'aide d'acides hydrosolubles, éventuellement avec addition d'eau.

22. Laques ou vernis pour trempage électrophorétique, à base d'eau, ce produit comprenant les aminouréthannes selon une ou plusieurs des revendications 1 ainsi que 3 à 11, au moins une partie des groupes amino basiques de ces aminouréthannes étant neutralisée.

23. Laques pour trempage électrophorétique, à base d'eau, ces laques contenant les aminouréthannes selon une ou plusieurs des revendications 2 à 11, au moins une partie des groupes amino basiques de ces aminouréthannes étant neutralisée.

24. Utilisation des aminouréthannes selon une ou plusieurs des revendications 1 à 11 ou selon les revendications 22 ou 23, comme liant dans des préparations de laques et vernis.

25. Utilisation selon la revendication 23, caractérisée en ce qu'il s'agit de systèmes aqueux.

**Revendications pour les Etats contractants: AT ES**

1. Procédé pour préparer des aminouréthannes autodurcissables, procédé caractérisé en ce qu'on fait réagir:

(α) des polyamines de formule générale (I)

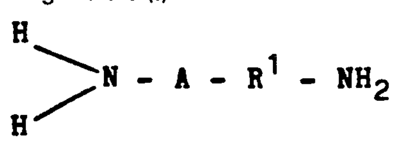 (I),

dans laquelle

$R^1$ représente un reste d'hydrocarbures divalents, avantageusement un reste alkylène linéaire ou ramifié ayant 2 à 18 atomes de carbone, et

A représente une liaison chimique ou $—(R^1—NH)_r—R^1—NH$, formule dans laquelle r est nul ou représente un nombre entier valant 1 à 6, et $R^1$ a le sens précité,

(β) Des composés oligomères ou polymères contenant au moins deux groupes oxo-2 dioxolannes-1,3 terminaux et en outre, au moins en partie, des groupes uréthannes, et

(γ) des composés choisis parmi des polyisocyanates partiellement coiffés, des monoépoxydes, des monocarbonates et des amines de formule générale (I')

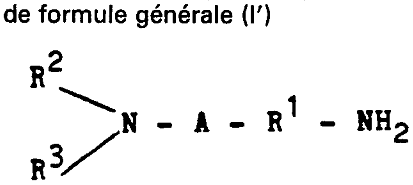 (I'),

dans laquelle

$R^1$ et A ont le même sens que dans la formule (I) ci-dessus,

$R^2$ et $R^3$ représentent chacun un groupe alkyle ayant 1 à 8 atomes de carbone ou un groupe hydroxyalkyle ayant 1 à 8 atomes de carbone et dans le reste alkyle, ou bien

$R^2$ et $R^3$ forment ensemble un composé cyclique, ou bien $R^2$ représente un atome d'hydrogène et $R^3$ représente un atome d'hydrogène et $R^3$ représente au moins l'un des restes a) à d):

a) 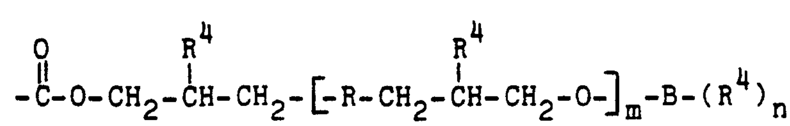

dans lequel les R peuvent être identiques ou différents et représentent le reste d'un oxyde diglycidylique ou ester diglycidylique pouvant éventuellement contenir aussi des groupes ($NR^2$), $R^2$ ayant le sens ci-dessus, ou bien représente un reste d'hydrocarbures divalents comportant 2 à 18 atomes de carbone; $R^4$ représente un groupe hydroxyle ou le reste

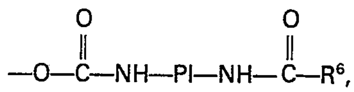

dans lequel PI représente le reste d'un polyisocyanate et $R^6$ le reste d'un alcool monovalent (monoalcool) aliphatique, cycloaliphatique ou arylaromatique, d'un aminoalcool, d'une cétoxime, d'un composé comportant un groupe CH ou NH acide, B représente le reste d'un polyol, m est un nombre entier valant 1 à 3 et n est un nombre entier valant 1 à 6, ou

b) 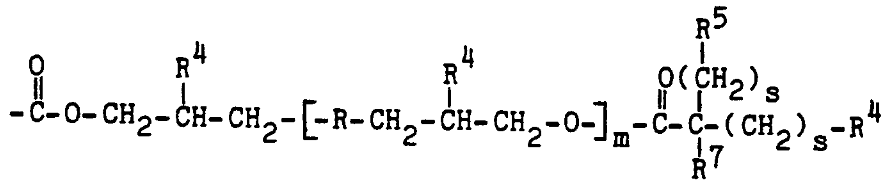

dans lequel R, $R^4$ et m ont le sens déjà indiqué; $R^5$ représente un atome d'hydrogène ou $R^4$; $R^7$ représente un reste alkyle ayant 1 à 8 atomes de carbone, et s est un nombre entier valant 1 à 6, ou bien

c) 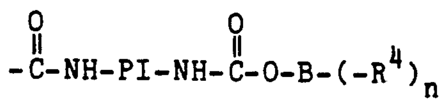 ou

d) les groupes $R^8—CHOH—CH_2—O—CO—$ ou $R^8—CHOH—CH_2—$,

dans lesquels $R^8$ représente un atome d'hydrogène, un groupe alkyle ayant 1 à 18 atomes de carbone ou des restes d'esters ou d'oxydes glycidyliques, avantageusement des esters glycidyliques d'acide versatique, ou le groupe

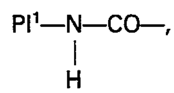

dans lequel $PI^1$ représente le reste d'un polyisocyanate partiellement coiffé.

2. Procédé pour préparer des aminouréthannes, caractérisé en ce qu'on fait réagir

(α) des polyamines de formule générale (I)

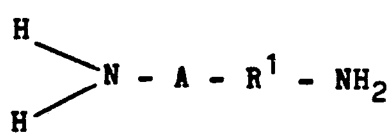 (I),

dans laquelle

$R^1$ représente un reste d'hydrocarbures divalents, avantageusement un reste alkylène linéaire ou ramifié ayant 2 à 18 atomes de carbone, et

A représente une liaison chimique ou $—(R^1—NH)_r—R^1—NH$, formule dans laquelle r est nul ou représente un nombre entier valant 1 à 6, et $R^1$ a le sens précité, avec

(β) Des composés oligomères ou polymères contenant au moins deux groupes oxo-2 dioxolannes-1,3 terminaux et

(γ) des composés choisis parmi des polyisocyanates partiellement coiffés, des monoépoxydes et des amines de formule générale (I')

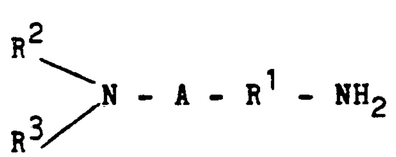 (I'),

dans laquelle

$R^1$ et A ont le même sens que dans la formule (I) ci-dessus,

$R^2$ représente un atome d'hydrogène et $R^3$ représente au moins l'un des restes a) à d)

a) 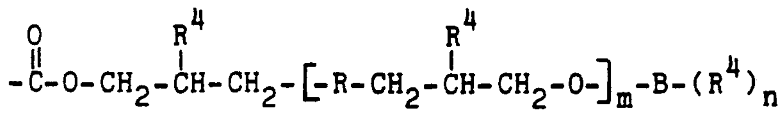

dans laquelle

les R peuvent être identiques ou différents et représentent chacun le reste d'un oxyde ou ester diglycidylique, qui peut éventuellement contenir aussi des groupes ($NR^2$), $R^2$ ayant le sens ci-dessus, ou bien R représente un reste d'hydrocarbures divalents comportant 2 à 18 atomes de carbone, $R^4$ représente un groupe hydroxyle ou le reste

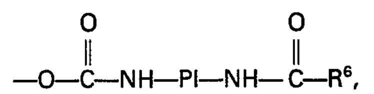

dans lequel PI représente le reste d'un polyisocyanate et $R^6$ le reste d'un alcool monovalent (monoalcool) aliphatique, cycloaliphatique ou alkylaromatique, d'un aminoalcool, d'une cétoxime, d'un composé comportant un groupe CH ou NH acide, B représente le reste d'un polyol, m est un nombre entier valant 1 à 3 et n est un nombre entier valant 1 à 6, ou

b) 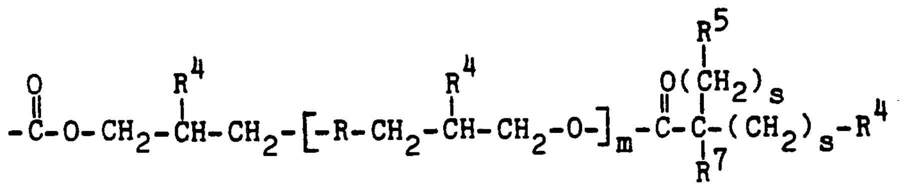

dans laquelle R, $R^4$ et m ont le sens déjà indiqué; $R^5$ représente un atome d'hydrogène ou le reste $R^4$; $R^7$ représente un reste alkyle ayant 1 à 8 atomes de carbone, et s est un nombre entier valant 1 à 6, ou bien

c) 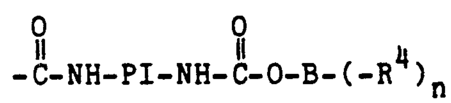 ou

d) les groupes $R^8—CHOH—CH_2—O—CO—$ ou $R^8—CHOH—CH_2—$,

dans lesquels $R^8$ représente un atome d'hydrogène, un groupe alkyle ayant 1 à 18 atomes de carbone ou des restes d'esters ou d'oxydes glycidyliques, avantageusement des esters glycidyliques d'acide versatique, ou le groupe

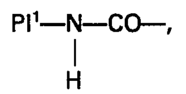

dans lequel $PI^1$ représente le reste d'un polyisocyanate partiellement coiffé.

3. Procédé selon la revendication 1 ou 2, caractérisés en ce que la quantit de (α) et de ( ) ensemble représente 35 à 85 moles% et la quantité de (β) représente 65 à 15 moles%.

4. Procédé selon l'une au moins des revendications 1 à 3, caractérisé en ce qu'il s'agit, pour le composant (a), de la N-méthyléthylènediamine, de la N,N-diméthylaminopropylamine, de la diéthylènetriamine, de la tétraéthylènepentamine, de la pentaéthylènehexamine ou de la dioxa-4,7 décanediamine-1,10.

5. Procédé selon l'une ou plusieurs des revendications 1 à 4, caractérisé en ce que le composé (β) oligomère ou polymère contient au moins deux groupes oxo-2 dioxolannes-1,3 et présente la formule générale (III)

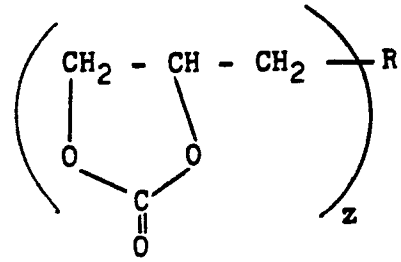 (III),

dans laquelle les R peuvent être identiques ou différents et représentent chacun le reste d'un oxyde ou ester diglycidylique pouvant éventuellement contenir aussi des groupes ($NR^2$), $R^2$ ayant le sens ci-dessus,

ou bien R représente un reste d'hydrocarbure divalent comportant 2 à 18 atomes de carbone, la valence de R correspondant à la valeur de z, et z est un nombre entier valant 2 à 5.

6. Procédé selon la revendication 5, caractérisé en ce que z vaut 2.

7. Procédé selon la revendication 5 et/ou 6, caractérisé en ce que R représente un reste alkylène ou le reste d'un polyéther, d'un polyéther polyol, d'un polyester, d'un polyester polyol, un reste de polyamine secondaire, d'un produit de réaction d'un époxy-carbonate avec des polyamines, des polyols, des polycaprolactone polyols, des polyesters contenant des groupes OH, des polyéthers, des polyglycols, des huiles polymères comportant des groupes fonctionnels hydroxyles, carboxyles et amino, des acides polycarboxyliques, des polytétrahydrofurannes contenant des groupes fonctionnels hydroxyles ou amino, ou un produit de réaction de polyamines avec des esters glycidyliques de l'acide versatique.

8. Procédé selon la revendication 5 et/ou 6, caractérisé en ce que dans la formule (III), R représente le reste

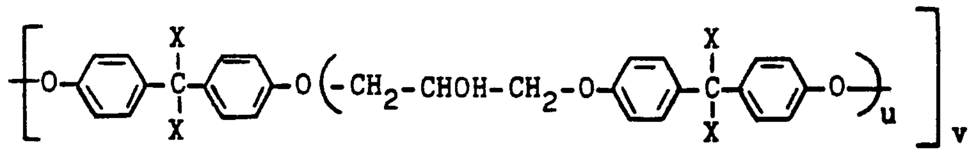 ,

dans lequel X représente un atome d'hydrogène ou un groupe méthyle, u est un nombre nul ou valant jusqu'à 5 et v est un nombre valant 1 à 20.

9. Procédé selon l'une ou plusieurs des revendications 1 à 8, caractérisé en ce que le composé (β) est préparé à partir d'époxy-carbonate de formule générale (IV)

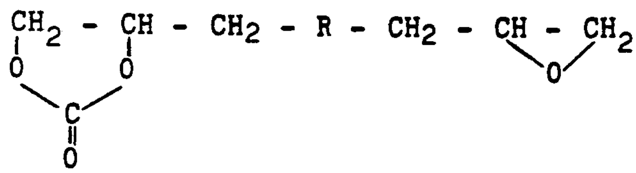 (IV)

par réaction de composés comportant plusieurs groupes multifonctionnels et pouvant être fixés par addition sur le groupe époxyde, le symbole R ayant le sens indiqué ci-dessus, et les réactions étant effectuées dans des conditions dans lesquelles seuls les groupes époxydes réagissent et les groupes carbonates ne sont pas attaqués.

10. Procédé selon la revendication 1, caractérisé en ce qu'on utilise comme composés (γ) des monocarbonates de formule

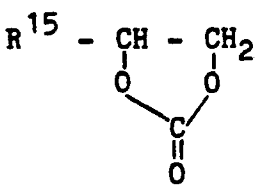 (V)

dans laquelle $R^{15}$ représente un atome d'hydrogène, un groupe alkyle ayant 1 à 18, avantageusement 1 à 10 atomes de carbone, ou des restes de l'ester glycidylique de l'acide versatique, des esters glycidyliques ou des oxydes de glycidyle.

11. Procédé selon une ou plusieurs des revendications 1 à 10, caractérisé en ce que la réaction a lieu entre 20 et 150°C.

12. Procédé selon une ou plusieurs des revendications 1 à 11, caractérisé en ce qu'on soumet les groupes amino basiques présents dans les aminouréthannes obtenus, qui sont avantageusement dissous dans un solvant organique inerte miscible à l'eau, partiellement ou totalement à une neutralisation à l'aide d'acides hydrosolubles, éventuellement avec addition d'eau.

13. Laques ou vernis pour trempage électrophorétique, à base aqueuse, ce produit comprenant les aminouréthannes selon une ou plusieurs des revendications 1 ainsi que 3 à 12, produit dans lequel au moins une partie des groupes amino basiques de ces aminouréthannes étant neutralisée.

14. Laques ou vernis à base aqueuse pour trempage électrophorétique, ce produit contenant les aminouréthannes selon une ou plusieurs des revendications 2 à 14, au moins une partie des groupes amino basiques de ces aminouréthannes étant neutralisée.

15. Utilisation des aminouréthannes selon une ou plusieurs des revendications 1 à 12 ou selon les revendications 13 ou 14, comme liant dans des préparations de laques et vernis.

16. Utilisation selon la revendication 15, caractérisée en ce qu'il s'agit de systèmes aqueux.

**Claims for the Contracting States: BE CH DE FR GB GR IT LI LU NL SE**

1. A self-crosslinking aminourethane consisting essentially of structural units derived from (α) polyamines of the formula (I)

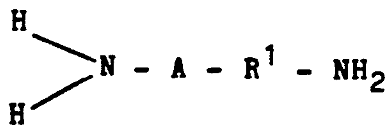 (I),

in which

$R^1$ denotes a divalent hydrocarbon radical, preferably a straight-chain or branched alkylene radical of 2 to 18 carbon atoms, and

A is a chemical bond or $-(R^1-NH)_r-R^1-NH-$, in which r is zero or an integer from 1 to 6 and $R^1$ has the above meanings,

structural units derived from (β) oligomeric or polymeric compounds which contain at least two terminal 2-oxo-1,3-dioxolane groups and additionally at least in some instances urethane groups, and structural units which are derived from polyamines (γ) of the formula (I')

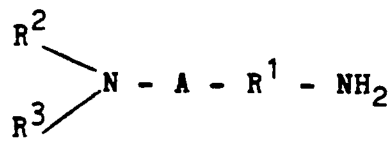 (I'),

in which

$R^1$ and A have the same meanings as in the above formula (I),

$R^2$ and $R^3$ denote alkyl of 1 to 8 carbon atoms or hydroxyalkyl of 1 to 8 carbon atoms in the alkyl radical or

$R^2$ and $R^3$ together form a cyclic ring compound or $R^2$ is hydrogen and $R^3$ denotes at least one of the radicals a) to d)

a) 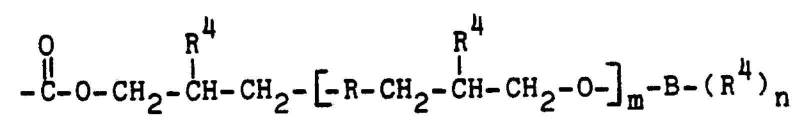

in which the Rs may be identical or different and denote a radical of a diglycidyl ether or ester which may also contain $(NR^2)$ groups, $R^2$ having the above meanings, or denotes a divalent hydrocarbon radical of 2 to 18 carbon atoms, $R^4$ is hydroxyl or the radical

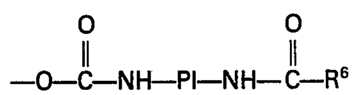

in which PI is the radical of a polyisocyanate and $R^6$ is the radical of an aliphatic, cycloaliphatic or alkylaromatic monohydric alcohol, of an aminoalcohol, of a ketoxime, of a CH- or NH-acidic compound, B denotes the radical of a polyol, m is an integer from 1 to 3 and n is an integer from 1 to 6, or

b) 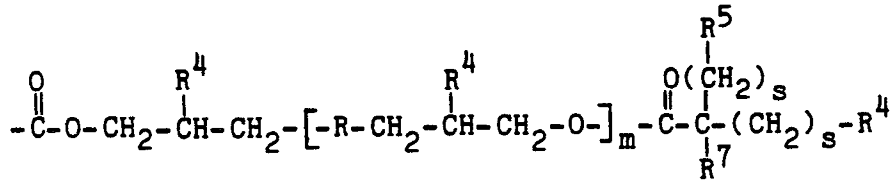

in which R, $R^4$ and m have the previously mentioned meanings, $R^5$ is hydrogen or $R^4$, $R^7$ denotes an alkyl radical of 1 to 8 carbon atoms and s represents an integer from 1 to 6 or

c) 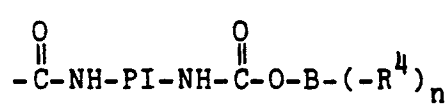 or

d) the groups $R^8-CHOH-CH_2-O-CO-$ or $R^8-CHOH-CH_2-$,

in which $R^8$ is hydrogen, alkyl of 1 to 18 carbon atoms or radicals of glycidyl esters or ethers, preferably glycidyl esters of Versatic acid or represents the group

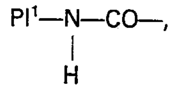

in which $PI^1$ is the radical of a partially blocked polyisocyanate.

2. An aminourethane consisting essentially of structural units derived from (α) polyamines of the formula (I)

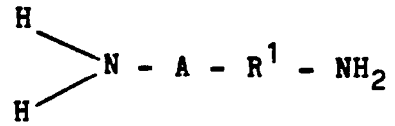 (I),

in which

$R^1$ denotes a divalent hydrocarbon radical, preferably a straight-chain or branched alkylene radical of 2 to 18 carbon atoms, and

A is a chemical bond or $—(R^1—NH)_r—R^1—NH—$, in which r is zero or an integer from 1 to 6 and $R^1$ has the above meanings,

structural units derived from (β) oligomeric or polymeric compounds which contain at least two terminal 2-oxo-1,3-dioxolane groups, and structural units which are derived from polyamines (γ) of the formula (I')

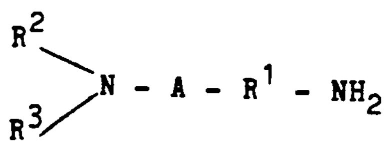 (I'),

in which

$R^1$ and A have the same meanings as in the above formula (I),

$R^2$ is hydrogen and $R^3$ denotes at least one of the radicals a) to d)

a) 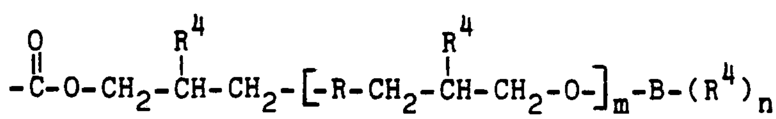

in which the Rs may be identical or different and denote a radical of a diglycidyl ether or ester which may also contain $(NR^2)$ groups, $R^2$ having the above meanings, or denotes a divalent hydrocarbon radical of 2 to 18 carbon atoms, $R^4$ is hydroxyl or the radical

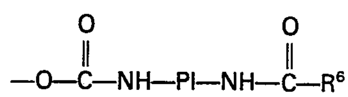

in which PI is the radical of a polyisocyanate and $R^6$ is the radical of an aliphatic, cycloaliphatic or alkylaromatic monohydric alcohol, of an aminoalcohol, of a ketoxime, of a CH- or NH-acidic compound, B denotes the radical of a polyol, m is an integer from 1 to 3 and n is an integer from 1 to 6, or

b) 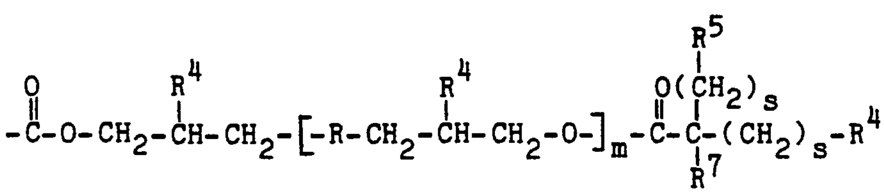

in which R, $R^4$ and m have the previously mentioned meanings, $R^5$ is hydrogen or $R^4$, $R^7$ denotes an alkyl radical of 1 to 8 carbon atoms and s represents an integer from 1 to 6 or

c) 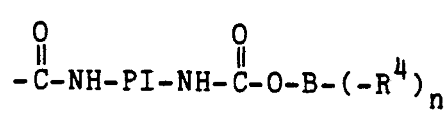 or

d) the groups $R^8—CHOH—CH_2—O—CO—$ or $R^8—CHOH—CH_2—$,

in which $R^8$ is hydrogen, alkyl of 1 to 18 carbon atoms or radicals of glycidyl esters or ethers, preferably glycidyl esters of Versatic acid or represents the group

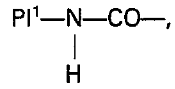

in which $PI^1$ is the radical of a partially blocked polyisocyanate.

3. An aminourethane as claimed in claim 1 or 2, wherein the amount of (a) and (γ) together is 35 to 85 mol-% and that of (β) is 65 to 15 mol-%.

4. An aminourethane as claimed in at least one of claims 1 to 3, which has the formula (II)

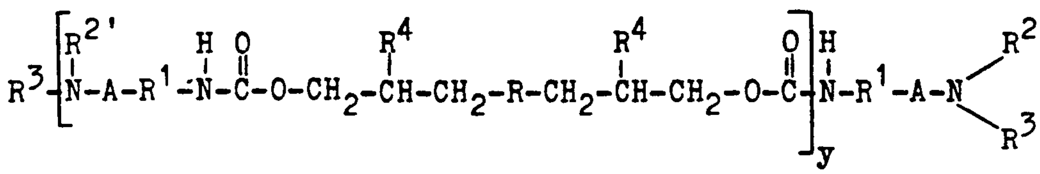 (II),

in which

R and $R^1$ to $R^4$ and A have the above meanings, $R^{2'}$ is equal to $R^2$, with the proviso that $R^{2'}$ only represents hydrogen if the nitrogen atom in question is not at the end of a chain, and

y denotes an integer from 1 to 10, preferably 1 to 5.

5. An aminourethane as claimed in one or more of claims 1 to 4, which has the formula (IIa)

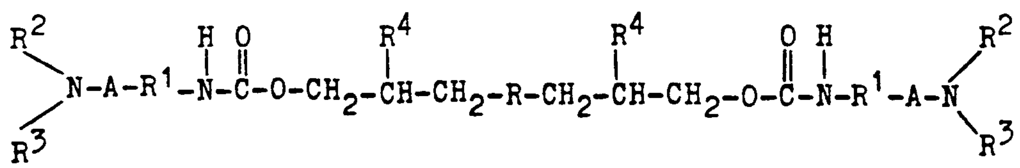 (IIa),

in which R and $R^1$ to $R^4$ and A have the above meanings.

6. An aminourethane as claimed in claim 4 and/or 5, wherein R in the formulae (II) and (IIa) is the radical

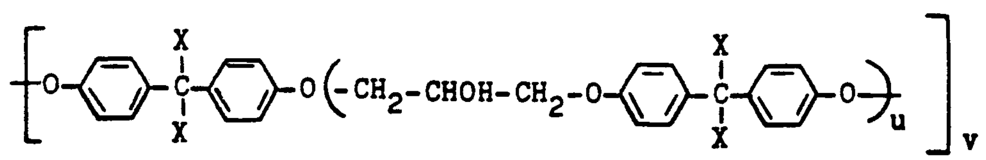

in which X is hydrogen or methyl, u is zero to 5 and v is 1 to 20.

7. An aminourethane as claimed in claim 4 and/or 5, wherein R in the formulae (II) and (IIa) represents the radical

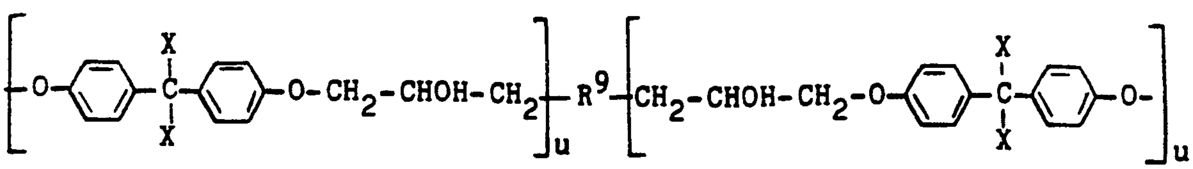

in which X and u have the abovementioned meanings and $R^9$ is O-alkyl-O, N-alkyl-N having in each case 2 to 18 carbon atoms in the alkyl radical and the radical of polyamines, polyols, polycaprolactone-polyols, OH-containing polyesters, polyethers, hydroxyl-, carboxyl- and amino-functional polymer oils, polycarboxylic acids, hydroxyl- or amino-functional polytetrahydrofurans and reaction products of polyamines with glycidyl esters of Versatic acid.

8. An aminourethane as claimed in claim 7, wherein $R^9$ represents the radical

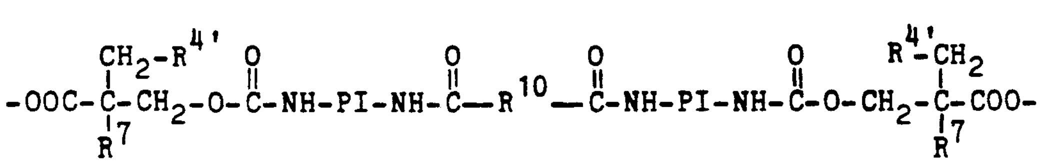

where the radicals $R^7$ and PI have the above meaning, $R^{4'}$ is equal to $R^4$ and can additionally be hydrogen,

$R^{10}$ stands for the radicals listed under $R^9$ with the exception of the polycarboxylic acids and the carboxyl-functional polymer oils or represents the radical

$$—OOC—R^{11}—CO—R^{10}—CO—R^{11}—COO—$$

in which $R^{10}$ is as defined above and $R^{11}$ denotes the aliphatic, cycloaliphatic or aromatic radical of a polycarboxylic acid.

9. An aminourethane as claimed in claim 4 and/or 5, wherein R in the formulae (II) and (IIa) is the radical

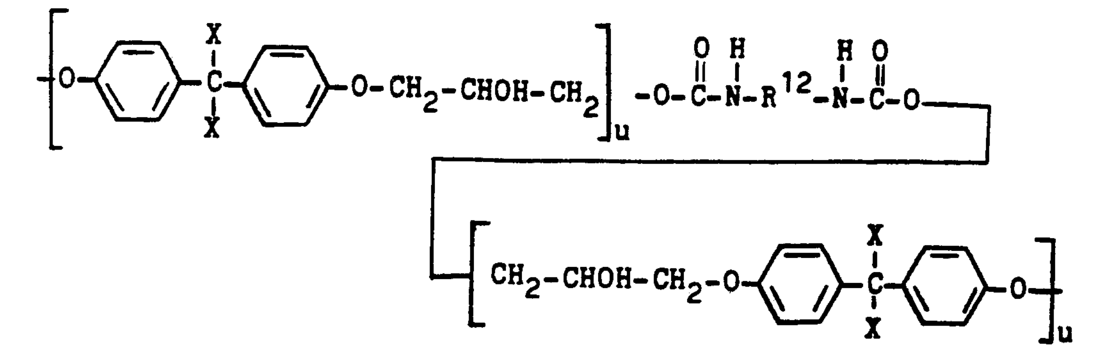

in which X and u have the abovementioned meanings and $R^{12}$ is alkylene of 2 to 18 carbon atoms and the radical of a poly(sec.)amine or of an amino-functional polytetrahydrofuran.

10. An aminourethane as claimed in claim 4 and/or 5, wherein R in the formula (II) or (IIa) is the radical

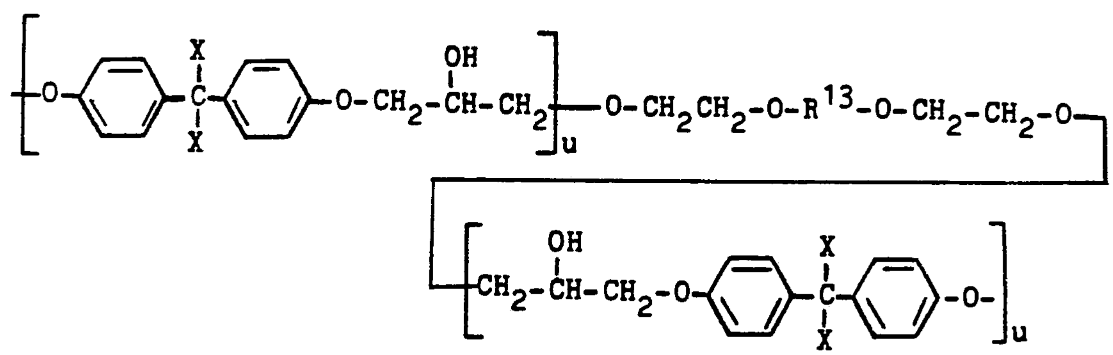

in which X and u have the abovementioned meanings, but u is preferably 1, and $R^{13}$ represents the radical

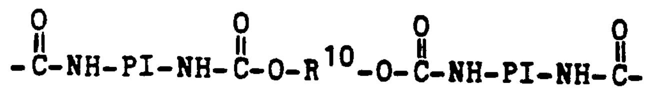 or 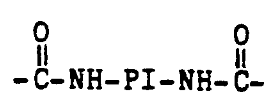

where $R^{10}$ and PI have the previously mentioned meanings.

11. An aminourethane as claimed in one or more of claims 1 to 10, wherein some or all of the hydroxyl or amino groups present have been reacted with partially blocked polyisocyanates.

12. A process for preparing the self-curing aminourethanes claimed in one or more of claims 1 and 3 to 11, which comprises reacting (α) polyamines of the formula (I)

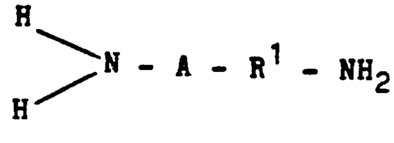 (I),

in which $R^1$ and A are as defined in claim 1, with (β) oligomeric or polymeric compounds which contain at least two terminal 2-oxo-1,3-dioxolane groups and additionally at least in some instances urethane groups, and with compounds (γ) (chain stoppers) selected from the group consisting of partially blocked polyisocyanates, monoepoxides, monocarbonates and amines of the formula (I')

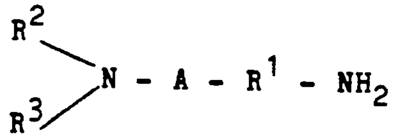 (I'),

in which $R^1$ to $R^3$ and A are as defined in claim 1.

13. The process for preparing an aminourethane as claimed in one or more of claims 2 to 11, wherein (α) a polyamine of the formula (I)

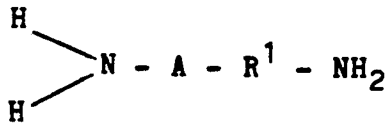 (I),

in which $R^1$ and A have the meanings as claimed in claim 1, is reacted with (β) an oligomeric or polymeric compound which contains at least two terminal 2-oxo-1,3-dioxolane groups and with a compounds (γ) (chain stoppers) selected from the group consisting of partially blocked polyisocyanates, monoepoxides and amines of the formula (I')

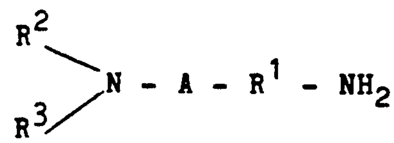 (I'),

in which $R^1$ to $R^3$ and A have the meanings as claimed in claim 2.

14. The process as claimed in claim 12 or 13, wherein the amount of (α) and (γ) together is 35 to 85 mol-% and that of (β) is 65 to 15 mol-%.

15. The process as claimed in at least one of claims 12 to 24, wherein the oligomeric or polymeric compound (β) contains at least two 2-oxo-1,3-dioxolane groups and has the formula (III)

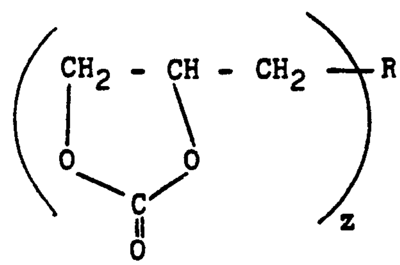 (III),

in which R has the meaning claimed in claim 1 but corresponds in its valence to z, and z stands for 2 to 5.

16. The process as claimed in claim 15, wherein z stands for 2.

17. The process as claimed in claim 15 and/or 16, wherein R is an alkylene radical or the radical of a polyether, of a polyetherpolyol, of a polyester, of a polyesterpolyol, of a poly(sec.)amine radical, of a reaction product of an epoxy-carbonate compound with polyamines, polyols, polycaprolactonepolyols, OH-containing polyesters, polyethers, polyglycols, hydroxyl-, carboxyl- and amino-functional polymer oils, polycarboxylic acids, hydroxyl- or amino-functional polytetrahydrofurans or of a reaction product of polyamines with polyglycidyl esters of Versatic acid.

18. The process as claimed in one or more of claims 12 to 17, wherein compound (β) has been prepared from epoxy carbonates of the formula (IV)

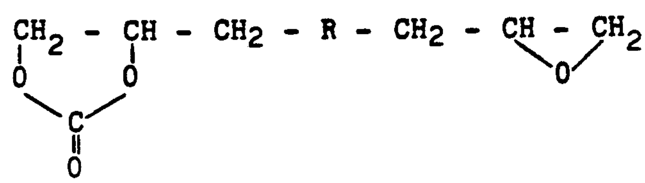 (IV)

by conversion of multifunctional compounds which are addable onto the epoxy group, R having the abovementioned meanings and the conversions being carried out under conditions where only the epoxy groups react and the carbonate groups are not attacked.

19. The process as claimed in claim 12 or 13, wherein the chain stoppers (γ) used are monocarbonate compounds of the formula

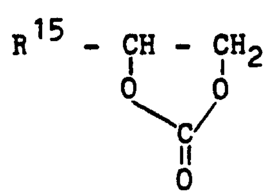 (V)

in which $R^{15}$ represents hydrogen, alkyl of 1 to 18, preferably 1 to 10, carbon atoms, or radicals of the

glycidyl ester of Versatic acid, glycidyl esters or glycidyl ethers.

20. The process as claimed in one or more of claims 12 to 19, wherein the reaction is carried out at 20 to 150°C.

21. The process as claimed in one or more of claims 12 to 20, wherein the basic amino groups of the aminourethanes obtained, preferably dissolved in an inert water-miscible organic solvent, are partly or wholly neutralized with water-soluble acids, in the presence or absence of water.

22. A waterborne electrocoating composition containing the aminourethanes as claimed in one or more of claims 1 and 3 to 11, wherein at least some of the basic amino groups of these aminourethanes have been neutralized.

23. A waterborne electrocoating composition containing the aminourethanes as claimed in one or more of claims 2 to 11, wherein at least some of the basic amino groups of these aminourethanes have been neutralized.

24. Use of an aminourethane as claimed in one or more of claims 1 to 11 or as claimed in claim 22 or 23, as a binder in coating compositions.

25. Use as claimed in claim 23, wherein the coating compositions are aqueous systems.

**Claims for the Contracting States: AT ES**

1. A process for preparing a self-curing aminourethane, which comprises reacting (α) a polyamine of the formula (I)

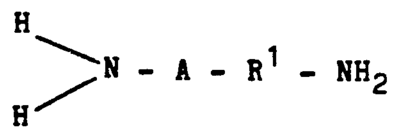 (I),

in which

$R^1$ denotes a divalent hydrocarbon radical, preferably a straight-chain or branched alkylene radical of 2 to 18 carbon atoms, and

A is a chemical bond or $-(R^1-NH)_r-R^1-NH-$, in which r is zero or an integer from 1 to 6 and $R^1$ has the above meaning, with

(β) oligomeric or polymeric compounds which contain at least two terminal 2-oxo-1,3-dioxolane groups and additionally at least in some instances urethane groups, and with (γ) compounds selected from the group consisting of partially blocked polyisocyanates, monoepoxides, monocarbonates and amines of the formula (I′)

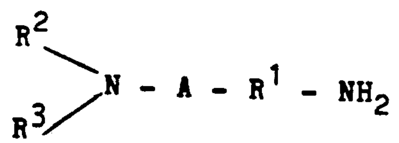 (I'),

in which

$R^1$ and A have the same meanings as in the above formula (I),

$R^2$ and $R^3$ denote alkyl of 1 to 8 carbon atoms or hydroxyalkyl of 1 to 8 carbon atoms in the alkyl radical or

$R^2$ and $R^3$ together form a cyclic ring compound, or $R^2$ is hydrogen and $R^3$ denotes at least one of the radicals a) to d)

a) $$-\overset{O}{\overset{\|}{C}}-O-CH_2-\overset{R^4}{\overset{|}{C}H}-CH_2-[-R-CH_2-\overset{R^4}{\overset{|}{C}H}-CH_2-O-]_m-B-(R^4)_n$$

in which the Rs may be identical or different and denote a radical of a diglycidyl ether or ester which may also contain ($NR^2$) groups, $R^2$ having the above meanings, or denote a divalent hydrocarbon radical of 2 to 18 carbon atoms, $R^4$ represents hydroxyl or the radical

$$-O-\overset{O}{\overset{\|}{C}}-NH-PI-NH-\overset{O}{\overset{\|}{C}}-R^6,$$

in which PI is the radical of a polyisocyanate, and $R^6$ is the radical of an aliphatic, cycloaliphatic or alkylaromatic monohydric alcohol, of an aminoalcohol, of a ketoxime, of a CH- or NH-acidic compound, B is the radical of a polyol, m is an integer from 1 to 3 and n is an integer from 1 to 6, or

b) 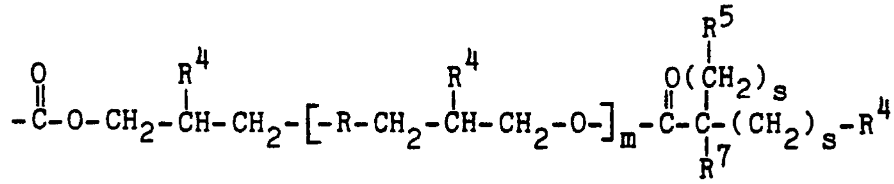

in which R, $R^4$ and m have the previously mentioned meanings, $R^5$ is hydrogen or $R^4$, $R^7$ denotes an alkyl radical of 1 to 8 carbon atoms and s is an integer from 1 to 6, or

c) 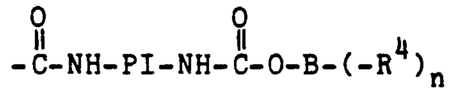 or

d) the groups $R^8$—CHOH—$CH_2$—O—CO— or $R^8$—CHOH—$CH_2$—,
in which $R^8$ is hydrogen, alkyl of 1 to 18 carbon atoms or radicals of glycidyl esters or ethers, preferably glycidyl esters of Versatic acid or represents the group

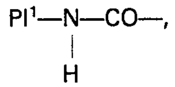

in which $PI^1$ is the radical of a partially blocked polyisocyanate.

2. A process for preparing an aminourethane, which comprises reacting (α) polyamines of the formula (I)

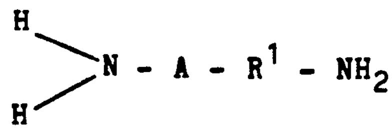 (I),

in which

$R^1$ denotes a divalent hydrocarbon radical, preferably a straight-chain or branched alkylene radical of 2 to 18 carbon atoms, and

A is a chemical bond or —$(R^1$—$NH)_r$—$R^1$—NH—, in which r is zero or an integer from 1 to 6 and $R^1$ has the above meaning, with

(β) oligomeric or polymeric compounds which contain at least two terminal 2-oxo-1,3-dioxolane groups and with (γ) compounds selected from the group consisting of partially blocked polyisocyanates, monoepoxides and amines of the formula (I')

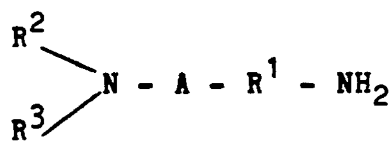 (I'),

in which

$R^1$ and A have the same meanings as in the above formula (I),

$R^2$ is hydrogen and $R^3$ is at least one of the radicals a) to d)

a) 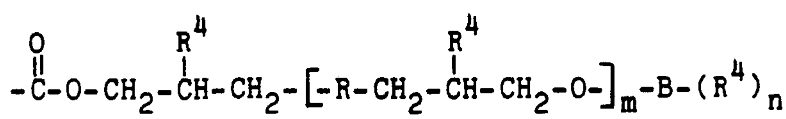

in which the Rs may be identical or different and denote a radical of a diglycidyl ether or ester which may also contain ($NR^2$) groups, $R^2$ having the above meanings, or denote a divalent hydrocarbon radical of 2 to 18 carbon atoms, $R^4$ represents hydroxyl or the radical

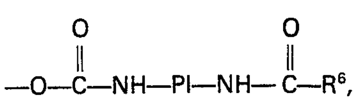

in which PI is the radical of a polyisocyanate, and $R^6$ is the radical of an aliphatic, cycloaliphatic or alkylaromatic monohydric alcohol, of an aminoalcohol, of a ketoxime, of a CH- or NH-acidic compound, B is the radical of a polyol, m is an integer from 1 to 3 and n is an integer from 1 to 6, or

b) 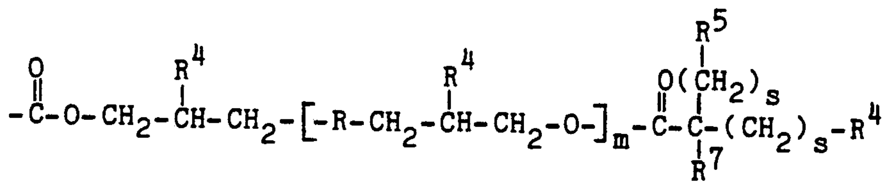

in which R, $R^4$ and m have the previously mentioned meanings, $R^5$ is hydrogen or $R^4$, $R^7$ denotes an alkyl radical of 1 to 8 carbon atoms and s is an integer from 1 to 6, or

c) 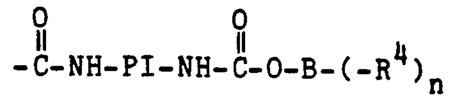 or

d) the groups $R^8$—CHOH—$CH_2$—O—CO— or $R^8$—CHOH—$CH_2$—,
in which $R^8$ is hydrogen, alkyl of 1 to 18 carbon atoms or radicals of glycidyl esters or ethers, preferably glycidyl esters of Versatic acid or represents the group

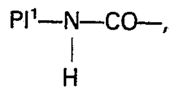

in which $Pl^1$ is the radical of a partially blocked polyisocyanate.

3. The process as claimed in claim 1 or 2, wherein the amount of (α) and (γ) together is 35 to 85 mol-% and that of (β) is 65 to 15 mol-%.

4. The process as claimed in at least one of claims 1 to 3, wherein component (a) is N-methylethylenediamine, N,N-dimethylaminopropylamine, diethylenetriamine, tetraethylenepentamine, pentaethylenehexamine or 4,7-dioxadecane-1,10-diamine.

5. The process as claimed in one or more of claims 1 to 4, wherein the oligomeric or polymeric compound (β) contains at least two 2-oxo-1,3-dioxolane groups and has the formula (III)

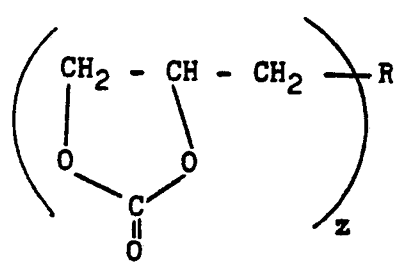 (III),

in which R stands for the radical of a diglycidyl ether or ester which may also contain ($NR^2$)-groups, $R^2$ having the above meanings, or for a divalent hydrocarbon radical of 2 to 18 carbon atoms, the valence of R corresponding to the numerical value of z, and z denotes integers from 2 to 5.

6. The process as claimed in claim 5, wherein z stands for 2.

7. The process as claimed in claim 5 and/or 6, wherein R is an alkylene radical or the radical of a polyether, of a polyetherpolyol, of a polyester, of a polyesterpolyol, of a poly(sec.)amine radical, of a reaction product of an epoxy-carbonate compound with polyamines, polyols, polycaprolactonepolyols, OH-containing polyesters, polyethers, polyglycols, hydroxyl-, carboxyl- and amino-functional polymer oils, polycarboxylic acids, hydroxyl- or amino-functional polytetrahydrofurans or of a reaction product of polyamines with glycidyl esters of Versatic acid.

8. The process as claimed in claim 5 and/or 6, wherein R in formula (III) is the radical

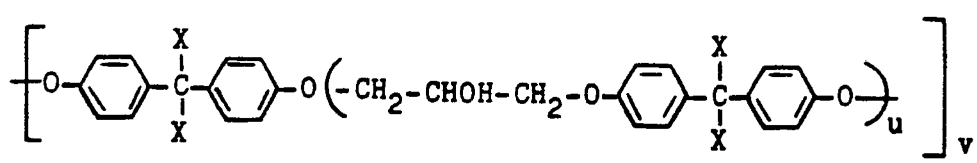 ,

in which X is hydrogen or methyl, u is zero to 5 and v is 1 to 20.

9. The process as claimed in one or more of claims 1 to 8, wherein compound (β) has been prepared from epoxy carbonates of the formula

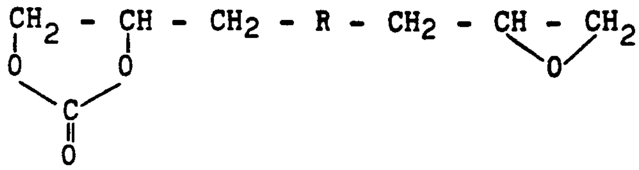

(IV)

by conversion of multifunctional compounds which are addable onto the epoxy group, R having the abovementioned meanings and the conversions being carried out under conditions where only the epoxy groups react and the carbonate groups are not attacked.

10. The process as claimed in claim 1, wherein the compounds (γ) used are monocarbonate compounds of the formula

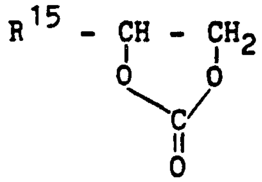

(V)

in which $R^{15}$ represents hydrogen, alkyl of 1 to 18, preferably 1 to 10, carbon atoms or radicals of the glycidyl ester of Versatic acid, glycidyl esters or glycidyl ethers.

11. The process as claimed in one or more of claims 1 to 10, wherein the reaction is carried out at 20 to 150°C.

12. The process as claimed in one or more of claims 1 to 11, wherein the basic amino groups in the aminourethane obtained, preferably dissolved in an inert water-miscible organic solvent, are partly or wholly neutralized with water-soluble acids, in the presence or absence of water.

13. A waterborne electrocoating composition containing aminourethanes obtained as claimed in one or more of claims 1 and 3 to 12, wherein at least some of the basic amino groups of these aminourethanes have been neutralized.

14. A waterborne electrocoating composition containing aminourethanes obtained as claimed in claims 2 to 12, wherein at least some of the amino groups of these aminourethanes have been neutralized.

15. Use of an aminourethane obtained as claimed in one or more of claims 1 to 12 or as claimed in claim 13 or 14, as a binder in coating compositions.

16. Use as claimed in claim 15, wherein the coating compositions are aqueous systems.